(19) Europäisches Patentamt / European Patent Office / Office européen des brevets

(11) EP 3 411 717 B1

(12) # EUROPEAN PATENT SPECIFICATION

(45) Date of publication and mention of the grant of the patent: **20.08.2025 Bulletin 2025/34**

(21) Application number: **17747943.3**

(22) Date of filing: **26.01.2017**

(51) International Patent Classification (IPC):
***G01N 33/66*** *(2006.01)* ***G16H 50/70*** *(2018.01)*
***G16H 20/60*** *(2018.01)* ***G16H 20/17*** *(2018.01)*
***G16H 50/20*** *(2018.01)* ***G16H 40/63*** *(2018.01)*
***A61B 5/00*** *(2006.01)* ***A61M 5/142*** *(2006.01)*
***G06N 20/00*** *(2019.01)* ***G06N 5/04*** *(2023.01)*
***A61B 5/145*** *(2006.01)* *A61M 5/20 (2006.01)*
*G06Q 10/10 (2023.01)*

(52) Cooperative Patent Classification (CPC):
**A61M 5/14244; A61B 5/14532; A61B 5/4839; A61B 5/7264; G06N 5/048; G06N 20/00; G16H 20/17; G16H 20/60; G16H 40/63; G16H 50/20; G16H 50/70;** A61M 5/20; A61M 2005/14208; A61M 2205/502; A61M 2205/581; (Cont.)

(86) International application number: **PCT/US2017/015125**

(87) International publication number: **WO 2017/136218 (10.08.2017 Gazette 2017/32)**

(54) **SYSTEM AND METHOD FOR DECISION SUPPORT USING LIFESTYLE FACTORS**

SYSTEM UND VERFAHREN ZUR ENTSCHEIDUNGSUNTERSTÜTZUNG UNTER VERWENDUNG VON LEBENSSTILFAKTOREN

SYSTÈME ET PROCÉDÉ D’AIDE À LA DÉCISION À L’AIDE DE FACTEURS DE MODE DE VIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.02.2016 US 201662289825 P**

(43) Date of publication of application: **12.12.2018 Bulletin 2018/50**

(73) Proprietor: **Dexcom, Inc.**
**San Diego, CA 92121 (US)**

(72) Inventors:
- **DAVIS, Anna, Leigh** San Diego, CA 92121 (US)
- **BHAVARAJU, Naresh, C.** San Diego, CA 92121 (US)
- **BLACKWELL, Jennifer** San Diego, CA 92121 (US)
- **BOWMAN, Leif, N.** San Diego, CA 92121 (US)
- **CABRERA, Jr., Esteban** San Diego, CA 92121 (US)
- **CONSTANTIN, Alexandra, Elena** San Diego, CA 92121 (US)
- **DATTARAY, Basab** San Diego, CA 92121 (US)
- **DRAEGER, Rian** San Diego, CA 92121 (US)
- **HEINTZMAN, Nathaniel, David** San Diego, CA 92121 (US)
- **JEPSON, Lauren, Hruby** San Diego, CA 92121 (US)
- **KAMATH, Apurv, Ullas** San Diego, CA 92121 (US)
- **KOEHLER, Katherine, Yerre** San Diego, CA 92121 (US)
- **PAL, Andrew, Attila** San Diego, CA 92121 (US)

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

(Cont. next page)

- **REIHMAN, Eli**
  **San Diego, CA 92121 (US)**
- **WALKER, Tomas, C.**
  **San Diego, CA 92121 (US)**

(74) Representative: **Dentons UK and Middle East LLP**
**One Fleet Place**
**London EC4M 7WS (GB)**

(56) References cited:
**US-A1- 2003 195 404** **US-A1- 2009 143 725**
**US-A1- 2013 282 302** **US-A1- 2015 227 710**

- **"International Conference on Computer Analysis of Images and Patterns. CAIP 2017: Computer Analysis of Images and Patterns", vol. 8700, 1 January 2015, SPRINGER, Berlin, Heidelberg, ISBN: 978-3-642-17318-9, article KLAUS DONSA ET AL: "Towards Personalization of Diabetes Therapy Using Computerized Decision Support and Machine Learning: Some Open Problems and Challenges", pages: 237 - 260, XP055381519, 032548, DOI: 10.1007/978-3-319-16226-3_10**
- **MARLING CINDY ET AL: "The 4 Diabetes Support System: A Case Study in CBR Research and Development", 12 September 2011, INTERNATIONAL CONFERENCE ON COMPUTER ANALYSIS OF IMAGES AND PATTERNS. CAIP 2017: COMPUTER ANALYSIS OF IMAGES AND PATTERNS; [LECTURE NOTES IN COMPUTER SCIENCE; LECT.NOTES COMPUTER], SPRINGER, BERLIN, HEIDELBERG, PAGE(S) 137 - 150, ISBN: 978-3-642-17318-9, XP047427677**
- **ZARKOGIANNI KONSTANTIA ET AL: "A Review of Emerging Technologies for the Management of Diabetes Mellitus", IEEE TRANSACTIONS ON BIOMEDICAL ENGINEERING, IEEE SERVICE CENTER, PISCATAWAY, NJ, USA, vol. 62, no. 12, 1 December 2015 (2015-12-01), pages 2735 - 2749, XP011590078, ISSN: 0018-9294, [retrieved on 20151118], DOI: 10.1109/TBME.2015.2470521**
- **CERNAZANU-GLAVAN COSMIN ET AL: "DeeDee - A mobile intelligent system able to assist a type 1 diabetic through the daily life", 2014 IEEE 9TH IEEE INTERNATIONAL SYMPOSIUM ON APPLIED COMPUTATIONAL INTELLIGENCE AND INFORMATICS (SACI), IEEE, 15 May 2014 (2014-05-15), pages 343 - 347, XP032607997, DOI: 10.1109/SACI.2014.6840089**

(52) Cooperative Patent Classification (CPC): (Cont.)
G06Q 10/109; G16H 20/30; G16H 20/70;
G16H 40/67; Y02A 90/10

## Description

FIELD OF THE INVENTION

The present development relates generally to medical devices such as analyte sensors, including systems and methods for using the same to provide support for treatment decision-making.

BACKGROUND

Diabetes mellitus is a disorder in which the pancreas cannot create sufficient insulin (Type I or insulin dependent) and/or in which insulin is not effective (Type 2 or non-insulin dependent). In the diabetic state, the victim suffers from high glucose, which may cause an array of physiological derangements (for example, kidney failure, skin ulcers, or bleeding into the vitreous of the eye) associated with the deterioration of small blood vessels.

It is extremely important for persons with diabetes to be aware of their current blood glucose values, so as to know if they should take steps to increase or decrease the same, in order to stay with then a target glucose range.

One device used by persons with diabetes to monitor glucose is a self-monitoring blood glucose (SMBG) monitor, which typically uses finger pricks to obtain blood samples for measurement. Another device used to monitor glucose is a continuous analyte sensor, which typically includes a sensor that is placed subcutaneously, transdermally (e.g., transcutaneously), or intravascularly. The sensor measures the concentration of a given analyte within the body, and generates a raw signal that is transmitted to electronics associated with the sensor. The raw signal is converted into an output value that is displayed on a display. The output value that results from the conversion of the raw signal is typically expressed in a form that provides the user with meaningful clinical information, such as glucose expressed in mg/dL.

Enhancements have been made in the area of continuous analyte sensors. For example, it is known to calculate a predicted glucose value based on various parameters measurable by such sensors. It is also known to use glucose values and predicted values in bolus calculators, where bolus calculators take such parameters as well as meal data and determine an amount for a user to bolus. Finally, it is known to use parameters measurable by such sensors in the calculation of values for insulin sensitivity and insulin resistance.

Klaus Donsa et al: "Towards Personalization of Diabetes Therapy Using Computerized Decision Support and Machine Learning: Some Open Problems and Challenges", 1 January 2015 concerns computerized decision support systems (CDSS) for improving the treatment process of diabetes in patient’s self-management but also in institutional care. This document describes relevant parameters for personalizing diabetes therapy and how CDSS cam support the therapy process and the role of machine learning in this context. Moreover, the document identifies open problems and challenges for the personalization of diabetes therapy with focus on decision support systems and machine learning technology.

This Background is provided to introduce a brief context for the Summary and Detailed Description that follow. This Background is not intended to be an aid in determining the scope of the claimed subject matter nor be viewed as limiting the claimed subject matter to implementations that solve any or all of the disadvantages or problems presented above.

SUMMARY

The present invention provides a non-transitory computer-readable medium, containing instructions for causing a computing environment to perform a method of operating and tuning or adapting a decision-support application that forms a part of a continuous glucose monitoring, CGM, application, in accordance with claim 1.

BRIEF DESCRIPTION OF THE DRAWINGS

The present embodiments now will be discussed in detail with an emphasis on highlighting the advantageous features. These embodiments depict the novel and non-obvious systems and methods according to present principles, shown in the accompanying drawings, which are for illustrative purposes only. These drawings include the following figures, in which like numerals indicate like parts:

Fig. 1 is a schematic physical illustration of a decision-support application/functionality according to present principles, indicating in a modular way where the application/functionality may reside.

Figs. 2-5 illustrate logical locations of decision-support application/functionality within various devices, as well as its relation to other applications.

Fig. 6 is a diagram showing a functional dependence of outputs on inputs according to a first implementation of systems and methods according to present principles.

Fig. 7 is a flowchart corresponding to the diagram of Fig. 6.

Fig. 8 is a diagram showing inputs connected to outputs as part of a decision-support application/functionality, as the input and output user interfaces, and the processing itself, are affected by other parameters/inputs.

Fig. 9 is a flowchart corresponding to the diagram of Fig. 8.

Figs. 10(A) and 10(B) are diagrams showing determination of a system model of a patient, as well as the tuning of system parameters according to user-desired functionality (UDF).

Fig. 11(A) is a flowchart corresponding to the diagram of Fig. 10(B).

Fig. 11(B) illustrates types of correlative parameters.

Fig. 11(C) illustrates a state model implementation of a parameterized model.

Figs. 12(A) and 12(B) are flowcharts corresponding to the state model of Fig. 11(C).

Fig. 13 is a flow chart for converting goal information into quantifiable criteria.

Fig. 14 is a flowchart illustrating ways of converting non-crisp inputs into quantifiable criteria.

Fig. 15 illustrates use of one type of correlative parameter within a system model, e.g., a food sensitivity, in the determination of decision-support for a meal bolus.

Fig. 16 illustrates various inputs that may be employed in decision-support application/functionality.

Fig. 17 illustrates various physical sources of input data.

Figs. 18(A) and 18(B) illustrate nontraditional ways of obtaining meal data.

Fig. 19 is a flowchart for obtaining signal signature information which may then be employed as an input for decision-support, e.g., for state definitions and determination.

Fig. 20 is a chart illustrating gastric emptying duration, which can serve as an input into decision-support, e.g., for state definition and determination.

Fig. 21 is a chart illustrating modes, e.g., modes of operation of CGMs, and modes too may be used as inputs into decision-support, e.g., for state definition and determination.

Figs. 22(A) and 22(B) illustrate user input adjusting threshold levels.

Fig. 23A illustrates various types of physical outputs of the decision-support application/functionality.

Fig. 23B illustrates a progressive sequence of modes, phases, or stages, detailing levels or phases of control within an artificial pancreas system.

Fig. 23C illustrates a schematic diagram of an artificial pancreas system.

Fig. 23D is a flowchart depicting exertion of varying levels of pump control within therapeutic modes or phases.

Fig. 24 illustrates a landscape trend graph in an exemplary implementation of a bolus calculator.

Figs. 25A and 25B illustrate a flow of steps in a bolus calculator implemented as part of a CGM app.

Fig. 26 illustrates how a CGM app bolus calculator may detect and notify a user of the recognition of a pattern.

Figs. 27A and 27B illustrate plots useful in determining rate of change impact on insulin bolus.

Figs. 28 and 29 illustrate exemplary user interfaces that may be displayed to a user to provide information regarding bolus and carb values.

Figs. 30 and 31 provide a depiction of an exemplary analyte sensor system.

Elements are not to scale unless otherwise noted.

## DETAILED DESCRIPTION

Systems and methods according to present principles relate to decision-making for management of diabetes. People with diabetes face many problems in controlling their glucose because of the complex interactions between food, insulin, exercise, stress, activity, and other physiological and environmental conditions. Established principles of management of glucose sometimes are not adequate because there is a significant amount of variability in how different conditions impact different individuals and what actions might be effective for them.

The systems and methods according to present principles may be applied to any type of analyte monitoring device, e.g., SMBGs, CGMs, or other types of sensor systems including those with invasive sensors, noninvasive sensors, and implantable sensors. Generally, exemplary embodiments disclosed herein include discussion of a glucose sensor that measures a concentration of glucose or a substance indicative of the concentration or presence of another analyte. The sensor may be a continuous device, for example a subcutaneous, transdermal, transcutaneous, non-invasive, intraocular and/or intravascular (e.g., intravenous) device. In some embodiments, the device or sensor system can analyze a plurality of intermittent blood samples. The glucose sensor can use any method of glucose measurement, including enzymatic, chemical, physical, electrochemical, optical, optochemical, fluorescence-based, spectrophotometric, spectroscopic (e.g., optical absorption spectroscopy, Raman spectroscopy, etc.), polarimetric, calorimetric, iontophoretic, radiometric, and the like. The glucose sensor can use any known detection method, including invasive, minimally invasive, and non-invasive sensing techniques, to provide a data stream indicative of the concentration of the analyte in a host. The data stream is typically a raw data signal that is used to provide a useful value of the analyte to a user, such as a patient or health care professional (HCP, e.g., doctor, physician, nurse, caregiver), who may be using the sensor.

Accordingly, systems and methods according to present principles minimize the impact of the vagaries of diabetes on individuals, i.e., by looking for patterns and tendencies of an individual and customizing the management to that individual. Consequently, the same reduces the uncertainty (that diabetes typically is associated with) and improves quality of life.

In more detail, systems and methods according to present principles meet the needs of the above in several ways. In particular, decision support aspects are supported by an application or by functionality added to an application, where the application runs on a user device such as a mobile phone or smart watch (or by the two in combination, e.g., where the smart phone performs the application/functionality but where certain notifications and/or requests for confirmation, e.g., accepting or denying a therapy prompt, are provided to the smart watch). The decision support application or functionality (hereinafter "decision-support application/functionality") generally supports user-desired functionality, where the user-desired functionality is defined by, e.g., a lifestyle goal to be attained, a problem to be solved, a decision to be made, a pattern to be addressed, and so on. User-desired functionality may be specified by the user but may also be inferred from user actions. For example, if during a period of hypoglycemia a user interacts with their smartphone running a glucose monitoring and decision support application to a greater degree than otherwise, the decision-support application/functionality may infer that the user desires greater knowledge of hypoglycemic events. In other cases, particularly for new users, a goal may be employed which is a default one, e.g., one that is common to many users, e.g., to lessen glucose variability or obtain better control of their diabetes. The decision-support application /functionality may also support decision making based on lifestyle factors, even if such are not specifically stated as a user goal or are unrelated to a user goal.

A number of ways can be employed to provide the decision-support application/functionality.

In one aspect, a functional dependence of decision support needs is defined by inference, or in an ad hoc or heuristic manner. Independent variables are defined, and the same are monitored as patient-specific variables. When the defined functional dependence requires a decision support interaction with the user, as defined by the values of the independent variables, an appropriate interaction is initiated, e.g., a user therapy prompt is displayed.

In another aspect, systems and methods according to present principles provide for creating a parameterized model of the patient. A particularly emphatic parameter, also known as a correlative parameter, is determined or defined. In one implementation, the correlative parameter is one that has a particularly strong relation to decision support aspects, e.g., a particularly high correlation with some aspect of decision support and/or outcomes. In another implementation, the correlative parameter may be one that the user has particular control over, e.g., meal or exercise data, as opposed to insulin sensitivity. In another implementation, the correlative parameter is an aspect of user behavior, e.g., a lifestyle or situational factor that bears on decision support, particularly in combination with a clinical context. For example, the correlative parameter may be a particular pattern determined by the system to occur with respect to certain events or days or times. In yet another implementation, the correlative parameter is an aspect, determined by the system, that the user would not otherwise be aware of or able to calculate on their own, e.g., a sensitivity. However the correlative parameter is determined, the user-desired functionality may then be modified according to the parameterized model and the value of the correlative parameter.

In another aspect, the system provides a calculated insight, i.e., determines a parameter not previously known to the system or not known to the user, e.g., insulin sensitivity, and then uses the same in combination with a lifestyle or a situational parameter, e.g., time of day, day of the week, or various other parameters, to provide a user prompt related to a therapeutic insight.

The decision-support application/functionality receives input comprising user glucose monitoring data, insulin delivery and food/meal data, as well as insulin sensitivity, and the output includes insulin dose recommendations, e.g., bolus calculator functionality, carbohydrate recommendations, e.g., in response to low glucose, and retrospective pattern analysis to optimize meal/insulin matching, to best suit the user's lifestyle and preferences, to minimize user burden, and to minimize the safety risk. In one implementation, the decision-support application/functionality recommends a target glucose value, or target range, that is in part influenced by safety risk. For example, a higher target glucose value may be chosen for a user with a relatively high insulin sensitivity as compared to the target chosen for user with relatively lower insulin sensitivity, as a means to reduce safety risk. The decision-support application/functionality may recommend, at relatively high glucose levels, an insulin dose intended not to return glucose levels to the target, e.g., 120 mg /dl, but rather to an upper limit of the target range, e.g., 180 mg/dL, again to minimize safety risk associated with a large insulin dose.

Similar safety risks associated with device signal quality or confidence may be taken into account in decision support.

In an alternative implementation, instead of using a correlative parameter, data known about the user may be employed to stratify the user into one or more of a number of user profiles. As above, the user-desired functionality may then be modified according to the parameterized model and the determined user profile.

In yet another implementation, a first set of data may be used as inputs to a decision-support application/functionality, while a second set may be used as variables that affect how the decision-support application/functionality operates, e.g., affects the type of user interface provided.

In yet another implementation, user states may be defined and determined which, in combination with a real-time input, lead to a therapy prompt supporting a user decision.

The support application/functionality may be implemented as instructions on a computer-readable medium, as firmware, or as an ASIC, it may be situated as part of sensor electronics, a dedicated receiver, or a mobile device such as a smart phone and /or smart watch. Larger devices such as tablets, laptops, and desktop computers may also be employed. It will be understood that the application/functionality may be embodied as modules on these devices, and the modules and functionality may spread across multiple such devices.

In use, a glucose signal is detected by the sensor and transmitted to a dedicated receiver or mobile device. While processing may occur in any of the locations described above, for simplicity it will be assumed that the device is a mobile device, and that processing occurs on the mobile device. Using a determined calibration, the glucose signal in clinical units is calculated and displayed to the user. Calculations may be performed on the received signal including on stored historic values thereof and further including the determination of time rates of change to various orders. Patterns, trend data, or other lifestyle factors may be determined and employed in the decision-support application/functionality, the same determined by the decision support algorithm or by functionality added to another algorithm, e.g., a continuous glucose monitoring algorithm, a bolus calculator app, or an app associated with or operating a medicament pump/pen. Additional data pertaining to the user may be received from various sources, including other sensors and user input. Such additional data may also be received from cloud-based sources or other data stored and retrievable about the user.

All of these input data may then be potentially used to provide decision support for the user. In some cases, the data may serve as independent variables to a decision-support application/functionality, which outputs a dependent variable of a therapy recommendation. In other cases, data may serve as "content" or "value" variables, whose value is important to the decision support determination, while others serve as "control" variables, whose value is important to the input user interface, the output user interface, or how the processing of the decision-support application/functionality occurs. Content and control variables need not be mutually exclusive, i.e., some variables may serve both purposes, but in other cases the sets of variables may be distinct.

A first aspect is directed towards a non-transitory computer-readable medium, containing instructions for causing a computing environment to perform a method of operating and tuning or adapting a decision-support application, the decision-support application stand-alone, a part of an analyte monitoring application, or a part of a medicament delivery device control system, the tuning or adapting performed in a way to support user-desired functionality, the method including steps of: receiving an indication of user-desired functionality; and tuning or adapting the decision-support application to support the user-desired functionality, such that the tuning or adapting results in an output on a user interface at least partially supported by the decision-support application to be based on the user-desired functionality and on input real-time data.

Implementations may include one or more of the following. The user-desired functionality may be selected from the group consisting of: a lifestyle goal to be attained, a problem to be solved, or a decision to be made. The input real-time data may be from a sensor. The sensor may form part of an analyte concentration monitor. The analyte concentration monitor may be a CGM. The input real-time data may be user entered. The input real-time data, and the output, may be fuzzy or categorized. The method may further include steps of performing a calculation on the input real-time data and/or on one or more stored data to determine derived data, and the output may be further based on the derived data. The derived data may correspond to a pattern or a type of sensitivity, in which the type of sensitivity may be selected from the group consisting of: meal sensitivity, insulin sensitivity, exercise sensitivity, and sleep sensitivity. The medicament delivery control system may drive a pump or pen. The decision-support application may form a part of a bolus calculator, an analyte monitoring application, or vice-versa. The output may be a bolus value. The decision-support application may operate in conjunction with an analyte monitoring application through an API. The tuning or adapting may act as part of an operating system component for the decision-support application. The tuning or adapting may be caused by the decision-support application. The tuning or adapting may act on an input user interface, on an output user interface, or on a processing of the decision-support application.

The method may further include a step of formulating a model of the user as a parameterized state model system. The method may further include a step of modifying the machine human interface between the user and the parameterized state model system. The decision-support application may perform a processing step, and in which the processing step is configured to operate on crisp inputs, and the method may further include performing the tuning or adapting to adjust the input user interface to accept crisp inputs or to transform non-crisp inputs into crisp inputs. The non-crisp inputs may be fuzzified inputs. The decision-support application may display an output, and in which the output may be fuzzified. The decision-support application may display an output, and in which the output may be crisp. The decision-support application performs a processing step, and in which the processing step may be configured to operate on fuzzy or categorized inputs, and further including performing the tuning or adapting to adjust the input user interface to accept fuzzy inputs or to transform non-fuzzy inputs into fuzzy inputs. The decision-support application may display an output, and in which the output may be fuzzified. The decision-support application may display an output, and in which the output may be crisp. The method may further include a step of translating the lifestyle goal to be attained, the problem to be solved, or the decision to be made, into one or more objective criteria. The one or more objective criteria may be clinical criteria. The method may further include a step of translating the user-desired functionality into one or more objective criteria. The tuning or adapting may be configured to iteratively update its source algorithm, so as to improve the tuning or adapting over time.

The user-desired functionality may correspond to a level of alerting/alarming during an event on the user's calendar. The user-desired functionality may be controllable by one or more slider bars. The tuning or adapting the decision-support application to support the user-desired functionality may include disposing the decision-support application in one of a plurality of modes. At least one of the modes may have a plurality of sub modes. One of the modes may be a user goal mode, and in which the user goal mode may be further specified into a sub mode by user selection from a suitable user interface, in which the sub mode may be selected from one or more of the group consisting of: a goal the user would like to accomplish, a user desire, or a problem the user would like to solve. One of the modes may be a device uncertainty mode, and in which the mode may be further specified into a sub mode by determination of data signal quality or confidence. One of the modes may be a risk stratification hierarchy mode, and in which the mode may be further specified into a sub mode by determination of a glycemic urgency index. The tuning or adapting may cause the output to be represented by a data file having a data structure, the data structure based on the tuning or adapting. The tuning or adapting may be based at least partially on one or more of: device uncertainty, physiological uncertainty, and/or behavioral uncertainty.

A second aspect is directed towards a non-transitory CRM, containing instructions for causing a computing environment to perform a method of operating and tuning or adapting a decision-support application for analyte control, the decision-support application stand-alone, a part of an analyte monitoring application, or a part of a medicament delivery device control system, the tuning or adapting performed in a way to support user-desired functionality, the method including steps of: defining a system model of a user; determining a plurality of system parameters of the system model of the user; determining a correlative parameter, the correlative parameter having a significant effect on a clinical variable related to an analyte; and modifying the format of an input or output user interface supported by the decision support application, the modifying based on a value of the correlative parameter and values of one or more of the determined system parameters.

Implementations may include one or more of the following. The correlative parameter may be a parameter determined to be influential in control of the analyte. The correlative parameter may be a parameter determined to be controllable by a user. The correlative of parameter may be a lifestyle factor. The clinical variable may be the analyte concentration. The correlative parameter may be a sensitivity. The sensitivity may be selected from the group consisting of: food sensitivity, insulin sensitivity, exercise sensitivity, and sleep sensitivity. One or more of the system parameters may be defined in a fuzzy or categorized manner. The modifying may be performed using a fuzzy process. The correlative parameter may be an index to a situation or circumstance with a significant correlation to an outcome. The correlative parameter may be meal data, one of the system parameters may be a food sensitivity, and the outcome may be a suggested meal bolus. The correlative parameter may be meal data, one of the system parameters may be a food sensitivity, and the outcome may be a suggested correction bolus. The correlative parameter may be meal data, one of the system parameters may be a food sensitivity, and the outcome may be a suggested change to a basal rate. The correlative parameter may be meal data, one of the system parameters may be a food sensitivity, and the outcome may be a suggested number of rescue carbs. The modifying may be further based on data entered by the user. The modifying may be further based on data received from a cloud-based database.

The method may further include classifying a user into one of a plurality of predefined profiles based on one or more of the plurality of system parameters. The system parameters may be distinct or alternatively two or more may be related. One system parameter may be analyte concentration variability and another may be a sensitivity of the analyte concentration to sleep, exercise, food, or medicament. The modifying the format may include: providing fields for analyte concentration, a therapy recommendation, and an indication of confidence in the analyte concentration; and populating the fields with appertaining data. The modifying the format may include: providing fields for analyte concentration, a therapy recommendation, and a context indicator; and populating the fields with appertaining data. The method may further include prompting the user to confirm or ignore the therapy recommendation. The modifying may be further based on prior user confirmations or ignorations of prompts. The method may further include, if the user confirms the therapy recommendation, providing system feedback on the user's response. The method may further include prompting the user for user feedback on the therapy recommendation and the user's response, and basing subsequent modifications on the user feedback.

A system parameter may be a pattern determined using historical analyte data. The modifying may be based on case-based reasoning. The modifying may be further based on data entered by the user, in which the data entered by the user may be entered in a selected format, and the method may further include prompting the user to enter data in the selected format in subsequent data entries. The modifying may be further based on data entered by the user, in which the data entered by the user may be entered in a fuzzified or categorized format. The modifying may include modifying a level of prompting on the output user interface, whereby a level of interaction of a machine-human interface may be modified to match the user-desired functionality.

A third aspect is directed towards a non-transitory CRM, containing instructions for causing a computing environment to perform a method of operating and tuning or adapting a decision-support application for analyte control, the decision-support application stand-alone, a part of an analyte monitoring application, or a part of a medicament delivery device control system, the tuning or adapting performed in a way to support user-desired functionality, the method including steps of: determining a plurality of system parameters of a system model of the user; from the determined plurality of system parameters, stratifying the user into one of a plurality of stratifications; and modifying the form or content of an input or output user interface supported by the decision support application, the modifying based on the determined stratification.

Implementations may include one or more of the following. The plurality of stratifications may correspond to a plurality of predefined profiles. The method may further include receiving a measured value of an analyte concentration, and the modifying may be further based on the measured value.

A fourth aspect is directed towards a non-transitory computer readable medium, containing instructions for causing a computing environment to perform a method of operating and tuning or adapting a decision-support application, the decision-support application stand-alone, a part of an analyte monitoring application, or a part of a medicament delivery device control system, the tuning or adapting performed in a way to support user-desired functionality, the method including steps of: receiving an indication of user-desired functionality; receiving a first datum in real time, the first data having a clinical context; receiving or retrieving a second datum, the second datum providing a clinical or behavioral or lifestyle context; tuning or adapting the decision-support application to support the user-desired functionality, such that the tuning or adapting results in an output on a user interface at least partially supported by the decision-support application to be based on the user-desired functionality and on the first and second data.

Implementations may include the following. The user-desired functionality may be a user goal.

A fifth aspect is directed towards a non-transitory computer readable medium, containing instructions for causing a computing environment to perform a method of operating and tuning or adapting a decision-support application, the decision-support application stand-alone, a part of an analyte monitoring application, or a part of a medicament delivery device control system, the tuning or adapting based on uncertainty, the uncertainty selected from the group consisting of: device uncertainty, physiological uncertainty, behavioral uncertainty, and combinations thereof.

Implementations may include the following. If behavioral uncertainty is high, the decision-support application may be tuned or adapted to be less aggressive, whereby a patient who is prone to hypoglycemia because of behavioral tendencies may be more effectively treated.

A sixth aspect is directed towards a method of providing decision support functionality to a user, the functionality supporting decision-making in the management of the disease, including: performing machine learning about a user by: receiving first and second data about a user; and defining at least two states associated with the user based on the received first and second data; displaying a decision-support output to the user by: determining a current state associated with the user, the determined state from among the defined at least two states; receiving a first real-time input; and displaying a therapy prompt based on the first real-time input and the determined state associated with the user.

Implementations may include one or more of the following. The first real-time input may include CGM data and a datum selected from the group consisting of: calendar data, time of day data, location data, meal data, or exercise or activity data. The defined states may correspond to two or more different insulin sensitivity profiles. The defined states may correspond to two or more different activity profiles. The defined states may correspond to two or more different exercise profiles. The defined states may correspond to two or more different workout profiles. The determining a current state associated with the user may include receiving a second real-time input, and basing the determined state at least partially on the received second real-time input. The first real-time input may be the same as the second real-time input. The displayed therapy prompt may be the result of a bolus calculation.

The defining at least two states associated with the user may further include defining two or more sub states associated with the user, in which the sub states are selected from the group consisting of a lifestyle sub state, a clinical sub state, a situational sub state, and a device sub state. The clinical sub state may include a number of clinical sub sub states selected from the group consisting of a hypoglycemic sub sub state, a hyperglycemic sub sub state, a euglycemic sub sub state, and in which the clinical sub sub state further includes sub sub states corresponding to whether the patient's glucose value is rising, falling, or stable. The lifestyle sub state may include a number of lifestyle sub sub states selected from the group consisting of: a mealtime sub sub state, an activity sub sub state, an illness sub sub state, a pregnancy sub sub state. The device sub state may include a number of device sub sub states corresponding to levels of signal quality or confidence in determined measurement data.

The displayed therapy prompt may include an indication of signal quality or confidence. The method may further include switching from a first defined state associated with the user to a second defined state associated with the user, receiving a second real-time input, and displaying a therapy prompt based on the second real-time input and the second state associated with the user.

A seventh aspect is directed towards a non-transitory computer-readable medium, including instructions for causing a computing environment to perform any of the above methods.

In further aspects and implementations, the above method features of the various aspects are formulated in terms of a system as in various aspects. Any of the features of an implementation of any of the aspects, including but not limited to any implementations of any of the aspects, is applicable to all other aspects and implementations identified herein, including but not limited to any implementations of any of the aspects. Moreover, any of the features of an implementation of the various aspects, including but not limited to any implementations of any of the aspects, is independently combinable, partly or wholly with other implementations described herein in any way, e.g., one, two, or three or more implementations may be combinable in whole or in part. Further, any of the features of an implementation of the various aspects, including but not limited to any implementations of any of the aspects, may be made optional to other aspects or embodiments. Any aspect or implementation of a method can be performed by a system or apparatus of another aspect or implementation, and any aspect or implementation of a system or apparatus can be configured to perform a method of another aspect or implementation, including but not limited to any embodiments of any of the aspects. In addition, where disclosed as a method, systems and methods according to present principles encompass non-transitory computer readable media containing instructions which when run on a computing environment can perform said method. Moreover, when disclosed as a non-transitory computer readable medium comprising or containing instructions for performing a method, systems and methods according to present principles encompass the underlying method itself or themselves.

Advantages of the implementations and aspects may include, in certain implementations, one or more of the following. Problems that may be solved by the decision-support application/functionality may include, but are not limited to: missed boluses, overcorrection for hypoglycemia or hyperglycemia, early-morning hypoglycemia, meal over bolusing, hypoglycemia or hyperglycemia upon waking, pre-meal hyperglycemia or hypoglycemia, postmeal hyperglycemia/hypoglycemia, nocturnal hyperglycemia/hypoglycemia, hyperglycemia shortly after going to bed, excessive glycemic variability, hyperglycemia during or after exercise, hypoglycemia during or after exercise, as well as possible pump problems affecting insulin delivery. Systems and methods according to present principles may further provide guidance on food type and quantity, including based on exercise or other history, insulin dose recommendations based on history and other input variables, prompts on unexpected or repeated patterns, or the like. Systems and methods according to present principles may employ non-glucose information like meal size, exercise, stress, etc., as inputs to fuzzy logic or other dose determinations or glucose predictions. Systems and methods according to present principles may provide model-based prediction of glucose or control methods. Systems and methods according to present principles allow real-time knowledge of individual lifestyle factors, e.g., insulin sensitivity, to enable better insulin dose calculations and decisions.

Other advantages will be understood from the description that follows, including the figures and claims.

This Summary is provided to introduce a selection of concepts in a simplified form. The concepts are further described in the Detailed Description section. Elements or steps other than those described in this Summary are possible, and no element or step is necessarily required. This Summary is not intended to identify key features or essential features of the claimed subject matter, nor is it intended for use as an aid in determining the scope of the claimed subject matter. The claimed subject matter is not limited to implementations that solve any or all disadvantages noted in any part of this disclosure.

## DEFINITIONS

In order to facilitate an understanding of the preferred embodiments, a number of terms are defined below.

The term "analyte" as used herein generally relates to, without limitation, a substance or chemical constituent in a biological fluid (for example, blood, interstitial fluid, cerebral spinal fluid, lymph fluid or urine) that can be analyzed. Analytes can include naturally occurring substances, artificial substances, metabolites, and/or reaction products. In some embodiments, the analyte for measurement by the sensor heads, devices, and methods is glucose. However, other analytes are contemplated as well, including but not limited to acarboxyprothrombin; acylcarnitine; adenine phosphoribosyl transferase; adenosine deaminase; albumin; alpha-fetoprotein; amino acid profiles (arginine (Krebs cycle)), histidine/urocanic acid, homocysteine, phenylalanine/tyrosine, tryptophan); andrenostenedione; antipyrine; arabinitol enantiomers; arginase; benzoylecgonine (cocaine); biotinidase; biopterin; c-reactive protein; carnitine; carnosinase; CD4; ceruloplasmin; chenodeoxycholic acid; chloroquine; cholesterol; cholinesterase; conjugated 1-β hydroxy-cholic acid; cortisol; creatine kinase; creatine kinase MM isoenzyme; cyclosporin A; d-penicillamine; de-ethylchloroquine; dehydroepiandrosterone sulfate; DNA (acetylator polymorphism, alcohol dehydrogenase, alpha 1-antitrypsin, cystic fibrosis, Duchenne/Becker muscular dystrophy, analyte-6-phosphate dehydrogenase, hemoglobin A, hemoglobin S, hemoglobin C, hemoglobin D, hemoglobin E, hemoglobin F, D-Punjab, beta-thalassemia, hepatitis B virus, HCMV, HIV-1, HTLV-1, Leber hereditary optic neuropathy, MCAD, RNA, PKU, Plasmodium vivax, sexual differentiation, 21-deoxycortisol); desbutylhalofantrine; dihydropteridine reductase; diptheria/tetanus antitoxin; erythrocyte arginase; erythrocyte protoporphyrin; esterase D; fatty acids/acylglycines; free β-human chorionic gonadotropin; free erythrocyte porphyrin; free thyroxine (FT4); free tri-iodothyronine (FT3); fumarylacetoacetase; galactose/gal-1-phosphate; galactose-1-phosphate uridyltransferase; gentamicin; analyte-6-phosphate dehydrogenase; glutathione; glutathione perioxidase; glycocholic acid; glycosylated hemoglobin; halofantrine; hemoglobin variants; hexosaminidase A; human erythrocyte carbonic anhydrase I; 17-alpha-hydroxyprogesterone; hypoxanthine phosphoribosyl transferase; immunoreactive trypsin; lactate; lead; lipoproteins ((a), B/A-1, β); lysozyme; mefloquine; netilmicin; phenobarbitone; phenytoin; phytanic/pristanic acid; progesterone; prolactin; prolidase; purine nucleoside phosphorylase; quinine; reverse tri-iodothyronine (rT3); selenium; serum pancreatic lipase; sissomicin; somatomedin C; specific antibodies (adenovirus, anti-nuclear antibody, anti-zeta antibody, arbovirus, Aujeszky's disease virus, dengue virus, Dracunculus medinensis, Echinococcus granulosus, Entamoeba histolytica, enterovirus, Giardia duodenalisa, Helicobacter pylori, hepatitis B virus, herpes virus, HIV-1, IgE (atopic disease), influenza virus, Leishmania donovani, leptospira, measles/mumps/rubella, Mycobacterium leprae, Mycoplasma pneumoniae, Myoglobin, Onchocerca volvulus, parainfluenza virus, Plasmodium falciparum, poliovirus, Pseudomonas aeruginosa, respiratory syncytial virus, rickettsia (scrub typhus), Schistosoma mansoni, Toxoplasma gondii, Trepenoma pallidium, Trypanosoma cruzi/rangeli, vesicular stomatis virus, Wuchereria bancrofti, yellow fever virus); specific antigens (hepatitis B virus, HIV-1); succinylacetone; sulfadoxine; theophylline; thyrotropin (TSH); thyroxine (T4); thyroxine-binding globulin; trace elements; transferrin; UDP-galactose-4-epimerase; urea; uroporphyrinogen I synthase; vitamin A; white blood cells; and zinc protoporphyrin. Salts, sugar, protein, fat, vitamins, and hormones naturally occurring in blood or interstitial fluids can also constitute analytes in certain embodiments. The analyte can be naturally present in the biological fluid, for example, a metabolic product, a hormone, an antigen, an antibody, and the like. Alternatively, the analyte can be introduced into the body, for example, a contrast agent for imaging, a radioisotope, a chemical agent, a fluorocarbon-based synthetic blood, or a drug or pharmaceutical composition, including but not limited to insulin; ethanol; cannabis (marijuana, tetrahydrocannabinol, hashish); inhalants (nitrous oxide, amyl nitrite, butyl nitrite, chlorohydrocarbons, hydrocarbons); cocaine (crack cocaine); stimulants (amphetamines, methamphetamines, Ritalin, Cylert, Preludin, Didrex, PreState, Voranil, Sandrex, Plegine); depressants (barbiturates, methaqualone, tranquilizers such as Valium, Librium, Miltown, Serax, Equanil, Tranxene); hallucinogens (phencyclidine, lysergic acid, mescaline, peyote, psilocybin); narcotics (heroin, codeine, morphine, opium, meperidine, Percocet, Percodan, Tussionex, Fentanyl, Darvon, Talwin, Lomotil); designer drugs (analogs of fentanyl, meperidine, amphetamines, methamphetamines, and phencyclidine, for example, Ecstasy); anabolic steroids; and nicotine. The metabolic products of drugs and pharmaceutical compositions are also contemplated analytes. Analytes such as neurochemicals and other chemicals generated within the body can also be analyzed, such as, for example, ascorbic acid, uric acid, dopamine, noradrenaline, 3-methoxytyramine (3MT), 3,4-Dihydroxyphenylacetic acid (DOPAC), Homovanillic acid (HVA), 5-Hydroxytryptamine (5HT), and 5-Hydroxyindoleacetic acid (FHIAA).

The term "calibration" as used herein generally relates without limitation to the process of determining the relationship between sensor data and corresponding reference data, which can be used to convert sensor data into meaningful values substantially equivalent to the reference data, with or without utilizing reference data in real time. In some embodiments, namely, in continuous analyte sensors, calibration can be updated or recalibrated (at the factory, in real time and/or retrospectively) over time as changes in the relationship between the sensor data and reference data occur, for example, due to changes in sensitivity, baseline, transport, metabolism, and the like.

The terms "calibrated data" and "calibrated data stream" as used herein generally relate without limitation to data that has been transformed from its raw state (e.g., digital or analog) to another state using a function, for example a conversion function, to provide a meaningful value to a user.

The term "algorithm" as used herein generally relates without limitation to a computational process (for example, programs) involved in transforming information from one state to another, for example, by using computer processing. In implementations described here, algorithms may implement decision-support application/functionality, which takes input from sensors, computer applications, or user input, and converts the same into outputs rendered to a user on a user interface or to other devices.

The term "sensor" as used herein generally relates without limitation to the component or region of a device by which an analyte can be quantified.

The terms "glucose sensor" generally relates without limitation to any mechanism (e.g., enzymatic or non-enzymatic) by which glucose can be quantified. For example, some embodiments utilize a membrane that contains glucose oxidase that catalyzes the conversion of oxygen and glucose to hydrogen peroxide and gluconate, as illustrated by the following chemical reaction:

$$\text{Glucose} + O_2 \rightarrow \text{Gluconate} + H_2O_2$$

Because for each glucose molecule metabolized, there is a proportional change in the co-reactant $O_2$ and the product $H_2O_2$, one can use an electrode to monitor the current change in either the co-reactant or the product to determine glucose concentration.

The terms "operably connected" and "operably linked" as used herein generally relate without limitation to one or more components being linked to another component(s) in a manner that allows transmission of signals between the components. For example, one or more electrodes can be used to detect the amount of glucose in a sample and to convert that information into a signal, e.g., an electrical or electromagnetic signal; the signal can then be transmitted to an electronic circuit. In this case, the electrode is "operably linked" to the electronic circuitry. These terms are broad enough to include wireless connectivity.

The term "variation" as used herein generally relates without limitation to a divergence or amount of change from a point, line, or set of data. In one embodiment, estimated analyte values can have a variation including a range of values outside of the estimated analyte values that represent a range of possibilities based on known physiological patterns, for example.

The terms "physiological parameters" and "physiological boundaries" as used herein generally relate without limitation to the parameters obtained from continuous studies of physiological data in humans and/or animals. For example, a maximal sustained rate of change of glucose in humans of about 4 to 5 mg/dL/min and a maximum acceleration of the rate of change of about 0.1 to 0.2 mg/dL/min$^2$ are deemed physiologically feasible limits; values outside of these limits would be considered non-physiological. As another example, the rate of change of glucose is lowest at the maxima and minima of the daily glucose range, which are the areas of greatest risk in patient treatment, and thus a physiologically feasible rate of change can be set at the maxima and minima based on continuous studies of glucose data. As a further example, it has been observed that the best solution for the shape of the curve at any point along a glucose signal data stream over a certain time period (for example, about 20 to 30 minutes) is a straight line, which can be used to set physiological limits. These terms are broad enough to include physiological parameters for any analyte.

The term "measured analyte values" as used herein generally relates without limitation to an analyte value or set of analyte values for a time period for which analyte data has been measured by an analyte sensor. The term is broad enough to include data from the analyte sensor before or after data processing in the sensor and/or receiver (for example, data smoothing, calibration, and the like).

The term "estimated analyte values" as used herein generally relates without limitation to an analyte value or set of analyte values, which have been algorithmically extrapolated from measured analyte values.

As employed herein, the following abbreviations apply: Eq and Eqs (equivalents); mEq (milliequivalents); M (molar); mM (millimolar) μM (micromolar); N (Normal); mol (moles); mmol (millimoles); μmol (micromoles); nmol (nanomoles); g (grams); mg (milligrams); μg (micrograms); Kg (kilograms); L (liters); mL (milliliters); dL (deciliters); μL (microliters); cm (centimeters); mm (millimeters); μm (micrometers); nm (nanometers); h and hr (hours); min. (minutes); s and sec. (seconds); °C (degrees Centigrade).

The phrase "continuous glucose sensor" as used herein generally relates without limitation to a device that continuously or continually measures the glucose concentration of a bodily fluid (e.g., blood, plasma, interstitial fluid and the like), for example, at time intervals ranging from fractions of a second up to, for example, 1, 2, or 5 minutes, or longer.

The phrases "continuous glucose sensing" or "continuous glucose monitoring" as used herein generally relate without limitation to the period in which monitoring of the glucose concentration of a host's bodily fluid (e.g., blood, serum, plasma, extracellular fluid, tears etc.) is continuously or continually performed, for example, at time intervals ranging from fractions of a second up to, for example, 1, 2, or 5 minutes, or longer. In one exemplary embodiment, the glucose concentration of a host's extracellular fluid is measured every 1, 2, 5, 10, 20, 30, 40, 50 or 60 seconds.

The term "substantially" as used herein generally relates without limitation to being largely but not necessarily wholly that which is specified, which may include an amount greater than 50 percent, an amount greater than 60 percent, an amount greater than 70 percent, an amount greater than 80 percent, an amount greater than 90 percent, or more.

The terms "processor" and "processor module," as used herein generally relate without limitation to a computer system, state machine, processor, or the like, designed to perform arithmetic or logic operations using logic circuitry that responds to and processes the basic instructions that drive a computer. In some embodiments, the terms can include ROM and/or RAM associated therewith.

The terms "decision-support application" and "decision-support application/functionality," as used herein generally relate without limitation to algorithms that use sensor data and/or other data, e.g., user-entered data, derived data, or data from other applications or sensors, to provide a user prompt on a display and/or a command to a mechanical device.

The term "distinct" in regard to variables or parameters as used herein generally relate without limitation to a parameters and/or variables that are independent and do not rely one upon the other. Conversely, the term "related" in regard to variables or parameters as used herein generally relates without limitation to parameters and/or variables that depend in some way on each other or are derivable from each other. For example, a sensor signal time derivative is related to a sensor signal, while a user's gender and current analyte concentration would be considered distinct. It is noted here, however, that multiple parameters used in decision-support application/functionality may involve a single act or event, e.g., the same may concern a timing and duration of exercise. The term "independent" is used in the same way as "distinct", and similarly "dependent" is used in the same way as "related", although "independent" may also refer to variables used in a function, wherein the output of the function is a "dependent" variable, with the dependency based on the underlying independent variables.

The term "insulin sensitivity" as used herein generally relates without limitation to the relationship between how much insulin needs to be produced in order to deposit a certain amount of glucose. It is a physiologic measure everyone has, and is not limited to diabetes. The same may vary throughout a person's day, e.g., and may be driven by hormones, activity, and diet. It may further vary throughout a person's life, and may be driven by, e.g., illness, weight, obesity, and so on. It is a general measure, e.g., like weight, blood pressure, heart rate, and so on. There are healthy ranges for insulin sensitivity, as well as unhealthy ranges. In diabetes management, users should generally know their insulin sensitivity factor (ISF) when making decisions about boluses. The term ISF is sometimes used interchangeably with "correction factor" (CF). For example, a typical calculation users may be required to perform may be, e.g., "if my blood glucose is too high by 100 mg/dL, how many units of insulin do I need to take to correct the high and bring my blood glucose down by that 100 mg/dL?" Many users use a default CF of 1:50, which means that one unit of insulin will reduce blood glucose by 50 mg/dL. The determination of insulin sensitivity, as well as the determination of other types of sensitivities, are discussed in greater detail below, but here it will be noted that knowledge of insulin sensitivity may be based on, e.g., real-time analysis of data from CGM, activity monitors, and insulin pump data, as well as on data from retrospective analysis of such sensors as well as data from, e.g., an electronic health record. Other factors that may bear on insulin sensitivity or ISF may include correlations with time of day, pain, and/or exercise; heart rate variability, stroke volume, other cardiovascular health related to metabolic issues; ability to distribute insulin; temperature; insulin type, based on insulin sensitivity measurements, profiles, peaks, time between peaks; atmospheric pressure (thus "airplane mode" may be an input); whatever activity affects the patient or user the most; and so on.

The term "insulin resistance" as used herein generally relates without limitation to a medical condition in which the cells in a person's body cannot make proper use of insulin for the normal processes of cells importing glucose, or other metabolites, from the bloodstream. Insulin resistance reduces insulin sensitivity. While everyone has an insulin sensitivity, only certain individuals suffer from having insulin resistance.

The term "lifestyle factors" generally refers without limitation to quantitative or qualitative (but in some way convertible to quantitative) parameters that are generally not measured directly with a physiological sensor but which are related to disease management. In some cases the same relates to a trend or recurring event, however minor, that systems and methods according to present principles may determine and use in providing a therapy prompt to a user or in changing or altering a therapy prompt to a user. However, trend information need not necessarily correspond to a pattern, although some patterns will constitute trend information. In some implementations, a lifestyle factor may be equated to the correlative parameter discussed elsewhere. Lifestyle factors (also termed "lifestyle context") may be related to certain quantities that are physiological, e.g., insulin sensitivity, but may also be related to more external parameters, such as sleep sensitivity, meal sensitivity, exercise sensitivity, and so on. In other words, lifestyle factors are generally quantitatively determinable, but in most cases are not directly measured by a sensor.

The term "state" and "state model" generally refer without limitation to a data structure useful for modeling a patient for purposes of, e.g., decision-support. Generally a state model of a patient envisions the patient as occupying one of a plurality of states, the states dependent on various lifestyle factors and clinical factors. As a specific example, the state of a patient may correspond to a current insulin sensitivity profile. The plurality of states or state model may then be employed in combination with a real-time input, e.g., time, calendar, CGM glucose value, rate of change, and the like, in order to provide a therapy prompt to the user supporting a therapeutic decision. In one implementation a number of diabetes decision states are defined by one or more highly correlative parameters, which can be lifestyle parameters, and which can be selected by the user through a user interface or learned over time via machine learning and/or cloud analytics.

Exemplary embodiments disclosed herein relate to the use of a glucose sensor that measures a concentration of glucose or a substance indicative of the concentration or presence of another analyte. In some embodiments, the glucose sensor is a continuous device, for example a subcutaneous, transdermal, transcutaneous, non-invasive, intraocular and/or intravascular (e.g., intravenous) device. In some embodiments, the device can analyze a plurality of intermittent blood samples. The glucose sensor can use any method of glucose measurement, including enzymatic, chemical, physical, electrochemical, optical, optochemical, fluorescence-based, spectrophotometric, spectroscopic (e.g., optical absorption spectroscopy, Raman spectroscopy, etc.), polarimetric, calorimetric, iontophoretic, radiometric, and the like.

The glucose sensor can use any known detection method, including invasive, minimally invasive, and non-invasive sensing techniques, to provide a data stream indicative of the concentration of the analyte in a host. The data stream is typically a raw data signal that is used to provide a useful value of the analyte to a user, such as a patient or health care professional (HCP, e.g., doctor, physician, nurse, caregiver), who may be using the sensor.

Although much of the description and examples are drawn to a glucose sensor capable of measuring the concentration of glucose in a host, the systems and methods of embodiments can be applied to any measurable analyte. Some exemplary embodiments described below utilize an implantable glucose sensor. However, it should be understood that the devices and methods described herein can be applied to any device capable of detecting a concentration of analyte and providing an output signal that represents the concentration of the analyte.

In some embodiments, the analyte sensor is an implantable glucose sensor, such as described with reference to U.S. Patent 6,001,067 and U.S. Patent Publication No. US-2011-0027127-A1. In some embodiments, the analyte sensor is a transcutaneous glucose sensor, such as described with reference to U.S. Patent Publication No. US-2006-0020187-A1. In yet other embodiments, the analyte sensor is a dual electrode analyte sensor, such as described with reference to U.S. Patent Publication No. US-2009-0137887-A1. In still other embodiments, the sensor is configured to be implanted in a host vessel or extracorporeally, such as is described in U.S. Patent Publication No. US-2007-0027385-A1.

The following description and examples describe the present embodiments with reference to the drawings. In the drawings, reference numbers label elements of the present embodiments. These reference numbers are reproduced below in connection with the discussion of the corresponding drawing features.

Systems and methods according the present principles provide ways to incorporate "decision-support" in analyte monitoring systems, and in particular continuous glucose monitoring systems. In one implementation, decision-support can be provided by its own application or algorithm, which generally runs alongside a CGM application. In another implementation, decision-support can be implemented by additional programming/instructions added to an existing application, e.g., a CGM application. For this reason, in this specification, the decision-support provided is generally referred to as decision-support application/functionality.

Certain exemplary aspects are shown in Fig. 1. In the figure, a system 101 is illustrated in which a patient 102 wears a sensor 104, and the sensor transmits measurements using sensor electronics 106. More detailed aspects of the sensor itself and sensor electronics are described below with respect to Figs. 30 and 31.

The sensor electronics 106 may transmit measurements to a smart device 108 or to a dedicated receiver 110. The smart device 108 may be embodied by a combination of a smart phone and a smart watch, where, e.g., calculations and computations are performed on the smart phone and results transmitted to the smart watch for display and confirmation, e.g., to accept or deny the recommendation. Of course, it will be understood that the smart watch can perform calculations as well, and recommendations and other user therapy prompts may be displayed on the smart phone.

The smart device 108 or the dedicated receiver 110 generally have the capability of determining and displaying a clinically useful value of an analyte concentration. Each of these elements 106, 108, or 110, or a combination of the same, may also provide the decision-support application/functionality (abbreviated DS A/F in Figs. 1 - 3) through respective modules 112a-112c. The modules may each provide the application/functionality, or only one may provide the application/functionality, or the modules may interoperate so as to provide the application/functionality. In the system shown as system I, a smart device 108 works with the sensor electronics 106 to provide the functionality. In the system shown as system II, a dedicated receiver 110 works with the sensor electronics 106 to provide the functionality. In the system shown as system III, a smart device 108 and a dedicated receiver 110 works with the sensor electronics 106 to provide the functionality. While it is possible to provide decision-support application/functionality on sensor electronics 106 alone, typically the same does not have access to various lifestyle factors and other data often used in decision-support.

In many situations, a smart device 108 will work with signals received from sensor electronics 106 to provide the decision-support application/functionality. Signals may be transmitted in various ways, including through the use of Bluetooth®, NFC, and other wireless protocols. The smart device 108 will typically also perform calibration steps, although certain calibration aspects may also be performed on the sensor electronics 106.

In variations of the above, a module of the decision-support application/functionality may also be provided on a follower device 114, where a follower device is a device of a caregiver or relative/friend of the user, and where the follower device receives certain data related to the user, e.g., which data is often used to remind the user to take certain actions.

Measurements or other data may be transmitted from the dedicated receiver or smart watch to server 115 and/or a HCP 117, or to the follower device 114 as noted above, e.g., for notification, authorization, confirmation, approval, or for other purposes.

Referring to the system 150 of Fig. 2, the decision-support application/functionality module, which may be implemented on one device or across multiple devices, may be more specifically embodied by an analyte monitoring and display application 118, also termed a CGM app, which has incorporated decision-support functionality 120. For example, the decision-support application/functionality 120 may be a routine or subroutine running within the analyte monitoring and display application 118. Conversely, and referring to the application/functionality module 200 of Fig. 3, the same may be embodied by a decision-support application 124, e.g., running on a smart phone or other smart device, with incorporated monitoring/display functionality 126, where the monitoring/display functionality 126 accesses various communications capabilities of the smart device (for monitoring sensor signals via a sensor electronics) as well as user interface functionality of the smart device (for display purposes).

In yet another implementation, and referring to the system 250 of Fig. 4, the decision-support application/functionality module may be embodied by a decision-support application 128 running alongside a monitoring/display application or functionality 130. In this case, the monitoring/display application or functionality 130 may provide, through an appropriate API, inputs to the decision-support application 128, and the application 128 may analyze the received data to provide decision-support functionality, which may then be output to the monitoring/display application/functionality 130, again through the appropriate API.

In yet another implementation, as shown by the system 300 of Fig. 5, the application/functionality module may be embodied to provide output to a mechanical device 136, e.g., a pen or a pump. In this case the system 300 may include a decision-support application 132 running on a pump drive controller or even as part of a bolus calculator. The decision-support application 132 may be configured with appropriate output functionality 134 so as to provide an appropriate signal to the mechanical device 136, e.g., through a wired or wireless link 138. In this implementation as well, the decision-support application 132 will typically provide an output to a display, to provide a user prompt or notification of decision-support therapy recommendations. The same may further ask for confirmation prior to dosing.

In a particular variation of an implementation where output is provided to a mechanical device 136, e.g., a pen or a pump, a problem may be addressed wherein a person with diabetes may have to manually set the required dose. In particular, users with impaired vision or dexterity issues may have problems setting the required dose, and may in some cases even set an incorrect dose. To address these issues, and as noted above, the system 300 may provide output to a mechanical device 136 such as a pen or a pump, and in particular set the required dose as calculated by, e.g., insulin on board and CGM readings. The system 300 may set the required dose, i.e., the dose calculated to be correct at the time of calculation. An audible, visual, or vibratory alert may then be provided on the pen and/or on the CGM device to alert the user when it is time to dose. In a particular implementation, if the user does not dose at the given time, or within a range of time surrounding the given time, e.g., one or two minutes, the system 300 may recalculate the required dose on a periodic basis, e.g., every five minutes, and provide an updated required dose to the mechanical device 136, until insulin is no longer required, or until the patient performs the injection of the insulin.

However and wherever the decision-support application/functionality is situated, the same is tuned or adapted in such a way as to support user-desired functionality, where the user-desired functionality is defined by one or more of a number of aspects. Generally the user-desired functionality is not the sole determining factor of the tuning or adapting of the decision-support application/functionality, although in some cases it may be, or it may be a dominant factor. While numerous examples are given in the example section below, a summary of exemplary user-desired functionality is provided here. The user-desired functionality may be defined by, e.g., a goal to be attained, e.g., a lifestyle goal, e.g., "I want to feel more alert during the day" or "I want to wake up at 100 mg/dL". The user-desired functionality may be, in addition or alternatively, defined by a problem to be solved, e.g., "I want lower variability during the day". The user-desired functionality may also be defined by a decision to be made, e.g., "What should I do before I take a long road trip?".

In some cases the user-desired functionality is not provided by the patient but is inferred by the decision-support application/functionality itself. For example, a user, experiencing a low (i.e., hypoglycemia) may begin to increase usage of their device, e.g., become more interactive with a smart device, because they may become worried about their situation and desire additional data. The user may not even be consciously aware of this tendency, but the system may determine this pattern or trend and translate the same into a goal or problem to be addressed using the decision-support application/functionality according to present principles. As described in greater detail below, systems and methods according to present principles may be employed to determine and use as an input user cognitive impairment, even if the user is unaware of the impairment.

In the same way, the user may not be aware of aspects of their insulin sensitivity or insulin resistance, but the system can become aware of the same by pattern analysis, and can use the determined pattern to create a goal or problem to be addressed using the decision-support application/functionality. Put another way, if the system determines data, such as a pattern or trend that the user is not cognitively aware of, the system can use the determined pattern or trend to provide a user prompt relevant to decision-support, e.g., a therapy recommendation.

In yet another implementation, systems and methods according to present principles may have one or more default goals that may be immediately employed in treatment of a user, or from which the user may select. Typical default goals may concern reduced glycemic variability or the like.

In yet a further implementation, systems and methods according to present principles may provide, as default user-desired functionality, e.g., goals or problems to be addressed, goals or problems encountered by similar users, e.g., users with similar demographic characteristics, and so on.

The tuning or adapting according to the user-desired functionality may include tuning or adapting a user interface to support inputs or entered data to the decision-support application/functionality, tuning or adapting outputs from the decision-support application/functionality, tuning or adapting processing of the decision-support application/functionality, or tuning or adapting any combination of these.

In general systems and methods according to present principles implementing decision-support application/-functionality conduct automated analyses of data to identify patterns and insights, translate that into knowledge specific to a user's needs and goals, and finally enable actions that produce the outcomes required to reach the goals. A goal of the system may be to induce changes in user's behavior that reduce the occurrence of an undesired or unanticipated event. Systems and methods according to present principles may employ data mining tools and pattern identification and machine learning algorithms that can search and identify key patterns, trends, lifestyle factors, or the like, where the same are predictive of potential adverse events. In addition, fuzzy logic-based algorithms may be employed to translate behavioral and subjective or lifestyle information into actionable data. The algorithm architecture may be developed using a hierarchical approach, which enables new data to be integrated into the system effortlessly. For example, a more simple system may employ CGM alone, while more advanced systems may employ exercise, food, insulin, along with CGM. With each additional piece of data, the ability of the algorithms to predict unexpected adverse events generally improves.

Other ways of adapting or tuning the user-desired functionality are described below in connection with specific examples.

Fig. 6 illustrates one way of providing a decision-support application/functionality according to present principles. This method is illustrated in the coordinate system 350 by a surface 140. The surface 140 is defined by one or more coordinates, of which three are shown (for a 3-D coordinate system). More or less axes may be provided depending on the number of input variables. In this method, the surface 140 represents a value dependent on three independent variables, disposed along axes 142, 144, and 146. In other words, the surface 140 represents a function f(X), where X is a vector having a dimensionality equal to the number of independent variables on which f(X) depends. In other words, the system's interaction with the user, for decision-support, is a function of the factors within the vector X, which may relate to lifestyle context, clinical context, or a number of other variables and parameters as will be described. In many cases, the system will have determined a pattern or trend or lifestyle factor, or in some cases a key dependence of a variable or parameter on a clinical variable of interest, that the user is not aware of, and then may use the determined pattern or trend or variable to provide decision-support, based on in some cases on one or more additional real-time inputs, e.g., analyte concentration, rate of change, or acceleration. The inputs will be described below, but here it is noted that in this context or in other implementations the same may be "fuzzy", e.g., categorized and processed within categories by appropriate algorithms or lookup tables, or alternatively the inputs may be crisp, and still in other cases may be a combination of the two.

In a special case of this way of providing decision-support application/functionality, insulin sensitivity (IS) may be used as a learned parameter within a decision-support application/functionality. In this case, the functional dependence may be f'(IS, **X')**, where **X'** is a vector constituting a set of independent variables which do not include insulin sensitivity. In many cases the parameters or variables on which insulin sensitivity is based are also within the vector **X**, although the converse is not necessarily true.

f'(IS,**X**') or IS may be determinable with the decision-support application/functionality where the same is connected to a mechanical device such as a pump. For example, the pump may employ a preprogrammed algorithm to learn insulin characteristics such as insulin sensitivity and/or insulin resistance, e.g., by learning analyte concentration behavior as a function of basal rate, by learning and analyte concentration response to various times and sizes of boluses, or via other means.

In a method according to this way of providing decision-support application/functionality, as illustrated by the flowchart 400 of Fig. 7, a function is determined on which tuning of the decision-support application/functionality may be based (step 148). The function may be determined analytically or, for most realistic systems, the functional dependence may be inferred heuristically. Data is received or determined (step 152) corresponding to the independent variables and the same is used in the function in an evaluation (step 154) to determine how to tune the decision-support application functionality to support the user-desired functionality. An output is then provided (step 156). The output may have not only its content but also its manner of user interaction, e.g., its "output user interface," dependent upon the decision-support application/functionality. And as noted above, the output may also be provided to a mechanical device such as a pump or pen.

A system 450 shown in Fig. 8 illustrates another way of providing a decision-support application/functionality. In this system, a number of inputs are illustrated, an exemplary one illustrated by input 158a. A number of outputs are also illustrated, an exemplary one illustrated by output 166a. A decision-support application 170 is illustrated, and the same may be implemented by equivalent functionality as described above, e.g., as a separate module or as a subroutine within an analyte monitoring or other such application. The decision-support application 170 has an input user interface 168 and an output user interface 172. The input and output user interfaces are not necessarily entirely related to displays on user devices, but also include considerations of how data is requested or received from a user (on the input side) and provided or transmitted to the user (on the output side), including transmissions to mechanical devices such as pens or pumps. Such devices are shown in the figure collectively as device 174. In other words, the input and output user interfaces also relate to how the user interacts with their device. As additional functionality, the input user interface may serve the purpose of translating from "human" accessible data to "machine" processable data, and in the same way the output user interface may serve the purpose of translating from machine processable data to human readable data. In certain implementations this may be thought of as analog-to-digital-to-analog, or qualitative-quantitative-qualitative.

The input user interface 168, and the output user interface 172, as well as the decision-support application 170 itself, may each be affected by certain parameters or variables as shown in the figure by the vertically-oriented elements. In particular, the input user interface 168 is shown as being affected or influenced by a number of input user interface affectors (DSI#), an exemplary one illustrated by decision-support input 160a. The output user interface is shown as being affected or influenced by a number of output user interface affectors, an exemplary one illustrated by decision-support input (DSO#) 164a. The decision-support application 170 is shown as being affected or influenced by a number of decision-support application affectors, an exemplary one illustrated by decision support input 162a.

While the above text provides a general way of describing where inputs may occur, both for content of the algorithm (inputs 158) and for formatting of the user interface and output interface (inputs 160, 162, and 164), it will be understood that in a practical implementation decision-support inputs (or affectors) need only occur once, and may only be a single input. For example, once the system determines a pattern of insulin sensitivity, the same may be used with appropriate inputs 158 to determine the content and format of outputs according to the user-desired functionality.

In the system 450, a decision-support application is tunable based on various inputs 158 as well as on the interfaces 168 and 172, as well as on the decision-support application itself 170, as the same are tuned based on inputs 160, 162, and 164. Importantly, while the decision-support application 170 has inputs 158 and outputs 166, the user interaction for decision-support based on those inputs and outputs can change, i.e., can be tuned, based on other inputs, and in particular on inputs 160, 162, and 164, and it is these inputs that implement the user-desired functionality, e.g., the lifestyle goal, problem to be solved, or issue to be addressed. It is noted that the parameters or variables constituting inputs 158 may overlap to a greater or lesser degree with those constituting inputs 160, 162, and 164, or the same may have no overlap. Similarly, inputs 160, 162, and 164, may be distinct or may have nonzero overlap.

The data within inputs 158 or outputs 166, or for that matter data constituting inputs 160, 162, and 164, can be data streams or data bursts, i.e., streamed data or data constituted by discrete files. As noted above, the decision-support application can be a separate application that runs alongside an analyte monitoring and display application, or alongside a bolus calculator application, or can constitute a bolus calculator application, or can constitute functionality within these other types of applications. The decision-support application can also constitute the only (high level) application running within a smart device, and may have analyte monitoring, display, and bolus calculator functionality embedded within.

Finally, it is noted that while the output user interface is generally a function of the inputs and/or the input user interface, the converse is not necessarily true. In other words, the output user interaction can be influenced by the input user interaction. For example, if it is detected that a user likes to give lots of data about themselves, the output is adjusted to provide such a user with a greater level of detail in the output data.

A method of use of the system of Fig. 8 is illustrated by the flowchart 500 of Fig. 9. A first step in the method is to set up the initial decision-support application/functionality (step 176). Initially, default settings may be employed, or as noted above a default goal may be used. The decision-support application/functionality is then tuned with appropriate inputs (step 178). These constitute the modification or tuning of the input user interface, the processing, and/or the output according to received or determined decision-support inputs or "affectors", also termed above "formatting data".

A next step is the accepting or receiving of entered or input data (step 180). This step constitutes the reception of the inputs 158, also termed "content data". In some implementations such data is entered or received, and in some cases selected or chosen, based on the decision-support inputs or affectors, and these are driven in turn by the user-desired functionality.

The processing of the decision-support application/functionality then takes place (step 182), which generally involves evaluation of the entered or input data in light of the application 170, as the same has been tuned by the decision-support inputs. A final step is to provide the output according to the evaluated data (step 184). The output may be provided to a display, to a mechanical device such as a pen or pump, and so on.

Figs. 10(A), 10(B), and 11(A)-12(B) illustrate other ways to accomplish decision-support application/functionality. Here, the host (user or patient) 186 is modeled as a parameterized host system 186'. To accomplish this parameterization, data may be employed which is simply available or which is specifically generated for this purpose. In particular, a series of input data parameters 188 leads to a series of output data parameters 190. By analysis of the dependence of the output data parameters 190 on the input data parameters 188, a parameterized model 186' of the host system can be created. In this way, future input data 188 may be used by the modeled host system 186' to determine optimized outputs 190', i.e., outputs according to the user-defined functionality, or rather outputs determined to be more effective for the user.

In more detail, and referring to the flowchart 550 of Fig. 11(A), in a first step a model of the patient is defined (step 192). This host system definition need not be performed for every user, but may simply involve determining a number of parameters which bear on decision support, either in general or with respect to that particular user. The system model of the patient is defined by a number of parameters, preferably two or more, that influence diabetes control. In many cases the system model of the patient will involve consideration of one or more sensitivities, which are described below in the context of correlative parameters of which the user is unaware.

In one implementation, a system and method according to present principles starts with a population model of glucose dynamics, e.g., related to interaction between different types of food, insulin and related factors (e.g., I/C ratio, ISF), exercise, stress, and other physiological and emotional states, as well as glucose. Such models may form the 'prior' models that are used for providing guidance to users. These models may run at the cloud level by gathering and consolidating information from many subjects, and which result in ball-park estimates or solutions.

Data may then be mined in the background for events and conditions that are specific to an individual. Such data may pertain to individual level devices, e.g., transmitters, display devices, smart watches, smart phones, and the like. The mining activity may employ pattern recognition, clustering/classification, and/or case-based reasoning to identify prior conditions and events that are specific to the individual, but may be somewhat different from the population model. The algorithms can also use manual inputs to create a list of typical and atypical cases that it can train on for individuals. The data from different devices on the body may be gathered by the display devices and uploaded to the cloud. The data may then be used to generate more specific and accurate insights, predictions and guidance information.

In this implementation, some of the models/algorithm architectures that may be used include algorithms and models that take a layered approach, e.g., where each additional data available is added to the model to improve its ability to classify or predict. For example, logistic regression models allow easily expanding variables within the model to help predict outcomes better when more information is available. Decision trees and neural networks may also be used, as may fuzzy logic models to interpret subjective information or user-entered information. Case based reasoning and expert systems may also be employed. Certain of the auxiliary signals or information that the algorithms may use include: demographic or anthropometric information, CGM or SMBG data including location of the device on the body, insulin information from pumps, pens, or by manual entry, food intake information from menus, phone apps, or by manual entry, activity (exercise) information from phones, bands, watches, or other body worn sensors, and data from other physiological sensors such as temperature, heart rate, respiratory rate, stress monitors, or manually-entered indicators of physiological state. In general, the decision support provided is dependent on the quality of CGM or other signal information that is available.

Returning to the flowchart 550, a next step is to determine the system parameters (step 194) defined in the first step so that context can then be applied to the system model to result in the user-defined functionality. This step involves specifying the defined system model parameters in such a way as to be specific for that given user. In some cases if the defined system model parameters uniquely determine the host system response to all potential inputs, then the defined and determined host system model parameterization is usable for decision-support at that point. Such determinations of system parameters may take place on the cloud, on a smart device, or on other devices as described here.

While in some cases this system model parameterization may be used per se to implement the decision-support application/functionality, more commonly a parameter or variable is identified that is highly correlative with outcomes, i.e., has a significant contribution to or significant correlation with an outcome. In the below description such a parameter or variable is termed a "correlative parameter". The correlative parameter is identified (step 196), and then the decision-support application/functionality modifies the user interaction, e.g., modifies an input or output interface based on the identified correlative parameter as well as on a real-time input (step 198).

As a particular example, the correlative parameter may be a sensitivity such as a food sensitivity, i.e., the user's glucose value is highly dependent on meal intake. In this case the modified input or output interface may be geared especially towards food intake or lack thereof. Other and more involved examples are provided below.

Referring to Fig. 11(B), the nature of the correlative parameter may vary. For example, a correlative parameter 202 may be one related to any lifestyle factor or context 204. For example, it may be determined that a user has a low glucose value on weekday mornings. In some cases, one, two or more such behavioral or situational parameters may be employed, along with a clinical parameter. For example, a clinical parameter such as average glucose may be employed with a behavioral or situational parameter of exercise. As another example, eating a meal (which is a real-time data input, e.g., corresponding to behavior, situation, lifestyle) and an existing associated food sensitivity will have a significant correlation to an outcome of a user therapy prompt, e.g., an output meal bolus. In some cases the lifestyle context will mix with a clinical context, e.g., a pattern of lows at night. As another example, the system performs pattern learning of mealtimes, and then relevant parameters (lifestyle or behavioral or situational) may be mealtime and the same used in combination with glucose level or glucose rate of change.

As another example, the correlative parameter 202 may correspond to a stratification 206. That is, the correlative parameter may be that the modeled host system bears a strong resemblance in parameters (both inputs and outputs) with a known stratification of patients (determined from 'big data' and/or cloud analytics), and in this case recourse is made to known aspects, features, and other data of the identified stratification in order to provide the decision-support application/functionality. Such stratifications may stratify patients into one of a plurality of predefined profiles based on the model parameters. Such stratifications may include factors such as, e.g., age, gender, weight, and so on. Predefined profiles may include values of parameters or settings for a particular stratification of such patients, including bolus calculator settings.

The correlative parameter 202 can in another implementation be a parameter or variable 210 over which the user or host has significant control. For example, if the host is an athlete, the host may have significant control over how much and whether they exercise. Accordingly, decision-support application/functionality may take account of convenient and easy access and ability to exercise when giving therapy recommendations and other user prompts. In this case, if such a user were to experience hyperglycemia, the decision-support application/functionality may include an exercise suggestion as an alternative to or in combination with a bolus, and such a therapy prompt is based on information the system knows about the user.

In many cases, the correlative parameter 202 is a calculated insight 212 of which the user was or is cognitively unaware. For example, a user may not be aware that they have significant overnight lows, but through machine learning the decision-support application/functionality can learn such patterns and provide therapy recommendations to treat such patterns, particularly where the user-defined functionality is a goal such as "better glucose control" or "lower glycemic variability".

In many cases the calculated insight corresponds to a perturbation sensitivity that the user is cognitively unaware of, or of which it is difficult for the user to determine. These include: sensitivities to foods (including considerations of food types, amounts, and timing of consuming); sensitivities to therapy (e.g., sensitivities to insulin or other diabetes medications, again including type, amount, and timing); sensitivity to exercise (again based on type, amount, and timing); sensitivity to sleep (again based on type, amount, and timing, as well as other sensitivities, and combinations of the above. Determinations of such sensitivities may be by analysis of sensor data, e.g., CGM sensor data, in combination with other data, e.g., that corresponding to meals, exercise, or sleep.

The correlative parameter can also correspond to an identified state 211, as discussed in greater detail below.

It will be understood that there may be overlaps in these types of correlative parameters as well. For example, a calculated insight may be a lifestyle factor. As another example, a patient may be stratified into a particular type of predefined profile, but machine learning may then be performed to personalize the decision-support application/functionality for the user, including for the purpose of sub classifying the user into a sub profile.

In a particular implementation of a parameterized model, and referring to Fig. 11(C), a patient may be modeled as having a number of states, and such states may be learned using a computing environment 201, which indicates a learned or indicated state model 203 having a number of states 205i therein. The computing environment 201 can learn the states over time, e.g., learning an aspect of a patient's insulin sensitivity, or such states can be indicated by the user, e.g., "I want to treat my diabetes aggressively." or a combination of the two can be used, particularly as additional details about the patient are learned over time.

In diabetes disease management, the states may be thought of as diabetes decision states, which are defined by one or more highly correlative parameters. For example, a diabetes lifestyle decision state can be defined by glucose and one or more highly correlative lifestyle parameters, which can be selected by the user using an appropriate user interface or learned over time, e.g., by machine learning and/or cloud analytics. Sub states 205'i may be defined and used to quantify various factors that go into therapeutic decision-making, e.g., sub states corresponding to lifestyle, clinical quantities, situational aspects, and device aspects. Sub sub states (not shown) may then be defined to further delineate states. For example, the lifestyle substate may be divided into sub sub states corresponding to exercise, illness, meal, and the like. The states and sub states drive the way the algorithm runs and/or presents or determines output because inputs can determine the state (and various levels of substates) associated with the user (where the same or other inputs also determined prior learning of such states and substates). A real-time input, e.g., CGM glucose value, time, calendar, GPS, or the like, can then drive the output in combination with the determined state, which is subsequently calculated based on the lifestyle and other factors. In some cases the state(s) associated with the user are states the user is in, e.g., based on lifestyle factors such as activity and meals, and in other cases the states may be associated with the user but are not a state the user is in themselves, e.g., where the device signal quality or confidence informs the state. In this case, the state is associated with the user because it is informed by the device situated within the user, but it is not necessarily related directly to user clinical or lifestyle factors.

Fig. 12(A) is a high-level flowchart 121 pertaining to the system of Fig. 11(C). In a first step, input data is received and in some cases undergoes a step of learning (step 123). This step, termed hereafter "step A", has overlap with other input learning steps discussed below, and generally includes steps of learning what a user means by an input, when an input is user entered. For example, if a user enters that he ate a large meal, the glucose response is learned and associated with what the user means by "large". In this case, a number of such glucose responses corresponding to user-entered "large" meals will be received and averaged to provide an average glucose response for a large meal.

A next step corresponds to learning or determining one or more states (step 125) based on the received input data (termed hereafter "step B"). For example, the above learning of what a "large" meal means for a patient may be used in combination with time of day data to determine a meal sub substate, which is in turn part of a lifestyle substate of the state model of the user. In some cases, in combination with location data (such that the system can receive data about, e.g., a particular restaurant), meal composition may be inferred. As another example, time of day data, calendar data, location data, and accelerometer data, or a sub combination of these, may be used to learn that a patient exercises at about the same time every week, and with the same approximate level of exertion.

The learned or determined state from step 125 need not pertain directly to user physiology or lifestyle. For example, the same may correspond to a device signal quality or level of confidence in measurement data. If the level of confidence is low, a provided decision-support prompt can in some configurations be more conservative or configured to aim for a target at the upper end of an acceptable range.

Based on the learned or determined state, a therapy prompt is calculated or determined and displayed, the therapy prompt providing an output supporting a decision for the user (step 127), and such is termed hereafter "step C". Generally the therapy prompt is based on at least one of the states determined in the state model of the user from step 125, as well as on a real-time input 131. In a particular example, step 127 could correspond to the calculation of a bolus calculator, which then results in a display to the user of a bolus to inject or to pump. Another exemplary result of step 127 may be an action for the user to take, e.g., to take a 30 minute walk. The latter example may be particularly pertinent for type II patients.

In an alternative but related implementation, shown in the flowchart 133 of Fig. 12(B), one or more real-time inputs 131 are considered as entering the decision-support application/functionality at step A (step 135). States are determined or learned (step 137), their determination or learning based on various inputs 139 received over time. A decision-support output is then calculated or otherwise determined (step 141), and a therapy prompt displayed.

With respect to providing decision support (e.g., a bolus amount, a suggestion of an action to take, and the like) with respect to a user-entered or device-detected occurrence X, or a set of factors Y, generally the factors known about the patient may be compared before a change is made (via user therapy prompt) and after a change is made. Such factors may be controls such as physiological parameters known about the patient, problems encountered, lifestyle factors, or the like. Such quantifications can be done on a "run to run" basis. The values before and after are quantified and their difference(s) determined.

However, for many current mathematical models including insulin sensitivity factors, there are often too many parameters necessary to vary, and so an exact solution is not developed. Accordingly, in these implementations it is beneficial to attempt to estimate a percentage change in all of the parameters, and then increase the accuracy of this percentage change on a "run to run" basis. For example, based on the same event over time, a determination is made as to whether the change is making the blood sugar go high or low, and what is the duration of the change. Here the change is the therapy prompt, e.g., bolus calculation. A "binning" technique may be employed to categorize the changes, e.g., whether the change caused the blood sugar to raise or lower by 30-50 mg/dL, by 10-30 mg/dL, and so on. A subsequent change may be made using this information to cause the patient to have their glucose level be directed towards the target range, and a quantification made as to, as a result of the subsequent change, is the patient in the target range more than they were previously. Once an optimal percentage is determined, the same may be employed as an optimal percentage change for a given state, where the state is defined by the lifestyle and other factors corresponding to the occurrence X, or to the set of factors Y. Once that occurrence or set of factors recurs, i.e., once the state recurs, the learned optimal percentage may be applied.

Where user therapy prompts are used as suggestions to a user for operation of a pump, the learned percentage changed and optimized is applied by the device in the determination of a bolus or basal rate.

In an implementation, a "usual" meal bolus is defined for a patient. The procedure above is performed, based on lifestyle parameters or factors as well as on a real-time input such as a CGM value, a time of day, a day of the week, or the like. The procedure above results in a percentage change to the usual meal bolus, and the same is applied as a multiplier to determine the resulting user therapy prompt. That is, the optimization of the basil/bolus parameters is conveniently applied in a "summary" fashion to the calculated meal bolus/basal rate, by application of a multiplier, as opposed to modifying the individual parameters themselves.

The above technique may be particularly useful with regards to states relating to health events, meals, and exercise. In one example, for a state of exercise, a particular percentage change may be calculated, and the same can be quantified into sub states of light workout, medium workout, and intense workout, with different percentage changes (to bolus or basal values) applying to each. Similar categorization into sub states of the meal state may be understood, and so on.

While insulin sensitivity is a useful quantity to profile (and define states for) in a user for diabetes decision support, other useful profiles/states include those corresponding to carbs on board, insulin on board, blood sugar, CGM glucose value, past CGM values on the same kind of day (weekend, weekday) at the same approximate time of day, exercise, exercise on board, and so on. A given parameterized model can be optimized based on values over time and variations determined and quantified of these above states.

As a specific example, where the decision-support application/functionality pertains to a bolus calculator, the incorporation of a user state generally leads to a modified content or presentation of the user therapy prompt. For example, if the system determines that the state is one of exercise, the decision-support application/functionality may determine that a calculated meal bolus should be reduced by a certain amount. In many cases a reduction or increase of a size of a bolus is calculated as a percentage of a preset meal bolus. Thus the decision-support application/functionality could determine that the bolus should be 90% of the usual. As another example, if the user is having a large meal, as opposed to a medium meal, the decision-support application/functionality could determine that the bolus should be 110% of the usual. As yet another example, if the rate of change of the glucose is considered, as is described in greater detail below in a bolus calculator implementation, the suggested meal bolus may be suitably modified.

Numerous benefits inure to the above implementation, including that, by having the determination of states occurring via machine learning and accessible to the user at any location, the user is provided ambulatory decision-making support for management of their disease. Processing can occur on, e.g., a smart phone, or in the cloud or via other server-based processing, or in some configurations can be performed by a combination of both. In any case, decision-support is then provided wherever the user may be, affording a portable solution for therapy assistance. In addition, the function of continuous glucose monitors is improved in that additional functionality is added that could not practically be done without the sensors and the processing functions disclosed. The definition and use of states in decision-support results in a technological improvement to the monitoring of the past.

Inputs which are then employed as applied to the host system parameterization, or state model, whether highly correlative or not, may constitute data in a number of forms. For example, such input or entered data may be crisp, disposed within categories, or "fuzzy". In some cases the input data form may depend on how the data is measured. For example, if the data is from a sensor such as a continuous glucose monitor, an accelerometer, or other such sensor, the data may be as "crisp" as the sensor is able to measure. Other data, such as meal data, is obtained from user input into a smart device, and for the sake of user convenience, is more easily entered as fuzzy or within categories, e.g., small, medium, or large, with further categorizations (or "fuzzifications") corresponding to, e.g., relative amounts of carbohydrates/fats/proteins. Similar data may be entered as "crisp" if the user is aware of the number of carbohydrates or other such data. The tuning may be based at least in part on whether the user provides precise or crisp data, approximate data, categorized data, and so on. For example, the tuning may differ if the user enters "3 carb units" versus "big meal". Initially, for fuzzy food inputs, a top of a target range of food sizes may be employed, with the system learning actual meal sizes over time. Similarly, the system can assume certain carbohydrate amounts for different meal sizes, and may use a conservative guess to start or as a seed value.

As noted above a user goal is often a very important factor in decision-support. Where a user goal has been specifically stated, or even if just a default goal is employed, such may also be translated into quantifiable criteria. For example, referring to the flowchart 600 of Fig. 13, a first step is the identification of a goal (step 209), and the subsequent translation of the same into quantifiable criteria (step 213). As noted above, the same may be a default goal or may be selected by the user from a set of common goals.

For example, if the user often encounters nighttime lows, a lifestyle goal may be to avoid nighttime lows, and to translate the same into quantifiable criteria, the goal may be translated into "maintain glucose levels above 90 mg/dL during sleep". The translated quantified goal may then be used within decision-support application/functionality, e.g., as a measure of success (step 215), and an appropriate output provided based at least in part on the translated quantified goal (step 217). For example, in an iterative process, a therapy recommendation to avoid nighttime lows may provide a user prompt to the user to eat a snack before bedtime. If, according to the quantified goal, the user still experiences nighttime lows, a subsequent user prompt, determined by the machine learning of the decision-support application/functionality, may increase the size of the suggested snack or may suggest the user exercise less prior to bedtime.

As another example, even a more vaguely-stated goal can be employed to affect the decision-support application/functionality. For example, if the user is a student, their "lifestyle" may be considered to have a major feature of "attending school daily", and a lifestyle goal appropriate to such a user may be to optimize their bolus calculator based on the lifestyle of being in school for a certain amount of time every day. In this case, quantifying such a goal may include providing not only different target ranges for school time versus home time but also different manners, frequencies, or timings of alerts and alarms between school time and home time.

Other goals may incorporate clinical aspects, e.g., a goal may be for the user to have a glucose value of 100 upon awakening, or to achieve a desired A1C value. These goals may be translated into quantifiable criteria using the desired glucose value or A1C value.

The flowchart 650 of Fig. 14 indicates ways of quantifying non-crisp inputs. In particular, upon reception of a non-crisp input (step 219), one way of quantifying the same is performing a step of natural language processing (step 221) on the input to determine a "crisp" equivalent. For example, if the user indicates qualitatively that they consumed a glass of orange juice, natural language processing may determine how many carbs are involved in a glass of orange juice, thus translating the input from non-crisp to crisp. Historical pattern data may also be employed in this regard. Similar such natural language processing may be employed to determine the crisp nutritional content of an input such as "I had a quarter pounder at McDonald's". Social networks and social media may also be employed (step 223) to give context to non-crisp inputs or to otherwise tighten down values pertaining to non-crisp inputs. Big data may also be employed (step 225) to give context and crisp values to non-crisp inputs. For example, population data may be employed to determine what a particular user means by a particular phrase or indication of a parameter, e.g., by analysis of cohort data, and the same may be employed along with any of the other techniques to render a crisp version of the input that will result in (step 227) quantifiable criteria that may be employed to influence decision-support.

Particular and other aspects of inputs are described below in the Inputs section.

However, prior to a general discussion of inputs, and referring to Fig. 15, particular types of relationships are shown, and a particular relationship is shown by the relationship 231 between a food sensitivity 233d and a meal bolus 235a. Other relationships may also be determined using systems and methods according to present principles, between inputs 233a-233e (only a sampling of such inputs are shown) and various outcomes or in particular outputs 235a-235e (again, only a sampling of such outputs are shown). Processing steps 237 are indicated to show how the correlative parameter, e.g., food sensitivity 233d, is used along with a real-time input (not shown) within the processing steps 237 to result in a therapy recommendation of a meal bolus 235a. Particular examples of these inputs and outputs are described below in the Examples section.

The identification of the correlative parameter and the lifestyle/situational parameter, as well as subsequent processing steps, can be performed in a number of ways. For example, the steps can include factors from various locations, including social networks, user-entered data, sensor data, data from user devices, population or big data, and so on. The processing and identification may include Bayesian analysis or the like, e.g., to identify strong connectors which are parameters or variables that bear a strong relationship to each other. Where multiple parameters are employed, the same may include multiple parameters relating to the same event, e.g., both intensity and duration of exercise. The processing and identification may include multiple steps, e.g., a first step of processing "internal data" for that patient, and a subsequent step of performing some processing in the cloud, with "big" data, e.g., using prepackaged subroutines and case-based reasoning. For example, case-based reasoning may be employed to determine what happened to the user in other like situations, or what happened to other like users in other like situations. As smart devices become even more computationally capable, much of the machine learning may happen on such devices. However, much of the current processing can happen in the cloud, and such processing may result in significant amounts of machine learning. In other words, the system learns how one element or "affector" X affects the patient, e.g., how anabolic exercise may impact insulin sensitivity. In this example, if a pump action was initiated with the bolus but did not consider the impact of exercise, and then the user were to go for a run, systems and methods according to present principles may detect this sequence of events and notify the user to "expect a drop" or may provide a recommendation such as "you should eat something now".

As noted above, a final step in this method is modifying the input or output interface based on the identified correlative parameter or state as well as on a real-time input. The real-time input may include a lifestyle or behavioral or situational parameter, e.g., a time of day, an event about to be undertaken as determined by a clock or calendar application or user input, glucose value, and so on. In other words, the machine/human interface is modified by the decision-support application/functionality, e.g., on the smart device, smart phone, smart watch, and/or as part of the input user interface or output user interface of any of these devices, based on the results of the above steps. For example, the output user interface, i.e., the type, format, and content of the output, may then depend on the lifestyle/situational context as well as on the parameterized model and/or the user-defined functionality. It should be noted that a specific user-defined functionality, e.g., a lifestyle goal, need not be employed in every implementation, although such may be advantageously used to guide certain therapy recommendations.

In modifying the machine human interface, just as inputs may be categorized or "fuzzified", outputs may be provided in categories or "fuzzifications" as well, and such is particularly true if the output is simply to be rendered on a user interface of a display. For example, if the therapy recommendation is to suggest that a user have a large glass of orange juice, or eat a medium apple, the user may find such a recommendation more useful than a recommendation to eat a certain amount of carbohydrates. Of course, if the output includes an output to a pump or pen, no such "fuzzification" is necessary and as much accuracy as possible is generally desired.

Exemplary outputs are as illustrated in Fig. 15, e.g., a recommendation of a type, amount, and timing of a meal bolus 235a, a recommendation of a type, amount, and timing of a correction bolus 235b, a recommendation of a type, amount, and timing of a meal 235c, a change in the user interface 235d, e.g., providing an alert or an alarm at a time determined to be effective for user, or a pre-event or situational decision 235e, e.g., a decision to be made before sleep, a meal, exercise, driving, activity, or the like. Other exemplary outputs include a permanent or temporary change to a basal rate setting, a recommendation for rescue carbs, or the like. Specific outputs, and the way the same may change according to the decision-support application/functionality, are discussed in the Outputs section below.

While various core methods have been described above with regard to providing decision-support application/functionality in a way that is supportive of user-defined functionality, it will be understood that the core methods need not be mutually exclusive. For example, various combinations of the core methods may be employed in a given implementation. In addition, multiple methods may be performed in parallel to determine optimal treatment decisions, and then the safest option chosen. That is, various algorithms may be employed as described above to use defined states, sensor data, user inputs, and other available information to calculate an optimal diabetes treatment decision. Multiple methods may be performed in parallel to calculate the optimal treatment decision, and then the most conservative or safest of the solutions may be provided to the user. For example, in times of sensor noise, either a filtered value or the raw sensor value may be used to calculate a current glucose estimate and subsequently a recommended insulin bolus. Doses could be calculated based on both sensor values in parallel and then the more conservative dose could be recommended. Another example is to use a glucose rate of change estimate based on either a trend calculated in the CGM algorithm or as a two-point difference of the most recent pair of glucose values, and the rate of change that provides the more conservative dose would be used.

## Inputs

A number of different types of inputs may be employed in systems and methods according the present principles. A set of exemplary such inputs are shown in the table of Fig. 17. The description of inputs immediately below gives certain exemplary uses to provide context for the input. However, other examples will be provided in the Examples section below. Such inputs, and the learning of inputs, generally relate to Step A of Figs. 12(A) and 12(B).

A number of exemplary inputs and notations about such inputs are given in the table. It will be understood that other inputs may also be employed, and that the notations given about the inputs are not necessarily limiting. For example, inputs may be noted as having a certain context, but in some cases the context may be thought of as overlapping certain categories. As another example of variations, certain inputs may be identified as user entered, but in some cases the same may be determined by a sensor or the like. For example, meal data may be determined by user input, but also by GPS, from pattern data or from an app such as a camera. As noted the decision-support application/functionality incorporates machine learning to increase the accuracy and appropriateness of therapy prompts over time. Meal data entered by the user, e.g., "small meal", "medium meal", and so on, may be "learned" by the system so that the decision-support application/functionality may better understand over time what the user considers a "small meal", and so on. Temporal patterns may be analyzed by the systems and methods to determine aspects of, e.g., meals and exercise; but users may also identify and quantify events they want to track, including those corresponding to meals and exercise.

In one alternative implementation, the algorithm may have just one "average" meal defined, but allow for a degree of automatic adjustment to define what is large or small for a user. In this implementation, the "average" meal may be defined for a population or for an individual user. This implementation may be more convenient to implement as only one meal size need be defined, and the same does not need to be changed for every meal. Rather, a multiplier may be applied, by the patient, by the HCP, or automatically, to assess if the meal is "average", "light", or "heavy/large". The algorithm may then automatically increase or decrease suggested insulin amounts according to the assessment. For example, an exemplary "multiplier" may be that a large meal is 1.5x the effect of the "average" meal, or that a small meal is 0.5 times the effect of the "average" meal. Such implementations may be particularly convenient for a user to implement, particularly where the user is not accustomed to carb counting. Extensions of such an idea to exercise and other applications will be understood.

Where multiple types of data are employed as inputs in a given algorithm, the multiple types may be related, e.g., may be a type, timing, and intensity of a variable, e.g., illness, exercise, meals, sleep, and so on, e.g., a duration and intensity of exercise.

In the table the inputs are divided into two categories: real-time data and non-real-time data. Referring also to Fig. 17, these categories of inputs 246 are then divided into subcategories as: data from sensors (248 and 252), user data 254 which may be user-entered data or data from other apps, e.g., from a suitable API, derived data, and other data. Some subcategories appear in both real-time data and non-real-time data. In any case, the inputs 246 then feed into a user device 256 which may be a dedicated receiver or smart phone, and more particularly into a decision-support application 260 disposed therein. Derived data 262 may be stored in the user device 256, and a display 258 may be provided with a user interface to display prompts to the user as well as for receiving user-entered data. The derived data 262 may be derived by a processor within the user device 256, and the derivation of the data may occur within the decision-support application/functionality or may occur outside of the same.

Referring back to the types of data illustrated in Fig. 16, the sub subcategories then describe specific types of data or are themselves categories. For example, a sub subcategory in real-time data is "physiological sensors" and these may be further broken into different types, e.g., CGM, SMBG sensors, body temperature sensors, skin conductivity sensors, hormone sensors, heart rate sensors, and the like. Besides physiological sensors, other sensor data include non-physiological sensors, such as those dealing with atmospheric pressure, accelerometers, GPS, sensors measuring aspects of the CGM system itself, temperature sensors, and the like. Such inputs may be provided from wearable devices/sensors, e.g., those available from an Apple Watch®, Fitbit®, or other wearable. Other devices providing inputs may include other mobile devices, including smart phones.

Sensors such as accelerometers and GPS may be conveniently used to provide data about exercise or activity performed by the user. In some cases GPS or other sensors may be employed to determine other types of activity data, e.g., if a user is driving, which may in turn inform how an output to a user may be displayed, e.g., on a smart watch versus on a smart phone.

In the particular case of an accelerometer, the same may be advantageously employed to detect sleep patterns or sleep quality as related to glucose control. Such may generally provide data related to position but also importantly to activity detection as well as duration and intensity. One potential type of accelerometer that may be advantageously employed is a three-axis accelerometer.

In some cases, the decision-support application/functionality may allow data to be entered about the start (or other marker) of a menstrual cycle. In this way, users may be able to track over several months the behavior of blood sugar at different times of the month with regard to the cycle. Of course, such an algorithm and data may also be used on its own to assist pregnancy efforts or if using the rhythm method for birth control.

Hormone sensors may provide another source of entered data, and in one example may also be a source of data related to the menstrual cycle. Other hormone sensors may include those that sense cortisone and/or epinephrine. Hormone sensors or the like may also be employed to provide a measure of "energy in" versus "energy out", particularly with regard to a parameterized system model of the patient.

Another subcategory of inputs in real-time data includes user-entered data, including goals, a problem to be solved, a desire for therapy, entered data about stress, emotion, or feelings, pain level data, sleep level data, data from a follower device, pump data, meal data, exercise data, blood glucose data from an external blood glucose meter, and so on.

User-entered data may include user estimates of glucose values, so that decision-support may be rendered in a way that helps the user to better correlate a perceived glucose value with a real one. For example, the decision-support application/functionality may be enabled to better assist the user in determining and discerning what different levels of hyperglycemia feel like. User-entered data may also include user demographic data such as age, gender, and so on. Such data may be conveniently obtained by, e.g., a swiping of the user's driver's license.

Where user-entered data includes exercise, systems and methods according to present principles may allow users to save specific workouts or events to allow patterns in post workout glycemic fluctuations to be analyzed and potentially found, resulting in appropriate automatic or manual therapy modifications to be made by the decision-support application/functionality. For example, users may save workouts such as "Cardio 1", "Cardio 2", "Get Swoll 1", "Get Swoll 2", and so on. Other gradations of exercise may include those pertaining to intensity, e.g., light, medium, hard, duration, e.g., 5 min., 10 min., 15 min., 20 min., 25 min., 30 min., one hour, and so on.

Goal data may include an indication of priorities in terms of glucose control, e.g., hypoglycemic versus hyperglycemic avoidance. Different control types may be provided as preprogrammed profiles that a user may choose in selecting a goal. For example, a user may choose a goal of hypo-minimizing control, hyper minimizing control, frequent versus infrequent correction boluses, and so on, so as to fit different treatment styles and goals.

In the same way, user feedback may also constitute an important input into decision-support. That is, the decision-support application/functionality may be modified based on user feedback, e.g., periodically querying the user as to what they would like to be different about their recent glycemic control, and providing options such as "I would like to reduce my number of nighttime hypos" or "I would prefer to not have to give correction boluses as often". Feedback may be prompted for in a convenient fashion to minimize annoyance for the user. For example, feedback may be provided by slider bars or other user interface elements convenient for user selection. Slider bars may also be provided for other user inputs, e.g., to indicate how much interaction is desired.

As noted above the decision-support application/functionality may be advantageously employed as part of a bolus calculator. Thus, user input may also include user input into such a bolus calculator. For example, the decision-support application/functionality may be rendered with a user interface that affords the user predetermined dose adjustments, e.g., +/- 0%, +/- 10%, +/- 20%, and so on. The dose adjustments presented may themselves depend on the CGM trend, e.g., negative dose adjustments may be provided in the case of a negative or falling glucose trend. The chosen adjustment would apply to the bolus dose calculated with or without any other trend adjustment. Alternatively, adjustments can be fuzzy. In yet another implementation, users can choose to customize the adjustment, within bounds. Over time, with data, the decision-support application/functionality may determine optimal adjustments based on this user-entered data (and other inputs), and with provider authorization (or alternatively without provider authorization/-confirmation), the adjustment options may be presented to the user and re-determined.

In a related implementation, rather than a percentage, rate of change data may be employed to increase/decrease the bolus value calculated by a fixed amount. In this way, the patient adds or subtracts enough insulin to cover that amount of glucose change from their meal bolus using their own insulin sensitivity/correction factor. In this implementation, initially the bolus calculation could be left unmodified, and the decision-support application/functionality may learn how much insulin needs to be added or subtracted based on rate of change as part of a case-based learning process. For example, in cases where glucose is rising or falling, rather than updating insulin sensitivity, the system may learn how far the glucose is from target and consider that to be the amount of glucose that should be accounted for. In this case a new insulin sensitivity is not learned, but rather the best insulin sensitivity estimate is used based on the other descriptors of the case, e.g., lifestyle factors, e.g., specific to an insulin sensitivity for breakfast with no exercise. As it may be desired to learn both how much to adjust insulin boluses based on rate of change and how insulin sensitivity changes for time of day, exercise, etc., systems and methods according to present principles may advantageously separate the two problems and only update insulin sensitivity when rates of change are relatively stable. Once it is learned how much the user should adjust their insulin based on rate of change, then such data may be pushed to the HCP, e.g., showing patterns of, e.g., how the user post-meal glucose is always around 30 mg/dL too high when they take a meal bolus and the trend arrow is 90 degrees up, and the HCP may be requested to approve the amount of insulin to add or subtract. Machine learning may also be used to determine if it is better to modify insulin boluses based on rate of change using a percent or a fixed amount, just in case such may vary patient-to-patient. Additional details of the use of rates of change data are provided below in an example.

Returning to the discussion of various inputs, inputs may include data about followers of the user, or data about other related users. This entered data may include data about social groupings, e.g., for the achievement of individual or group goals, and may further employ natural language processing to deduce objective and quantifiable desires and goals of the group or its members. Inputs may further include inputs from followers, e.g., in response to a query such as "What do you think is going on with your daughter?".

Another source of real-time data that is user entered includes meal data. Users enter meal data in a fuzzy or categorized fashion. The user may also enter meal data as a crisp input. Machine learning is employed to learn in an objective sense how a user categorizes the meal. For example, if a user terms a meal "small", in terms of what is entered into the decision-support application/functionality, the system may learn in a more quantitative or objective way what the user considers a "small" meal. The same is true for meals entered and termed as other sizes. The same is also true for user-entered data regarding meal composition. In other words, the system can learn what a user means when the user enters a statement of meal composition into the decision-support application/functionality. For example, if the user terms a meal "high carb", the system learns from the glucose response and data about delivered insulin (if available), in combination with the meal data, what the user considers "high carb".

Another subcategory of inputs in real-time data includes data from other applications or "apps", e.g., generally from a suitable API. Such apps may reside on the computing environment providing the decision-support application/-functionality, or may be in wired or wireless communication therewith. Such apps include: calendar apps, GPS apps, CGM apps, apps holding historical data or other information, SMBG apps, temperature monitor apps, Healthkit®, data from followers, e.g., from text messaging apps, bolus calculator apps, clock apps to provide time of day, apps indicating the elapsed time, so as to allow user prompts to be provided at optimum times, apps providing access to cloud data or "big" data, camera apps, pump applications, pen applications, bolus calculator applications, and the like. As a particular example, a texting or e-mail app may refer to an event, e.g., a dinner party. Through an appropriate API, the texting or e-mail app may then serve as a source for input of calendar data. In a specific example, there could be an option for "add event to CGM" or the CGM (or equivalently decision-support application/functionality) could automatically pull event data whenever a calendar event is added. Such facility would allow users to create calendar events and CGM events at the same time, providing an easier way to collect event data and see how events affect glucose trends.

For example, bolus calculator decision-support generally requires entry of meal data. In one implementation, a camera application may prompt the user to take a picture of a meal on a plate (see Fig. 18(A)). The user may use the phone camera to take the picture, and brackets may be provided as part of a user interface on the screen to guide the user on how to take the picture. The app may then measure the meal size in relation to the plate size and denote the meal as, e.g., small, medium, or large. The app may then give a dose recommendation based on the meal size. In a more enhanced implementation, the app may use photographic recognition techniques to determine, estimate, or guess what is on the plate, and provide an estimate as to the consequent meal composition. Variations will be understood. For example, in an alternative implementation, the user prompt may instruct the user to swipe through different circle (or other shape) sizes over a picture of the meal on the plate (see Fig. 18(B)). They app may then ask the user to find the circle size that best matches the meal size on the plate.

Other real-time data that may be provided from an app includes pump or related data, including recent boluses, basal rate, and the like. As another example, the prompt may be provided based on estimated insulin-on-board in conjunction with CGM inputs and other contextual information. Insulin-on-board is a term used to describe the amount of insulin that has been given to the patient but that has not been used by the body to transport glucose into the tissue. If after taking a meal bolus, the estimated insulin-on-board is still one half of the original dose, but measured glucose has already dropped to 80, an alert might be provided because continued insulin activity is expected, further lowering the glucose to hypoglycemic levels.

In more detail, patients who administer too much insulin are at risk of hypoglycemia and possible death. There are currently no real-time measurement methods to measure how much insulin is within the patient. Insulin stacking occurs when an insulin using patient administers multiple boluses of insulin within a short timeframe. Insulin from the previous dose may not have taken its full effect and therefore the patient may give themselves too much insulin on subsequent doses, posing a significant danger of hypoglycemia to the patients. A continuous direct insulin sensor may be employed to measure the amount of insulin that is within a patient. This detector can be used as an alert or as a failsafe to shut off a pump or an artificial pancreas. As each patient generally has a different insulin sensitivity (but this sensitivity may be learned using the decision-support application/functionality described here), multiple thresholds are generally needed for alert settings.

A secondary aspect to this implementation includes the use of a direct insulin sensor to monitor how much insulin is in the patient. The amount of insulin can be used to give the patient feedback to recommend a safe dose of insulin. The insulin information can also be transmitted directly to a calculator or pump and used in an insulin dosing calculation to better estimate the proper amount of insulin to dose. This could be used instead of the current insulin-on-board estimation used in bolus calculators.

A third aspect of this implementation describes the use of a direct insulin sensor to measure a patient’s insulin sensitivity. Insulin sensitivity can vary between patients and even over time with the same patient. Accordingly, systems and methods according to present principles may use a direct insulin detector to measure the amount of insulin in a patient’s body. By combining the information of glucose and insulin concentration within a patient into an algorithm, the insulin sensitivity can be calculated. The information contained in the insulin sensitivity can then be used to determine if an insulin sensitizer, insulin secretatgogue, or insulin, should be used to manage the diabetes, e.g., to increase or decrease the amount of insulin that the patient is given to best control their glucose levels.

Returning to the chart of inputs of Fig. 16, and as another example of real-time data, which may also be used in non-real-time, GPS data from an app or other such sensor may be employed for a user away from home, e.g., for a user at college, to determine potential followers or friends.

Calendar apps may be employed to provide data about users. As an example of determining data from multiple different sources, exercise data may be determined from calendar data, if the exercise is located within an appointment notation or if a pattern of exercise implies that a user exercises at the same time every day or week.

As another example of inputs, safety rules may be employed to provide decision-support to various levels of aggressiveness. For example, if a user does not provide detailed meal information, then a decision-support application/functionality which is providing a user prompt to drive the user's glucose level to a target range may aim for the high end of the target range rather than the middle of the target. In the same way, if the user provides meal data indicating a very large amount of carbohydrates, then the high-end of the target range may again be aimed for in the decision-support application/functionality, as errors in carb counting are known to have worse effects as the overall carb amount increases.

As another example of inputs, device diagnostics may serve as an input, including device signal quality and confidence, as well as to determine risk stratifications based on the same. For example, meal or correction boluses or other decision-support prompts may be informed at least in part by confidence intervals, and the confidence intervals may be informed at least in part by device signal quality and/or detected faults.

Cloud or big data may also be used. As an example of the use of cloud or big data, a HCP may desire to determine initial values for insulin resistance or insulin sensitivity. The HCP may enter demographic data such as weight, age, and gender. The app may then search through a database and find users most similar to the subject user, and may take an average of their settings for initial suggestions. Cloud or big data may further be employed for initial risk stratification, again based on aggregate numbers.

As noted above, a CGM app may provide an input to the decision-support application/functionality. For example, in some implementations it may be preferable to handle glucose levels outside of a range of, e.g., 40 - 400, using fuzzy logic. Yet other data which may serve as an input and which is available from a CGM app may include a target glucose level or range.

Yet another type of input involves derived data. Derived data, which also may also in some cases constitute real-time data, includes an analyte rate of change or acceleration, various types of sensitivities, including sensitivities to insulin, sleep, meal, and/or exercise, a hierarchy within a risk stratification (described in greater detail below), glucose variability, user stress/emotions/feeling data, a user desire for interaction with the device, sensor accuracy/confidence/signal quality, pattern data, and the like. Such sensitivities often constitute lifestyle factors as defined above. The above-noted detection of hormone levels may further aid in the detection and quantification of insulin sensitivity, as hormone levels are known to be an associated factor.

Insulin sensitivity can be based on sleep, energy intake versus energy output, food intake, and the like. Insulin sensitivity can change rapidly and with, e.g., circadian rhythms. Insulin sensitivity is known to be event driven, stress driven, and also driven by factors such as illness, activity, or the like. If a user employs a CGM monitor, events affecting insulin sensitivity can be detected, and a measure of insulin sensitivity may even be determined from knowledge of intake values of carbohydrates into glucose and basal/bolus amounts of insulin. Typical and atypical ranges of insulin sensitivity can also be determined. Insulin sensitivity can be measured by, e.g., measuring a basal rate at the same time each day, and seeing how glucose values change with respect to various events including meals, exercise, stress, or the like, and further by performing the same test with various changes in basal rate. Insulin sensitivity may also be detected by testing levels of various hormones, including cortisol and/or epinephrine.

Derived data corresponding to meal sensitivities may be made even more granular by determination of, e.g., sugar sensitivity, dinner sensitivity, and so on. Other such perturbation or interaction sensitivities will also be understood to be derivable.

"Insulin-to-carb" ratios may also constitute derived data, and the same are generally determined and optimized using retrospective analysis. The same may be used to provide decision-support education for a user by demonstrating graphically the impact of different insulin-to-carb levels. In particular, with knowledge of insulin delivery, the decision-support application/functionality, or a related or connected module, may display to the user the effect of using different insulin-to-carb ratios on their glucose levels, e.g., by modifying a retrospective glucose trend line. Within the decision-support application/functionality, optimal insulin-to-carb ratio settings could be automatically determined, and it may be further determined how insulin-to-carb settings vary with time of day, activity level, or the like.

In some cases derived data requires a time duration over which to analyze or an analysis of a pattern to determine. For example, a determination of insulin sensitivity or an insulin resistance may require analysis of data taken over a time duration. In a specific implementation, insulin resistance can change throughout the menstrual cycle of female diabetics. The decision-support application/functionality can use a resulting cycle of insulin resistance, e.g., including incorporating insulin consumption as well as glucose values, to determine a predictive insulin resistance alert to provide to the user. For example, the patient could receive a reminder at the beginning of the day that historically in their cycle their blood sugar tends to go higher or lower. Such a prompt made help the patient be more conscientious of how they could expect their blood sugar to behave.

For an initial or seed value of insulin sensitivity, population data may be employed from a database of patients, particularly those of similar weight, age, gender, length of diabetes, and so on. Such may be used for default values and then auto adjusted as additional information about the user becomes known over time.

Meal data may also be a form of derived data as the same may be derived from food patterns, food libraries, and so on. In another way of deriving meal data, retrospective analytics may be employed to use other data to estimate carb content for a given meal. Such other data may include, e.g., insulin delivered, glucose levels, exercise, health, meal time, and the like. By collecting such data, the decision-support application/functionality may be enabled to determine a user's typical meal and typical glucose response to that meal while mitigating the effect of other factors on their glucose. Such functionality has several benefits, including improving accuracy of bolus calculators, the ability to optimize alerts if the system notices an atypical glucose response, assistance to users in understanding their eating patterns and the effects of different meals on their glucose, and more accurate calculation of insulin to carb ratios.

Derived data may further include user estimation data, e.g., whether and by how much a user is apt to make an error in carb counting, e.g., to overestimate or underestimate.

Derived data may also include data determined about recognized patterns, where the patterns are recognized by analysis of historical data over time. Exemplary recognized patterns include patterns of nightly hypoglycemia, postprandial highs, post exercise lows, or the like.

One way of determining such patterns, or of detecting occurrences of glucose events not in patterns, is by "binning" certain events defined by particular characteristics. That is, portions of glucose traces may be detected that meet predefined criteria indicative of certain diabetic challenges, e.g., rebound hypoglycemia, and then patterns may be looked for in these events that have been "binned" accordingly.

In more detail, and referring to the flowchart 700 of Fig. 19, a signature or fingerprint in glucose sensor data may be identified or differentiated within an individual patient based on some predefined criteria (step 262). Such criteria may include time-based criteria and/or may include detected specific incidents within specific constraints. In the present system, according to present principles, bins may be differentiated by different criteria. A supervised learning algorithm may be employed that allows for more bins to be learned for individual specific patterns. For example, bins can be based on insulin data, rate of change of glucose data (or acceleration/deceleration thereof), patterns of data identified before and used to characterize data, e.g., events occurring before small meals, responsiveness of an individual to their glucose information, and so on.

A next step is that the incident may be characterized, e.g., based on a decay curve, waveform signature, or the like (step 264). Exemplary incidents may include meal bolus indicators, e.g., based on insulin data, which may be classified into small, medium, and large meal bins. Such may correlate with insulin data. Different meal types may also be characterized, which may then be sub-binned into different meal compositions. These may correlate with rate of change/acceleration/deceleration. Incidents can also be characterized based on their correlation with events before a meal, and such corresponds to the patterns of data noted above. Incidents may further be based on behavioral patterns, e.g., how often a user reviews their glucose data or responds to alarms, and such can be correlated with the responsiveness data noted above. It will be understood that other bins may also be employed.

The characterized incidents may then be placed into a bin (step 266). By then synchronizing by incident, bins may be tuned for specific patient physiology. The bins can then be normalized, i.e., a normal distribution of incidents in the bin may be defined (step 268).

The normalized bin information may then be used proactively as an input into the decision-support application/functionality (step 268), e.g., in the determination or definition of the state according to Step A. As one example, the normalized bin information may be an input to a bolus calculator. The normalized bin information may also be employed to respond to other requests proactively determined or requested by the user, e.g., when a user asks for a bolus calculation before a meal, or when the system prompts for a bolus calculation before a meal based on time or other factors. The binning technique may be employed to determine when a patient is more tuned into their data, based on behavioral input, which may then allow for more aggressive recommendations as opposed to the case when the user is not focused on glucose information, e.g., nighttime, when the bolus recommendation is generally more conservative.

Returning to Fig. 16, in non-real-time data, inputs may include user-entered data such as prior SMBG data, user savviness with technology, goal data, a problem to be solved, a certain desire for the therapy, a target glucose level, demographic data such as age and gender, their cardio health, cholesterol level, a type of mathematical calculation to be employed (typically entered by default or by a HCP), data about illness or pregnancy or menstruation, data about medications taken, smoking history, and the like. Non-real-time data may also include derived data, including historical patterns, gastric emptying duration, or the like. Other non-real-time data may include data about a follower, e.g., their tone of communications, their language, and so on. These may be employed in modifying outputs for greater effectiveness to the user.

With regard to gastric emptying duration, it is noted that 25% of the diabetic population (type 1 and type 2) experience some form of gastric emptying delay. 7 to 12% of the same group suffers from an elevated delay for gastric emptying. The population of gastric emptying delay is increasing at a very significant rate within the diabetic population, and is becoming a concern for health care professionals within the general population currently.

Current bolus wizards do not factor in gastric emptying, thus certain risks exist. For example, if gastric emptying is not factored into a closed loop pancreas system, the individual runs the risk of glucose variability where there are repeated corrections and insulin delivery reductions. This results in chaotic insulin delivery which would resemble a successive approximation wave form over time. If the same is not used within a bolus calculator for an insulin pump, the result is to endanger the person as they may suffer a hypoglycemic event which would roughly correlate to the size of the meal which they had dosed for. They may also suffer a hyperglycemic event which would roughly correlate again to the meal size, as its effects would take effect when the reactance of the insulin is reduced and is declining within the system.

Over time such variabilities can manifest themselves into further damage which may degrade gastric function over time. Additionally the person runs the risk of increased peripheral neuropathy.

Gastric emptying delays push out the process of moving processed and prepared nutrients and food into the small intestines where actual absorption of nutrients occurs, and can be triggered by such factors as neuropathy of the vagus nerve, nerve damage within the stomach, and muscle damage within the stomach. Gastric emptying studies are used by GI doctors to quantify the extent of the delay which may result in a prognosis of gastro paresis.

The general direction given to insulin dependent diabetics is to dose (bolus) between 0 and 30 minutes prior to a meal. The rational in this directive is to time the effect of the insulin to correspond to the time at which the consumed food enters the absorption phase. This timing is accurate in the case where gastric emptying is not an issue (75% of the type 1 & type 2 population). However in the balance of the population, the delivery time of the insulin is inaccurate. Fast acting insulin reaches maximum effectiveness roughly 90 to 120 minutes after a bolus is delivered. This is the delay from bolus delivery, propagation through the body and adherence to the A & B receptors on the surface of the cells. At that time, the cells are able to process sugars and do so. However for an individual with a gastric emptying delay, the delivery of the processed foods to the system has not occurred yet. For these individuals, roughly 90 to 120 minutes after eating (if they dosed a bolus 30 minutes prior to a meal) they will suffer a hypoglycemic event. This event is generally addressed with a liquid such as orange juice which enters the intestines and system more quickly than solid food.

The insulin reaction is on the decline at the end of the peak for an elevated case of gastric emptying delay at the time where the food is being processed and is entering the system. This results in a hyperglycemic event at which a correction bolus needs to be delivered. Thus, gastric emptying duration may be employed as an input in order to provide a time delay for delivery of a bolus after a meal. This delay may be applied to a bolus calculator or to a closed loop artificial pancreas system. Gastric emptying duration may also be employed as an input in order to provide a time delay for a modified basal rate after a meal. This delay may be applied to an insulin pump or to a closed loop artificial pancreas system.

One way of determining gastric emptying delay within an individual is as follows. If a CGM user enters a bolus and a meal, then the following 4 hours of data are analyzed within the CGM. If there is a repeatable event which occurs for the individual which follows a trend, then a gastric emptying delay may be inferred. The inferred delay may then be applied to the bolus and/or modified basal rates as described above.

Referring to the example shown in Fig. 20, time period (a) indicates bolus and food entries. They are also offset from one another. Time period (b) indicates the period when food processing should occur, however, for this exemplary patient, the food processing is delayed. Time period (c) indicates when food should be processed normally as it would align with the bolus. Time period (d) indicates when actual food processing occurs in a gastric delay. The slope of the BG decline is indicative of the rate of gastric emptying delay as well as the general delay. This delay is also represented within the BG value signal by determining the deltas between the start of (c) & (d) as well as their durations. In order to isolate this signal within the general noise within the blood glucose signal, insulin sensitivity is calculated from the individual's blood glucose data.

Referring again back to Fig. 16, non real-time data may further include stored data, as stored and retrieved from memory or other storage. Stored data may include historical data, both of determined patterns and data used for determining patterns or other glucose events. Stored data may include prior user events. For example, if a HCP has previously commented on a pattern, if the pattern repeats, the HCP comment may be re-displayed on the user interface.

Inputs can be "fuzzy", e.g., may fall into certain categories, and the categories themselves used as inputs, e.g., small/medium/large meals, or "less than normal"/normal/"more than normal", or alternatively the inputs may be crisp. Thus, also shown in the table are exemplary categorized or fuzzy inputs, as well as exemplary crisp inputs. The table also indicates an interpretation as to the type of context for the input, e.g., whether the same concerns lifestyle aspects, situational aspects, clinical aspects, or device aspects. In some cases an input may occupy more than one category.

The way in which inputs are used and aggregated may vary. In one implementation, a function may be determined, or a user system model may be created, and the inputs used directly on the function or model. In another example, in a hierarchical approach, the decision-support application/functionality may be designed to use information obtained from different sources in a layered approach. Bare-bones systems will use CGM data, and as additional data is received, the same may be used for decision support. The confidence in estimates and decisions then generally improves as the amount of information increases. In the same way, users or use conditions may be categorized in buckets or layers of risk. Each time information is available, the system may move up or down the hierarchy in order to ensure safety.

In addition, as may be understood from the above, combinations of inputs may themselves result in an additional input. For example, an alternative empirical approach may be to control aggressiveness based on historical levels or success of predictions. For example, aggressiveness in decision-support may be reduced when the situation, e.g., time of day, day of week, type of event, and so on, has historically resulted in variable or poorly-predicted glucose levels. For example, a user may consume a very consistent lunch during the week and inconsistent lunches on weekends, where the consistency refers to timing, meal size, and meal content. The inconsistency may result in less predictable results of pre-lunch boluses on weekends. The decision-support application functionality may then detect this poor predictability and adjust aggressiveness accordingly.

Referring to Fig. 21, modes of operation of the CGM app or decision-support application/functionality may serve as inputs which in turn may be categorized in different ways. For example, the mode of operation may refer to a calibration mode, such as device self-calibration or factory calibration. The mode of operation may also refer to a transmission mode (transmission of data to a display device), such as scheduled transmissions or unscheduled transmissions. It will be noted that all of these modes described are exemplary and that other modes of operation are also possible. The mode of operation may also refer to a decision-support mode, such as therapeutic, non-therapeutic, adjunctive, non-adjunctive, and so on. As may be seen in the figure, even where the modes may be thought of as being operationally sequential, the decision-support application/functionality may jump from one mode to an adjacent mode or from one mode to a nonadjacent mode. An example is the case of scheduled transmissions, where in mode 1 a transmission is made every 30 seconds, in mode 2 a transmission every minute, in mode 3 a transmission every 5 minutes, and so on. In many cases such modes will not be marked by just a change in a characteristic time, but rather by a change in functionality, e.g., from periodic to aperiodic.

Signals may be collected from sensors within smart watches such as the Apple Watch and the Microsoft Band to augment certain entered data, e.g., for hypoglycemia detection. The signals can include heart rate, sympathetic/parasympathetic balance (inferred from heart rate), perspiration, motion, and the like. The signals can be used in addition to the CGM signal. Algorithms employed to process the auxiliary signals can be trained on the patient's own data, using CGM to assist in the training. These outer limits can be optimized off-line, or in the cloud. Detection criteria then can be sent to the patient's smart phone and/or smart watch. For example, there may be instances when CGM fails to detect hypoglycemia, but when augmented with auxiliary signals indicating possible hypoglycemia, the patient may be alerted to suspected hypoglycemia and thereby enabled to avoid the consequences. Alternatively, after the algorithms used to process the auxiliary signals have been trained, the smart watch signals can be used to detect hypoglycemia without the use of CGM. In this use case, adjustments to the algorithms may be used to optimize sensitivity/specificity.

Detected cognitive ability may also serve as an input. In more detail, it is noted that cognitive ability and motor function may be affected by numerous varied inputs and effects. For example, an individual with diabetes may suffer from reduced cognition and/or motor functions as a function of hypoglycemia. As a hypoglycemic incident occurs, and blood sugar levels drop, the individual suffers from concentration and/or comprehension degradation. Additionally, due to the body responding to a lack of sugar, the muscles may tense and begin to act erratically.

With a capacitive ability on an input device, such as a smart phone or tablet, the user may view information, makes decisions, and press on screen indicators to perform device interaction. As a user becomes familiar with an application on such a device, their ability to make decisions increases, while the delay between interactions reduces. They additionally develop a series of familiar sequences in order to perform common tasks that they have become accustomed to.

However, when an individual suffers from a drug interaction, alcohol, or hypo / hyper glycaemia, these familiar tasks become less familiar. This results in a situation where the user must spend more time observing and comprehending a user interface which normally is intuitive and requires no concentration. The time spent on a user interface screen will lengthen compared to the historical norm of the individual. When the motor functions of a user degrades due to any of the mentioned influences, the ability to press an on screen indicator such as a button, a slider, or onscreen keyboard, become less uniform, and may deviate from the normal metrics for the user. In such situations, the initial press location in relation to the center point of the control may deviate from the user's normal press location. Additionally, where a press / lift action is the interaction to a button, in a motor control deficient situation, the press may be followed by a "drag" motion, and the lift location of the interaction may change. By accumulating the metrics of time and user interaction on a user interface screen, e.g., the press XY location for a control in relation to its center point, the XY distance travel prior to lifting the finger, the duration of pressed state, and the like, a profile of the user's interaction can be maintained. This data can then be correlated to the user blood sugar reading in order to determine a general population behavior, as well as sub populations based on gender, age, and so on. This information can further be used to determine where the cognitive ability / motor function ability of an individual rests, in relation to population groups to which the individual is a part of. A degree of impairment for a particular individual may also be determined and used as an input. In other words, the decision-support application/-functionality may make decisions and/or actions based on this information, e.g., by using the same in the definition of a state according to Step A. Such decisions and actions may be to alert another individual in order to notify or request assistance. It may also be used to disallow operation of a motor vehicle or other potentially unsafe action in such a condition.

In a related implementation, a guiding "wizard" component may instantiate if the cognitive ability is degraded. In this case, more verification may occur such as "You are about to change your settings, are you sure you want to do this?", safety guidance such as "you should wait until your blood sugar rises before changing your sensor" may occur, and so on.

In another implementation, once motor function degradation is detected, buttons and user interface components can be enlarged, or a larger press area may be utilized. It also may interpret a "drag" motion on a button as a "press" action, thus assisting the individual.

In this way, patients affected by hypo / hyper glycaemia may be assisted, and their followers notified of their condition. It may further keep those impaired by alcohol from trying to take unsafe or untimely actions. It may further identify anecdotally if a drug interaction or drug reaction is occurring. It may further track such data over time in order to accrue reaction over time to such events. It may generate data useful in determining a person’s condition in relation to a population of similar people.

Another type of data which may be employed in providing decision-support is diabetic subtype. In particular, diabetes is currently categorized into two different categories. Type I diabetics are unable to produce insulin and thus rely on an external source of the same. Type II diabetics have a nonoptimal insulin production capability and generally rely on injected insulin in order to offset the physiological insulin production anomaly. Various subtypes of these may also be understood, generally corresponding to genetic mutation. Genetic mutation #1 involves a marker within the genetic sequence of individuals which results in a surfaced apology aberration on the sales of the subject. This aberration causes a reduced ability for insulin to adhere to the A and B receptors on the cell surface. The effect of the inhibition or reduced adherence results in a difficulty for insulin to perform its effect. This causes insulin to take a longer period of time to adhere to cellular surfaces, thereby reducing the effect of a known volume of insulin. Additionally, a longer period of time is required in order for the insulin to adhere to an adequate population of cells in order to cause a measurable effect on blood glucose levels. This is referred to as insulin resistance. Genetic mutation #2 is the opposite of genetic mutation #1, and results in a surfaced apology deviation in individuals which allows for easier than normal adherence of insulin, resulting in a quicker cellular response as well as a larger population of cells responding to the insulin. By dividing patients by subtype of diabetes, additional personalization and tuning/tailoring of therapy prompts may be provided.

Another type of user-entered input which may be employed in the decision-support application/functionality is user input on alert thresholds so as to reduce alert batik. User-entered thresholds may reduce the number of clicks necessary for the user to set up alerts. In this implementation, and referring to Figs. 22(A) and 22(B), a user can, on the user interface, touch the actual threshold line on the graph and drag it to the alert threshold they desire. A pop-up value informs the user of the current exact value of the threshold. When the user takes their finger off the line, a pop-up will ask the user if the threshold is correct. This functionality mitigates accidental touch and drag. In a variation, the graph could be one allowing the user to follow daily trends and set alerts corresponding to their daily activities.

The above description has included various types of inputs. Other and more examples of inputs, and the use of the same in decision support, are described below with regard to specific examples. Examples are also provided in which variables are tied together to glean other and additional data, e.g., if GPS indicates very rapid movement, it may be inferred that the user is driving and this information used in decision-support. In addition, other examples of determining inputs, and in more detail the use of smart phones to glean lifestyle information, are disclosed in co-pending US Patent Application Serial Number 13/801,445, file March 13, 2013, entitled "Systems and Methods for Leveraging Smartphone Features in Continuous Glucose Monitoring", owned by the assignee of the present application.

## Outputs

A significant portion of the discussion of specific outputs will be in the context of specific examples described below. However, certain broad categories of examples are described here with respect to Fig. 23A. Generally, an output of the decision-support application/functionality is not just a CGM value or at alert, but also a prompt for a therapeutic action to take a response, e.g., a value to use in a bolus calculator. In particular, a user device 272 running a decision-support application 276 (subject to the above comments made with respect to the location of the decision-support application and whether it is a separate application versus functionality added to a CGM application) may have a display 274 on which is rendered a user interface on which therapy prompts may be rendered. In particular, the display 274 can display bolus information, modified basal information, rescue carbs, pre-activity recommendations, pre-sleep recommendations, and the like. The display may also be a user interface for the reception of user-entered data, including the inputs described above with respect to Fig. 16.

The outputs on the display 274 may be crisp or fuzzy, depending upon factors such as the input data and its accuracy, user desire, most efficient processing, and so on. For example, a crisp output may indicate a specific direction or CGM value, while a fuzzy output may include, e.g., "we recommend that you eat or exercise more/less." In many cases the displayed output will include a CGM value and a prediction or a rate of change. An indication of a decision-support mode may also be provided, so that the user knows whether the same may be employed in therapeutic decision-making, adjunctive decision-making, and so on.

Usually the display device will be associated with the subject user, and the display device may be, e.g., the subject user's smart phone. However, it will be understood that other subject user devices are possible, including a dedicated receiver, a smart watch, or the like.

The output, besides the therapy prompts, may include CGM data, trend data, and so on. In some cases, trend data may be turned or toggled on or off.

The output may also be provided to a different device 278, e.g., which may be the device of a follower or other user to whom the subject user has shared their data. On the device 278, again the data may be output or input in a fuzzy manner, a crisp manner, or via any of the other ways described here. The different device 278 may be a smart phone, a dedicated receiver, a smart watch, or the like. The output on the different device may be the same as that on the subject user device, or it may differ. In some cases, such as where the different device is associated with a parent and the subject user is a child, the display may be the same so that the parent can view the child's disease state to the same level of detail that the child sees, or even to a greater level of detail. For example, the child may see just one of three colors, e.g., red/yellow/green, indicating a general current disease state of bad/medium/good. The parent device may be provided greater detail, so that the parent can provide suggested actions, e.g., so as to address patterns of issues that the child is not addressing on their own. For example, the parent device may include the CGM value, the rate of change, and one or more suggestions by way of user prompts.

In another implementation, such as where a parent device is following an older child device, the output to the parent/follower device may be to a lesser level of detail than that provided to the subject user. For example, the parent may simply be given an indication that the subject user is "okay" or the like, or has taken action in response to an alert/alarm. For an even more responsible subject user, follower outputs may be even briefer or more minimal. For users above a certain age, e.g., 16 years old, 18 years old, or other parent- or guardian-set age, the subject user may provide filters on what is provided and displayed on the parent or follower device. For example, for a subject user away at college, the subject user may set a filter such that only important alarms are sent to follower devices. In yet another implementation, a one button UI may be provided to allow a subject user to quickly and conveniently inform followers of certain information, e.g., "I'm okay" or "Don't worry, it was compression.", or the like.

The output may also be provided to a pump 280, a pump 282, or an artificial pancreas system 284. Generally the outputs provided to these electromechanical devices are as accurate as possible, and thus are generally crisp. However, in some cases, fuzzified outputs may also be employed, particularly where quantized units of medicaments are being delivered. In these cases, the processing may occur on crisp variable values, however, the output itself to the medicament delivery device may be fuzzy.

In a system tending to more closed-loop aspects, the sensor system or CGM may automatically transmit parameters used in medicament delivery to the pen or pump. Here the term "closed-loop" includes various types of closed-loop or semi closed-loop systems, as well as fully closed-loop systems like the artificial pancreas that specifically includes pump functionality. For example, various hybrid closed-loop systems may include technology -assisted diabetes management devices, diabetes device integrations, and so on. Such terms are used in an exemplary but not limiting fashion to encompass integrated systems in which data measured by a CGM or other analyte sensor system is more than simply displayed to a user; such is transmitted to a medicament delivery device and potentially, but not necessarily always, used as part of a dosing regime, either on a one time basis or a continuing basis, e.g., periodic, occasional, or as needed. Use with continuing dosing regimens is also envisioned, e.g., for basal dosing. In any of these implementations data transmission need not be direct: such may be transmitted through a cloud or other server system.

When such a transmission is made, it will generally be received according to a protocol established for such transmissions, and the protocol is appropriate for and matches that of an operating system of the medicament delivery device. In some cases, the receiving will involve an instantiation of a receiving or reception routine by the operating system of the medicament delivery device. In other words, if the medicament delivery device is not ready to receive data, a step may be performed of readying the medicament delivery device for such reception.

An acknowledgment signal may be transmitted by the medicament delivery device and received by the CGM or other sensor system, the same indicating that the medicament delivery device has received the data, and such may include a step of verifying the data received.

Data received by a medicament delivery device may either be accepted as received (and subsequently employed in medicament delivery) or the patient may be asked to confirm the entry of the data prior to, contemporaneous with, or after, transmission of the same. In still other cases the control of the pen or pump based on the sensor system is more direct, and the sensor system may not only furnish the data but the medicament delivery system may directly and immediately rely on the data for medicament delivery even without patient confirmation.

In some cases, external data may also be used in the control of a pen or pump, along with the sensor system data. Such data may be transmitted from an external source to the CGM application for inclusion in its calculations, or the external data may be provided directly to a pen or pump, for its respective use in its calculations. The external data may be from a smart phone, a smart watch, an activity monitor, an accelerometer, a sleep monitor, a medicament pump, GPS device, a redundant analyte sensor, a smart pen, a server providing access to EMR data, a personal health application, or combinations of the above.

Data provided to the pan or pump may be employed in the operation of the same in a number of ways, including driving more complicated systems, for multiple phases of control, even up to and including artificial organ systems, including artificial pancreas systems.

Other details are available from US PGP 2016/0081597A1, owned by the assignee of the present application.

The use of the transmitted signal may be based on a mode of use of the medicament delivery device. For example, if the use is nontherapeutic or purely adjunctive, an indication of the signal may simply be displayed on a user interface. In some cases, the signal may even instruct a medicament delivery device in this situation to disregard received signals from the sensor system and/or monitoring device.

If the use or mode is therapeutic or not adjunctive, the signal may instruct the device to receive and employ signals from the sensor system in an appropriate way, generally depending on the phase or mode of operation. In many cases, for therapeutic modes, the signal may instruct the medicament delivery device to receive and follow signals from the monitoring device or sensor system to control the user glucose concentration value to a target value or a target range of values, or in some cases only when the glucose concentration value is below a predetermined value, above a predetermined value, or within a predetermined range of values.

In some implementations, the system may be employed to detect connection to and usage of a medicament delivery device, and thus to provide signals to the same accordingly. In a first step, a session begins and/or the monitoring device starts up. In some cases the monitoring device starts up in a fully configured mode as enumerated above, e.g., by simply starting up in a last-identified mode. In other implementations the monitoring device powers up and as part of its initial configuration determines what mode to start in. As part of this determination, the monitoring device may detect connection to or usage of a medicament delivery device. For example, the monitoring device may detect that it is signally coupled to a medicament delivery device such as an insulin pump. In this case, the monitoring device may start up in a therapeutic mode, subject to, e.g., optional patient confirmation.

The user interface of the monitoring device may also request input as to the usage of the device, if one is detected. For example, one or a series of questions could be asked of the user at the start of a session or when setting up the device that determines how the user intends to use the sensor data. A user could select a use case for the data, e.g., therapeutic versus adjunctive, or, e.g., educational e.g., just watching trends, versus decision-support based, and the entered data may be used to inform how the monitoring device and/or sensor system sends signals to the medicament delivery device, and/or what the monitoring device does when it receives such signals.

In some implementations, the system-detected usage and the user-entered information may be employed in a final determination of what mode the monitoring device is set to. In other words, the system-detected usage and the user-entered usage data may not by themselves uniquely determine the most appropriate mode, but the two may act as data inputs which together (and optionally with other data) determine the device mode. Moreover, in some cases it may not be desired to have a user enter a particular mode *per se,* but rather to have the user enter more user-friendly information that is in turn converted to a determined mode. For example, it may be more understandable to a user to indicate that their CGM is driving their pump then to necessarily have the user indicate the more technical distinction that the device is in a therapeutic mode rather than an adjunctive one.

As another example of where the usage type is determined in part by detection of medicament delivery devices, the monitoring device may further detect that it is connected to a medicament delivery pump that has a flag or setting that indicates its use in a closed-loop - decision support configuration. The monitoring device may then adjust criteria for operational modes based thereon, e.g., increasing the calibration requirements based thereon.

More generally, measurements from the sensor of analyte concentration can be transmitted via the sensor electronics to smart devices as well as to servers for subsequent analysis and calculations. Such measurement results can also be advantageously employed as inputs to medicament delivery devices such as pens and pumps. For example, in one implementation, an analyte sensor including a continuous glucose monitor is employed to inform operation of a pen or pump that delivers insulin. In some cases, the sensor system provides a display or readout that is then employed by a user in entering settings or parameters to a pen or pump. For example, such may occur if the user is requesting a bolus calculation or is intending a manual adjustment of their basal insulin rates or appertaining parameters.

The therapeutic/adjunctive paradigm may be broadened to a spectrum of levels of control of pump function, from an adjunctive mode where the CGM does not control the pump in any way, up to a point where analyte monitors control all medicament delivery, e.g., insulin as well as others, and no patient involvement is needed. In one exemplary implementation, the spectrum of levels of pump control may be the phases shown in Fig. 23B. In this diagram, the phases progress from the system exerting no control to the system exerting the most control.

In more detail, where the output is provided to an artificial pancreas system 284, the same may be provided to inform the operation thereof, as well as to inform one or more of the various phases of control leading up to a full closed-loop artificial pancreas system. In more detail, a spectrum of levels of control of pen or pump function may be seen, from a mode where the CGM does not control the mechanical device in any way, up to a point where analyte monitors control all medicament delivery, e.g., insulin as well as others, and no patient involvement is needed. Details of such systems are described in U.S. Patent Publication No. US-2016-0081597-A1, entitled SYSTEM AND METHOD FOR MODE SWITCHING, owned by the assignee of the present application. In this exemplary implementation, the spectrum of levels of pump control progress from the system exerting no control to the system exerting the most control. Generally, a control system receives a signal from an analyte sensor in a biological system and exerts control over the analyte concentration in the biological system by controlling intake of one or more substances into the biological system using, e.g., a medicament administration device, which may include, e.g., a pump, an IV, and/or one or more other devices which can controllably administer a substance into a body. In an artificial pancreas system, the analyte sensor may be that associated with a continuous glucose monitor, and the one or more substances may include insulin administered by pumps or injections. The control system may be situated within a display system or as part of the sensor and more particularly as part of sensor electronics, or even as part of the medicament administration device.

Parameters used by an artificial pancreas system may include parameters used in control of a given phase as well as parameters used to switch between phases. These parameters typically include data input to the system, including the following: glucose concentration and time rates of change thereof, glycemic variability, data confidence, data usability, presence of one or more detected faults, e.g., the likelihood of an "end-of-life" or other failure mode. Such faults may be detected by comparing current data to known failure modes as identified by signatures or patterns in trace data. Yet another type of data appropriate for informing AP parameters include those relating to glucose context, e.g., whether the glucose context is nighttime hypoglycemia, adjusting insulin dosing, or other such contexts. Another type of data which may be employed involves the use of user-perceived error. That is, if a user specifically indicates an error or provides a greater than average number of external meter values, or provides external meter values without prompting, a user-perceived error may be inferred. Accordingly data usability may be decreased and/or the user may be prompted to enter one or more reasons as to the perceived errors.

Fig. 23C illustrates schematically an exemplary such artificial pancreas system 411. In this system, control is exerted to various extents in respective various phases (in the implementation of Fig. 23B, for phases above and including phase 1). Generally, a control system 421 receives a signal from an analyte sensor 415 in a biological system 413 and exerts control over the analyte concentration in the biological system by controlling intake of one or more substances into the biological system using, e.g., a medicament administration device 417. The medicament administration device 417 may include, e.g., a pump, a pen, an IV, and/or one or more other devices which can controllably administer a substance into a body. In an artificial pancreas system, the analyte sensor may be that associated with a continuous glucose monitor, and the one or more substances may include insulin administered by pumps or injections. In a more advanced system, other analytes may be monitored including insulin, and the one or more substances may include, e.g., glucagon. Extending beyond the artificial pancreas, control may be extended in this way to other hormones besides insulin. While the control system 421 is illustrated in Fig. 23C as being situated within a display system 419, the same may also be situated as part of the sensor system and more particularly as part of sensor electronics (see control system 421' within analyte sensor block 415) or as part of the medicament administration device 417 (see control system 421"). Alternatively, control system modules within two or more of these blocks may work together to accomplish both the control required of the phase and the potential mode switching according to implementations described here.

The use of an artificial pancreas system according to Figs. 23B and 23C can also include the implementation of methods to drive, base, or inform switching between the various described phases of Fig. 23B (or other phases which may be developed). The use can also include use of the operating mode or phase to drive, base, or inform display on a user interface of the display system 419. In particular, the display on the user interface may include an indication of the monitored analyte, as well as in some implementations an indication of the mode or phase in which the system is operating, i.e., the mode of user interaction. For example, the user interface may display the glucose concentration as well as an indication that the artificial pancreas system is controlling for hypoglycemia but not hyperglycemia. In another example, the user interface may provide an indication that the user is in, e.g., "Phase 6" and that all control is currently being provided by the system. Numerous variations will be understood given this teaching and the examples provided.

The display system 419 may constitute, e.g., a dedicated receiver or a general purpose device such as a mobile device, e.g., a smart phone.

Referring back to Fig. 23B, in an initial phase, i.e., Phase 0, shown by reference numeral 449, the use may be adjunctive only. In other words, the CGM is not used for any sort of pump (or other sort of medicament delivery device) control. Significant levels of information may still be provided in this phase, as well as in the other phases. However, such information may be indicated on a user interface so as to be only for tracking purposes, determining trends, or for educational purposes. For a user who is pre-diabetic, phase 0 may be the only phase needed.

In a next phase, i.e., phase 1, shown by reference numeral 451, an insulin pump may be controlled so as to turn off at times when the user is encountering low glucose levels. In phase 1 and subsequent phases, it will be understood that while pump actions are disclosed, the same may be accomplished by an indication on a user interface of the monitoring device directly employable by a user for dosing, e.g., by injection, ingestion, or the like.

In a subsequent phase, i.e., phase 2, shown by reference numeral 453, phase 1 may be enhanced, e.g., by allowing hypoglycemia predictions to occur, and causing alarms when such conditions are present or likely to occur. If such alarms go unheeded, a phase 2 system may cause a reduction or cessation of insulin if the user's glucose level is below a threshold.

In a next phase, i.e., phase 3, shown by reference numeral 455, phase 2 may be enhanced, e.g., by including a step of insulin dosing when the user's glucose level passes above another threshold, i.e., a high threshold. In a subsequent phase, i.e., phase 4, shown by reference numeral 457, the system may be essentially closed loop, except for mealtime manual assist bolusing. In particular, the system may cause insulin reduction or cessation at low glucose levels, and insulin dosing at high glucose levels. However, due to glucose variability at meal times, bolusing at such times may be performed manually.

**In** a next phase, i.e., phase 5, shown by reference numeral 459, such mealtime manual assist bolusing may be removed, and the system may be closed loop for insulin. In a final phase, i.e., phase 6, shown by reference numeral 461, the closed loop system may be extended from just insulin to contemplate and control other hormones as well, for a closed loop multi-hormone system.

**In** one implementation, the above mode phases may be employed in a control scheme, and mode switching may occur between the various phases. Additional details on mode switching are disclosed in the patent application discussed above. While the modes or phases or have been described in the order of least control to most control, there is no requirement that the system proceed in such an order (in either direction) in the control of a therapy. Each mode or phase is stand-alone, and may be entered or exited independently according to the determined data and mode switching criteria. For example, the system may transition from phase six to phase zero, and more generally from phase i to phase j, where i and j are any of the phases zero to six.

According to, e.g., the sequence of Fig. 23B, various levels of pump control may be exerted including full pump control. Alternatively, even if data usability is high, a user or clinician may choose to exert, or the monitoring device may be configured to switch to a mode exerting, a varying or reduced level of pump control. The levels of pump control exerted may be considered to select an aggressiveness of the pump control. Specific examples of such aggressiveness levels are now described.

A number of actions are described within Fig. 23D, and many of these are described as "do or do not" perform a given step, e.g., treat hypoglycemia, control to target, calculate bolus, etc. As part of a procedure within phase 0, the "do not" portions of these steps may be generally employed, e.g., do not allow hypo/hyper minimizer (step 438), do not suspend for low glucose (step 440), do not calculate bolus (step 442), do not treat hyperglycemia/hypoglycemia (step 444), do not control to range (step 447), and do not control to target (step 448).

The "do" portions of the steps may then be enabled to perform one or more functions within a system for phased therapeutic control, e.g., as noted with regard to Fig. 23B.

For example, to accomplish phase 1, the system may be configured to suspend pump actions for low glucose concentration values (step 440). To accomplish phase 2, a level of prediction may be enabled, and alarms may be caused if hypoglycemia is predicted. The same may be accompanied by a reduction or cessation of insulin below a predetermined threshold.

To accomplish phase 3, the system may be configured to allow a "hypo-hyper minimizer" (step 438), where the same is a closed-loop system that only controls insulin using sensor data at low and high glucose levels, rather than using the same also within target ranges. To accomplish phase 4, the system may be configured to calculate a bolus (step 442) during mealtime. In this way the system acts in many ways closed loop but allows additional user control during particularly uncontrolled times, e.g., meal times.

To accomplish phase 5, the system made more generally be configured within to treat hyperglycemia and hypoglycemia, e.g., by dosing insulin and, e.g., glucagon, as appropriate. Finally, to accomplish phase 6, other hormones may be employed to provide even greater balance and stability to the patient's biochemistry. In so doing, the system may control to a target value of the hormone (step 448), or alternatively to a target range (step 447).

Within these phases, it will be understood that the exerted level of pump control may vary. For example, closed-loop control such as available in phases 4-6 may in certain implementations be used only within certain glucose ranges, e.g., 20 - 70 mg/dL. Ranges may be individually-controlled based on certain criteria and whether the mode is adjunctive or therapeutic or based on the phase. For example, depending on the evaluation step, there could be different criteria in adjunctive mode for treating hyperglycemia versus hypoglycemia.

Mode switching has been described above in the context of switching between phases of an artificial pancreas or other artificial organ for analyte control, both with regard to general phase changes and for specific reasons, e.g., based on data or signal usability. However, mode switching may also be applied more generally, and may be based on other factors as well. For example, a mode of operation of a CGM or other sensor system, or a mode or phase of an artificial organ system, may be based on one or more parameters associated with one or more models. For example, the mode or phase may be based on behavioral or contextual data, including where the same is described by a model, and including where the same is quantified by various system model parameters. For example, if certain system model parameters exceed a threshold or are within a target range, an appropriate mode may be entered. As a particular example, where the behavioral or contextual model is described by a pattern, the pattern based on time, time may be a system model parameter that affects the mode. So if, e.g., if a pattern indicates that it is desired to enter a particular mode at nighttime, and if a real time datum of "time" has a real time value that occurs during the night, a particular mode may be entered or departed from.

It will be understood that such switches of phases or modes may apply to the operations of sensor systems such as CGM devices as well as medicament delivery devices, and furthermore to other integrated devices accomplishing technology-assisted diabetes management, and other such hybrid closed-loop systems.

Where output is provided in a form directly to a user, the output is usually in the form of a recommendation or advice, e.g., a recommendation of an action to take before exercise, before sleeping, before driving, before or during meals, and so on. In many cases the system may calculate and display a bolus to be entered by the user in a pen or pump and thus delivered to the user. The therapy prompt may provide a decision-support recommendation based on one of the methods described above, such as that of Figs. 6- 12. For example, the recommendation or advice could be based at least in part on context, where context could be clinical context, lifestyle factors, situational context, demographic data, or the like. It is often based on a correlative parameter, as described above, which in many cases can be a parameter the CGM system can determine but which is information or data not generally accessible or determinable by the user. It is also often based on state.

In a displayed UI, the UI could be modified or varied in a number of ways, including with respect to required interactivity by the user, complexity, and so on. As another UI modification, menu items could be sorted according to influential or prioritized parameters and variables. Where such data is known, the UI could adopt a tone, either in written or spoken (voice generated) communications, known to influence the user. Such a tone may be determined by, e.g., analysis of user/follower communications. The tone may further be based at least in part on lifestyle and context.

In some cases the system may display a rendered user interface that provides a therapy recommendation and also provides a contextual reasoning, one after the other, e.g., as strings, where the therapy recommendation is determined as noted above. Such may increase confidence in the therapy prompt by giving a reason therefore.

In another implementation, the displayed output can be followed by a request for user feedback post recommendation, e.g., two, four, or six hours after the therapy prompt. Alternatively, the user can provide feedback on their own. In either case, the feedback can provide additional information to the system to enhance the machine learning and may also build user trust in the decision-support application/functionality. For example, the decision-support application/functionality may provide a prop such as "You under/overcorrected - why?", Or "You corrected enough, wait before considering a correction bolus", or "Good timing and dosing amount!", and so on.

Similarly, the decision-support application/functionality could ask for confirmation, e.g., "Because you have a soccer game at eight o’clock, we are recommending more insulin. Do you agree?" Such implementations are not only useful for gathering data but also serve the purpose of building trust in the decision-support application/functionality and its outputs, as the user may be more assured that their inputs are being factored into decision-support therapy prompts and recommendations. In the same way, the output may include a description of why the decision was made, further in order to build trust.

In yet another implementation, if the pattern repeats, and the user’s HCP has previously commented on the pattern, the user interface may display the prior HCP suggestion.

In yet another implementation, the decision-support application/functionality could provide open loop advice on bolus values, as well as recommendations for short term or long-term adjustments to basal insulin. As noted above, dose adjustments may be provided in predefined increments, e.g., 0%, +/- 10%, +/- 20%, and so on, for ease in administration. While specific examples are given below, here it is noted that an adjustment of a bolus value may be based on, e.g., insulin-on-board, insulin sensitivity, target glucose, current glucose, type of insulin, e.g., long-acting versus fast acting, activity after bolus, and so on. Primary inputs to a bolus calculator include current glucose level, the number of carbs to be consumed, and level of active insulin. Secondary factors include past blood glucose levels, previous carbs consumed, and the last bolus. Tertiary factors include how many carbs were eaten earlier, a current basal rate, and a history of boluses. Systems and methods according to present principles may prioritize in these inputs on the display screen, especially primary inputs, as well as assist the user in figuring out meal composition, e.g., carbon and fat. The adjustment of a bolus calculator generally attempts to drive the user towards a target glucose value, and such bolus calculators can be trained by entering in known carbohydrate values and tracking a response. Such may be employed as part of a "training phase" for the learning and determination of, e.g., insulin sensitivity. In a particular implementation of a bolus calculator, another common input is the "insulin-to-carb" ratio which serves as a correction factor within the bolus calculator. Yet another input may be various bounds which the bolus calculator should not exceed in its recommendations or advice. Such bounds can be based on, e.g., best estimates, worst-case scenarios, and so on. Such bounds provide an extra degree of safety to the bolus calculator and its outputs. Other inputs are as described above in Fig. 16 as well as in the examples below.

Where insulin-on-board values are output, in many cases the user is less interested in the particular value than whether or not they are in a good or bad state. For example, users generally desire to know if they are okay, should they eat, or should they bolus. Thus, outputs according to present principles may communicate this status quickly by the use of colors, e.g., traffic light colors. In this case the analyses need not just take insulin-on-board into account. For example, to achieve a more accurate depiction of the correct state, the calculation may also include EGV as well. For example, referring to the below table I, outputs may be based on inputs as follows:

Table I

| EGV | IOB | Status | Output Color |
|---|---|---|---|
| High | High | Good | Green |
| High | Low | Use caution | Yellow |
| Low | High | Bad | Red |
| Low | Low | Use caution | Yellow |

Outputs can also depend on the input of "mode", whether the mode corresponds to calibration, type of decision-support, and so on. In more detail, the description above describes modes with respect to inputs, but the output user interface, as well as the input user interface, can also be based on mode.

Modes of calibration and decision-support have been described in detail above with regard to inputs, and such description is not repeated here. However, certain types of modes are especially related to outputs, and are described below.

For example, output modes may include whether the decision-support application/functionality is running in a hierarchical mode versus a nonhierarchical mode. In a hierarchical mode, input data is used as it is available, and as more data becomes available, a different tier in a hierarchy may be entered. For example, the hierarchy may refer to a hierarchy of safety, where different tiers have different "buckets" of risk. Users may move up or down through the hierarchy as the safety profile changes, which is in many cases related to the input data available.

In more detail, a hierarchical approach to algorithm design may be employed such that the algorithm is designed to use information obtained from different sources in a layered approach. Bare bones algorithms may use CGM and as the decision-support application/functionality receives more data, the additional data and information from these sensors can be layered in. The confidence in estimates and decisions is improved as the amount of information increases. Similarly, users or use conditions can be categorized in buckets or layers of risk. Each time information is available or added, the system moves up or down the hierarchy to ensure safety.

The hierarchical approach described can be exemplified by an "edge case" scenario in which its implementation enhances system safety. For example, a "stack up" situation may occur where, in this example, a patient with high insulin sensitivity is about to eat a meal and pre-prandial CGM glucose is high, e.g., 350 mg/dL. Accounting for CGM uncertainty, the true blood glucose may actually be 250 mg/dL. If the meal is large and the carbohydrate content uncertain, the patient may have overestimated the carb content. With this "stack up" of factors, i.e., this "edge case", the resulting insulin dose may pose a risk to the patient. In such situations, the system accounts for the risk and suggests to the user one or more of the following. The user may be requested to enter a more precise carbohydrate content. Alternatively the user may be requested to take a photo of the meal and upload the same. The decision-support application/functionality may inform the user that, in this instance, the dose will be less aggressive than usual, and the same may target a glucose value of 180 mg/dL, rather than 100 mg/dL.

In addition, if a particular patient is prone to hypoglycemic events because of behavioral tendencies, the decision-support application/functionality may respond by tuning the decision-support to be less aggressive. It should be noted that this tuning is distinct from tuning the decision-support application/functionality based on changes to insulin-to-carb ratio or insulin sensitivity factors, which are physiologic parameters.

In this way, safety is enhanced by accounting for inputs such as device uncertainty, physiologic uncertainty, and behavioral uncertainty.

Like inputs, outputs can be based on user-desired functionality, e.g., more discretion versus less discretion, more aggressive versus less aggressive, tied to a specific user goal, and so on. Outputs can also depend on time, e.g., may be different for different days of the week or seasons, and the same can extend to different types or content of user prompts with respect to meals, sleeping, exercise, school, and so on. As with respect to hierarchies of risk, outputs may depend on how much data the user enters or how much data is available to the system. For example, if the entered or input data is less detailed, the output may be less detailed.

Processing

Specific examples of processing are described below with respect to particular examples. However, here it is noted that processing can be "fuzzy", crisp, or can include a combination of both, particularly where functions or routines are provided to translate fuzzy inputs/outputs to crisp inputs/outputs and vice versa, as described above. The processing may occur within or outside the decision-support application/functionality, and the decision-support application/functionality can be implemented within a CGM map, a bolus calculator app, another control device, or a dedicated device whose sole or primary purpose is to operate the decision-support application/functionality.

The decision-support application/functionality and subsequent processing may be performed by an app running on a smart phone in the background all of the time, or the same may form a portion of another app, e.g., CGM, bolus calculator, or so on. Received inputs may include those described above. The processing may include comparing the received data with historical data or other stored data and/or with thresholds, comparing received data and other received data, and further comparing the same with respect to other thresholds, and outputting results accordingly, e.g., based on the comparison. Where the processing includes derived data, the derived data may be derived from within the decision-support application/functionality or within another app implementing decision-support, e.g., a bolus calculator.

The decision-support application/functionality can adapt processing to how the user likes or is accustomed to enter data. For example, some users desire to enter data as a number of carbohydrates, others as a meal size, and still others as a combination of both. Mappings, categorizations, or the like, can be used to convert one type of entered data to another, to employ more efficient processing, thereby making the computing machine operate more efficiently.

Where the processing is fuzzy, decision-support matrices may be employed as indicated below in the partially completed Tables II and III. In some cases in these tables fuzzy inputs are mathematically combined with non-fuzzy input variables and subsequently linked with outputs. Generally changes in the inputs lead to changes in the content of the outputs, and in other cases such changes lead to changes in the form of the output, e.g., its implementation as part of the user interface.

Table II

| | | Device factors | | Lifestyle/Situational factors | | | Physiologic factors | | |
|---|---|---|---|---|---|---|---|---|---|
| | Traj. | Device uncertainty / confidence | Type of monitoring | Calendar (upcoming appts, etc.) | Time of day | Meal to be consumed | stress | exercise | **OUTPUT** |
| Eu | ↗ | Low | CGM | MEETING | NOON | LUNCH | HIGH | LOW | **BOLUS 2X** |
| Eu | ↘ | Low | CGM | ... | ... | ... | ... | ... | ... |
| Eu | → | Low | CGM | ... | ... | ... | ... | ... | ... |
| Hypo | ↗ | Low | CGM | ... | ... | ... | ... | ... | ... |
| Hypo | ↘ | Low | CGM | ... | ... | ... | ... | ... | ... |
| Hypo | → | Low | CGM | ... | ... | ... | ... | ... | ... |
| Hyper | ↗ | Low | CGM | ... | ... | ... | ... | ... | **BOLUS & EXERCISE MORE** |
| Hyper | ↘ | Low | CGM | ... | ... | ... | ... | ... | ... |
| Hyper | → | Low | CGM | ... | ... | ... | ... | ... | ... |

TABLE III

| | | Activity | | Meal | | | Other factors | |
|---|---|---|---|---|---|---|---|---|
| | Traj. | Not Exercising | Exercising | Small | Medium | Large | stress | Upcoming Event? |
| Eu | ↗ | $^{11}IU_{DS}$ | $^{12}IU_{DS}$ | $^{13}IU_{DS}$ | $^{14}IU_{DS}$ | $^{15}IU_{DS}$ | $^{16}IU_{DS}$ | $^{17}IU_{DS}$ |
| Eu | ↘ | $^{21}IU_{DS}$ | $^{22}IU_{DS}$ | $^{23}IU_{DS}$ | $^{24}IU_{DS}$ | ... | ... | ... |
| Eu | → | $^{31}IU_{DS}$ | $^{32}IU_{DS}$ | $^{33}IU_{DS}$ | ... | ... | ... | ... |
| Hypo | ↗ | $^{41}IU_{DS}$ | ... | ... | ... | ... | ... | ... |
| Hypo | ↘ | $^{51}IU_{DS}$ | ... | ... | ... | ... | ... | ... |
| Hypo | → | $^{61}IU_{DS}$ | ... | ... | ... | $^{ij}IU_{DS}$ | ... | ... |
| Hyper | ↗ | $^{71}IU_{DS}$ | ... | ... | ... | ... | ... | ... |

(continued)

| | | Activity | | Meal | | | Other factors | |
|---|---|---|---|---|---|---|---|---|
| | Traj. | Not Exercising | Exercising | Small | Medium | Large | stress | Upcoming Event? |
| Hyper | ↘ | $^{81}IU_{DS}$ | ... | ... | ... | ... | ... | ... |
| Hyper | → | $^{91}IU_{DS}$ | ... | ... | ... | ... | ... | ... |

## EXAMPLES SECTION

The above description indicated various systems and methods according to present principles, including types of inputs, types of outputs, and processing techniques. The systems and methods include ways to tune or adapt a user interface to provide improved decision-support features to a user, including, e.g., defining a state or parameterized model of the user which is then used to drive the inputs and outputs, etc. The systems and methods are tuned according to user-desired functionality and in some cases data is transformed into a more quantifiable or objective form in order to perform certain calculations. Data may also be categorized or "fuzzified" and used in fuzzy logic calculations. In other implementations, it may be more advantageous to use a model where DSI#, DS#, and DSO#, are altered based on various parameters.

To provide a better understanding of systems and methods according to present principles, a number of examples are given below, to various levels of detail.

The examples may be thought of as occupying various levels of therapy. In a first level, systems and methods according to present principles act as a bolus calculator. In the field of bolus calculators, multiple types of inputs may be provided. In present systems and methods, the bolus calculation is based on such inputs, but also on a lifestyle parameter or a state of the user. The system may set up appropriate initial parameters, and the same may be revised over time according to patient feedback and patient performance.

In a second level, providing a different level of therapy, systems and methods according to present principles act as a bolus calculator but may also provide other sorts of user prompts besides just bolus calculations.

In a third level, providing yet a different level of therapy, systems and methods according to present principles may be provided in particular for type II diabetics, or those desiring primarily behavioral control and suggestions. In this level, behavioral insights and therapies may be provided, with less emphasis on dosing, e.g., insulin dosing. However, even in the third level, bolus suggestions can be provided. For example, if the user is a type II diabetic and is insulin-dependent, but has a relatively "normal" regimen, the "normal" bolus may be modified slightly by a user prompt, even in this third level.

### FIRST LEVEL - BOLUS CALCULATOR IMPLEMENTATIONS

In many of the examples below, the user's lifestyle goals may be to have better glucose control by making better estimations before meals, by providing feedback after meals, by providing feedback or suggestions before bed, but to avoid disturbing the user during meals or bedtime unless necessary. Such goals may be mapped with timing elements, e.g., by integration with calendars, mapped with GPS data, e.g., the user location may indicate mealtime or whether they are at home, or through programming or learning and/or using other patterns to optimize timing and parameters for interactive decision-support in synchronization with goals.

#### Example 1- Eating a Meal / Meal Bolus / Post Meal / Correction

A diabetic may have several or many desires or goals, even when performing the rudimentary daily task of just eating a meal. Particular goals may include the avoidance of pre-meal or postmeal spikes or lows, a desire to quickly and discreetly dose insulin in public without drawing undue attention, a desire to eat a meal without any particular planning/-calculations/thinking, or just in general to eat a meal with others without their diabetes getting in the way, which could include, in particular, without having to delay their meal as compared to his or her nondiabetic colleagues. Some of these goals may even conflict; for example, a user may have to delay their meal to give time for their insulin to kick in.

By using systems and methods according the present principles, which implement the decision-support application/functionality described, the user may enter a particular goal e.g., as part of a prioritization, which the system then uses to base therapy prompts. For example, the user in the above scenario may believe that avoiding postmeal spikes/lows (lows caused by overreacting to spikes) is the most important goal, because the same can potentially be more embarrassing, and thus the decision-support application/functionality operates in a way so as to emphasize the avoidance of lows. The decision-support application/functionality may accordingly emphasize certain goals in bolus calculator determinations, or other such therapy prompts.

As a specific use case, a user may wish to eat pizza with her friends for dinner. As a diabetic, the user is aware that she should check her blood glucose and the trend. In this example, the user may check her blood glucose and the trend on her CGM application incorporating decision-support application/functionality. The user may then desire to know what would be the appropriate insulin dose for the pizza. However, the user may wait until the pizza arrives before the bolus, risking a potential high due to a late dose rather than a potential low because she dosed too early.

Incorporating the decision-support application/functionality according to present principles, the system may provide a therapy prompt to the user such that she does not go too high or too low, based on, e.g., what she is eating, where she is eating, who she is eating with, and so on. The therapy prompt may further be based on a state the user is and, as defined by the system (Step B), and determined for the current circumstance (Step C) by combination with a real-time input (about to eat pizza). Rather than having the user rely on mental math and guesswork, e.g., how many carbs are in a slice, how much for a correction, and the like, the system may perform these steps for her. For example, her CGM app may inform her of the best time to dose for her upcoming pizza meal. In some cases, using GPS, pattern data, or the like, the system may accurately guess that she is going to eat pizza, rather than having such data explicitly entered into the app. The user may confirm the dose recommendation and confirm the dose has been delivered. In this case she may eat her pizza right when it arrives, with just enough time for her insulin to properly kick in.

Similarly, postmeal, and as a trigger, the user may believe she has a high blood sugar, and accordingly may feel sick or dehydrated. The user may check her CGM app and see that she is at 250 mg/dL and is trending with two up arrows. In the past, the user may wonder why she is so high, and may decide to dose more short acting insulin. For example, she may wonder if she did not dose enough short acting insulin for her meal, she may wonder if she did not dose enough long-acting insulin in the morning, she may wonder if the insulin was bad, or if it was injected into a bad site, she may wonder if the insulin has reacted yet, and so on. In any case, she may dose/inject an arbitrary amount of short acting insulin, e.g., 1-2 units, although she may not feel confident in her dose calculation.

However, incorporating the decision-support application/functionality according to present principles described above, the user may be provided with a much more accurate dosage, based on state learning and determination, decreasing glycemic variability and moreover decreasing user anxiety and worry. For example, systems and methods according to present principles may determine, based on the inputs noted above and, e.g., historic data, that the site she used for her previous injection must be bad (a previous "bad site" may have been learned using the functionality). The decision-support application/functionality may suggest she give a correction injection, and may recommend different injection sites.

Similarly, when the user is getting ready for bed, she may encounter a low alert, and may consequently open her CGM app, realize she is at 80 mg/dL, and trending downward. Her understanding may be that she stacked her insulin after dinner, and is now worried about going low while sleeping. In the past, a solution may have been to correct her low by ingesting significant amounts of sugar. The user may even delay her going to bed to wait for her correction to kick in, e.g., she may wait for a trend arrow to flatten out or start trending up.

Systems and methods incorporating the decision-support application/functionality may accordingly be employed to inform the user what she should do in a more accurate, controlled, and personalized way to raise her blood sugar to her target nighttime range. Using such systems and methods, the user need not wait to go to bed; the system may inform her if her correction was effective or not.

Alternatively, using systems and methods according to present principles, where the glucose response has been better modulated by the decision-support application/functionality, the user may encounter no such problems getting ready for bed.

Further to this example, the user may be awoken in the middle of the night with a high alert, and upon checking may see that her glucose is at 210 mg/dL. Her belief may be that she overcorrected her low and that, although she is currently high, she would rather get alerted at 250 at night. The user may dose a small arbitrary amount of insulin to correct her high, as she may recognize she is too tired to do the mental math and desires to not dose too much. Using systems and methods according to present principles, the user may be prevented from having to do mental math, and may instead provide her a therapy prompt as to how much to dose so that she can easily correct her high and go back to bed. In systems and methods according to present principles, the user may in addition be not alerted at night unless it is a severe high and/or could lead to discomfort or ketoacidosis, requiring in many cases an injection for treatment.

Alternatively, using systems and methods according to present principles, the user CGM app may read that she is running somewhat high during sleep but may not alert her because the user has indicated a goal of wanting her sleep uninterrupted unless she is severely high or low. Accordingly, using systems and methods according to present principles, the user may continue to sleep soundly.

While certain inputs are described above, generally required inputs for meal bolus calculator determinations include carbs, target glucose, current glucose, insulin-to-carb ratio, insulin sensitivity factor, a meal bolus equation, and so on. Other inputs which may be employed include time of day, exercise, glucose rate of change, stress, illness, insulin-on-board, and so on. It is noted in this regard that a particularly challenging input for meal bolus determinations is carbs, as it is often difficult to know the exact number of carbs in a meal.

Example 2- Playing Sports or Exercising with Friends

A diabetic user may desire to play sports with her friends. Goals associated with such sports may include, e.g., the avoidance of lows or highs during a workout, the avoidance of lows or highs after the workout, the prevention of her diabetes from affecting her performance in the sport, a desire to not spend significant time and effort planning accounting for diabetes in her workout, a desire to focus on the workout, without interruption or embarrassment by diabetes, a desire to does discreetly if a high is encountered, and so on.

The decision-support application/functionality may accordingly emphasize certain goals in bolus calculator determinations, or other such therapy prompts.

In a more specific example, a user may be about to play an intramural soccer game with her friends. In current practice, she may check her blood glucose and trend, e.g., on her CGM app. She may see that she is in her target range and is steady. The user may desire to know if she should give herself some carbs or some insulin before her soccer game. In the past, she may take a small snack (with an arbitrary or unknown number of carbs) so as to avoid going low during the game. She also desires to not go high, but would rather go higher than low.

Subsequently, the user may feel that she has a high blood sugar during her soccer game, and may wait until halftime and check her CGM app during the same. The CGM app may indicate to the user that she is high, e.g., 250 mg / dL. The user may subsequently perform some mental math to figure out what her correction should be for her high glucose value. She may decide to give herself a shot with a little less insulin rather than doing a normal dose, cutting back because she does not want to go low during the game.

Nevertheless, if the user continues to feel like she is going low, she made sub out of the game and check her blood sugar. Upon a determination that she is low, the user may believe that she has overcorrected for her high glucose earlier in the game, and next is faced with a decision, often no more than a guess, as to how many carbs she should take to correct her low blood sugar and not go high. Even with this, the CGM app may provide a low alarm, which the user may believe is a delayed low from her earlier exercise, as exercise tends to have a delayed, but difficult to predict, effect on blood sugar. Again the user must guess at a number of carbs required to correct her blood glucose.

Thus, systems and methods according to present principles may avoid the problems of the prior art noted above in many ways. First, systems and methods may inform the user as to how to prepare for her exercise so that she does not go too low or too high during the same. Other inputs into this analysis may include a smart watch or other indicator of, e.g., stress (for example, losing the game may cause additional stress). Systems and methods may further allow the user to not have to leave her game to figure out if she is high or low, and may further indicate a more accurate amount of insulin that should be dosed to correct her high, but which also prevents her from going low later on in the game. Systems and methods according to present principles may further display additional information as to why she went high, and may indicate to her how to avoid this in her next game, increasing and building confidence and trust in the system. The same may further inform her as to how many carbs she needs to correct her low and finish her game. Finally, systems and methods may further help the user prevent delayed lows and highs from exercise; while if the user does go low, the systems and methods may accurately inform her how many carbs are necessary for correction.

For example, using the systems and methods informed by the inputs noted above, the users CGM app, employing decision-support application/functionality, determines, in this example, that she is in a "weekend soccer game" state and adjust parameters for therapeutic decision-support accordingly. The system accordingly indicates to her that she should dose a small amount of insulin because she is about to play in a soccer game and that she tends to go high during soccer games. The system is also aware that she sometimes goes low in practices and other workouts. In some implementations, systems and methods according to present principles may guess that she is about to play in a soccer game due to a calendar input, a recognized pattern, user input, and so on, and may also be configured to differentiate games from practice. The user may then dose with the recommended amount.

In an alternative example, the user may not dose before the game, but may consequently go somewhat high. She may not notice this until she goes to the bench at halftime. The CGM app may recognize that she is not currently playing, e.g., by an accelerometer input, and may display to her a high alert. Upon opening the CGM app, she may see that she is high and may see that the app recommends she should correct it with a slightly smaller than normal dose of insulin. Knowing the inputs as described above, the CGM app is aware that the user is still playing soccer and is not want her to overcorrect. With the trust in the system built according to present principles, and as described above, the user feels comfortable dosing the recommended amount because she does not want to go any higher and have her high blood glucose affect her performance, but is also confident she will not go low.

Subsequently, in an example where the user goes low during her game, she may hear her low alarm go off on the sideline. As she is aware that her CGM app will not alert her and interrupt her game unless the situation is serious, she checks her blood glucose on her CGM app even if the same requires her to substitute out of the game. The user may see that she is fairly low, and her CGM app may recommend that she take some fast acting carbs to correct it. Her app, knowing the user's patterns and with knowledge of the determined state, knows that her goal would be to continue playing in the soccer game. Accordingly, the same may provide an indication on the user-interface that the app will alert her when her blood glucose is good enough for her to go back in and play well. The user may then take the recommendation and eat a snack and wait for her blood glucose to rise.

After the game, the app may warn the user that she may see a drop in her blood sugar in a number of hours because her blood glucose normally drops after that amount of time after a soccer game. The app may further indicate that it will recommend slightly smaller doses of insulin if she decides to eat within that timeframe. The app may further alert her to, e.g., eat a small snack three hours after her game if she takes no other actions. In this way, the user may be confident she will not go low later on due to her soccer game. The user then acknowledges the warning.

After a period of time, e.g., three hours later, the user receives a therapy prompt to eat a snack. Using the therapy prompt, the user may eat the suggested snack because she does not want to go low later on due to her soccer game.

Example 3- Pre-Sleep Decisions

A diabetic user may desire to control their diabetes in such a way as to optimize their sleeping habits, e.g., to have confidence she will get a full night of sleep and wake up refreshed in the morning, to avoid lows during sleep, to prevent diabetes from interrupting sleep, to wake up with an in-range EGV, but still to be alerted if necessary of serious lows.

The decision-support application/functionality may accordingly emphasize certain goals in bolus calculator determinations, or other such therapy prompts.

In more detail, and in the past, if a diabetic user is about to go to bed, a common action is to check their blood glucose and their trend data, e.g., on their CGM app. Even if they are in their target range and trending in a steady manner, the user may wonder if they are going to go low or high during the night. For example, exercise or meals may affect their nighttime glucose value. They may have a goal to not wake up in the middle of the night to correct a low or high glucose, but there may be no way to accurately and controllably address these issues.

In a specific example, the user may be woken by a low alarm from her CGM app and may subsequently check their glucose and their trend, e.g., on their CGM app. From the CGM app, the user may see that they are low and trending down. The user may in turn wonder how many carbs are needed to correct this, with the ensuing fear of under correction with the risk of going too low, and overcorrection and going too high, particularly when the user has also been associated with a "dawn phenomenon", which would cause them to go even higher after breakfast. The user may, in this prior situation, quickly guess how many carbs are needed to correct her blood glucose and may even give herself extra to prevent her from going any lower.

Subsequently, a few hours later, the user may be woken again by a high alert from her CGM app. Again, she would generally check her blood glucose and trend on her CGM app. She may see that she is high and trending up, and believe that she overcorrected for her low. At this point many users would give themselves a shot of insulin, but in this case she may be unaware of how much insulin is proper, and may further be afraid of over correcting and going low again. Accordingly, she may give herself a shot with a slightly smaller correction.

The following morning, the user may wake up and see that she is still high. In prior systems and methods, the user may be left wondering if they are high because they under corrected or because of the dawn phenomenon. They may desire to prevent such from happening again, and may further have questions regarding appropriate dosages for breakfast, whether they should allow a correction to kick in before eating breakfast, and so on. Again they are required to perform mental math for their dose. In some cases, the user may wait for her blood sugar to drop back to normal before eating. In any case, there is an almost constant need to check the CGM app to see if she is normal or still high.

Thus, in systems and methods according to present principles, it is desired to more accurately and informatively tell the user whether she should eat something or dose in order to prevent lows/highs while asleep. Such enables the user to feel more confident that their diabetes will not interrupt their sleep, and further make her feel confident that she will wake up refreshed in the morning. The systems and methods may further tell the user how she may correct her low and not go high later in the night. Such systems and methods may advantageously avoid the need for the user to do mental math, especially at night, and especially after being woken up by an alert. Such systems and methods may further tell the user how to correct a high blood glucose in the middle of the night and further may be employed to reassure the user that she will not go low after her correction. Such systems and methods may further inform the user as to why they are low or high and give guidance on how to prevent future lows/highs from similar causes. The systems and methods may further tell the user how to correct the high blood glucose and how to dose for upcoming meals.

In more detail, in systems and methods implementing a decision-support application/functionality, the same may determine a defined state the user is in and employ the same in dose determination. The user may still check their blood glucose and trend on their CGM app, but in this case her app may tell her, with knowledge of her state, that she is expected to go low in a couple hours due to her soccer game, even if she is initially in her target range and trending steadily. Thus the app may recommend she take a snack prior to sleeping.

In one extension of this example, the user may be woken by a low alarm from her CGM app, and may subsequently check her blood glucose and trend on her CGM app. In systems and methods according to present principles, she may see that she is low and trending down, and may further see a recommendation on how to control her low blood glucose and how not to overcorrect. She may accept the recommendation of the therapy prompt and correct her low blood glucose quickly, without having to do mental math.

In another extension of this example, the user may be woken again, in the early morning, by a high alert from her CGM app. Upon checking her blood glucose and trend on her CGM app, she may see that she is high and trending up. Her app incorporating decision-support application/functionality may tell her why she is high, with knowledge of her state, and may tell her how to correct the high and prevent it from occurring in the future. The user may see that she can be reminded at later times to make these adjustments. The app may also suggested dosing for her breakfast, and may further suggest a particular amount. The user follows these suggestions and makes long-term changes to prevent future morning highs. She may further agree with the breakfast dose recommendation as she is about to eat that meal, and may dose the recommended amount, and may further provide user input telling the app to remind her to adjust her basal dosing at the appropriate time.

Example 4- Pre-Driving Decisions

A diabetic user may desire to control their diabetes in such a way as to optimize their driving experience, e.g., to avoid lows during driving, to prevent diabetes from interrupting driving, to limit distractions when driving, to be alerted before lows happen, and so on.

The decision-support application/functionality may accordingly emphasize certain goals in bolus calculator determinations, or other such therapy prompts.

In a specific example, a user may be about to drive to her friend's house. She may consequently check her blood glucose and trend information on her CGM app. She may see that her current blood glucose is in her target range and is steady. She may believe she is normal now, but may wonder if she is going to go low while driving. She does not want to drop unexpectedly and have to treat while on the road. Thus, in this prior art example, she does not either dose or eat but simply commences driving.

In a particular example, she may hear a low alert from her CGM app during the drive. Thinking she is low, she pulls off to the side of the road to check her blood glucose on her CGM app. She may see that she is low and trending down. In this prior art example, a user generally treats the low with whatever they have in their car and waits for their blood sugar to return to normal before commencing driving again. Accordingly, she may have a snack from her car and wait. If she does not have a snack in her car, she will wonder where she can quickly buy a snack to treat her low, as well as how to get to such a location, and may simply be left to hope that there is no traffic. In this case, if she has a smart phone, on the side of the road, she may try to find a nearby gas station on her GPS and may travel there to buy a snack. In more dangerous situations, depending on blood glucose, she may have to call 911.

Systems and methods according to present principles may be employed to help such a driver make important pre-driving decisions. For example, the systems and methods prevent the user from future lows or highs when she is driving, e.g., by an action taken before she leaves her house. The systems and methods inform the user how to treat her low blood sugar as quickly as possible so that she can get on the road again. The systems and methods may further indicate to the user where she can get a stack to treat her low if she has no snacks in her car.

In more detail, systems and methods incorporating decision-support application/functionality predict that she will go low during her drive to her friend's house, and the same would recommend she should eat a snack before leaving.

In the case where she still encounters a low, e.g., she hears a low alert from her CGM app while she is driving, she will likely still pull off to the side of the road to check her blood glucose on her app. In this case, her app displays a therapy prompt informing her how to correct appropriately so that she can get back on the road as quickly as possible. Her app may further indicate where to purchase snacks based on knowledge of her desired snacks and stores in her proximity. Again, she may eat a snack from her car and wait for her blood glucose to go back to normal before driving again, although in this case the recommendation will be tuned to her needs and user desired functionality. For example, upon acknowledging the alert, she may agree to redirect her GPS to the nearest gas station without having to stop or take her hands off the steering wheel. Consequently, she begins to receive turn by turn directions to the gas station with the earliest ETA.

Example 5

In another example of the decision-support application/functionality including a bolus calculator or "bolus wizard" functionality, the determination of states in a state model provides for such functionality by optimizing bolus calculator parameters, including ISF, ICR, and other parameters including total daily dose.

In one implementation, an improved or optimized total daily dose may be calculated. In addition, calibration values may be entered once for both bolus calculator and CGM monitoring purposes. For example, in some implementations the decision-support application/functionality includes a bolus wizard as part of entry of calibration values, leading to less repetitive user interactions, such as entering in target glucose ranges and BG values, and thus leads to more accurate insulin doses. The bolus wizard parameters may be automatically updated with trend data. Such trend data may in turn improve the Duration Of Insulin Action (DIA) and parameters related to the Total Daily dose (TDD).

For example, an improved total daily dose iTDD may be given by:

$$iTDD = TDD + \left[ \frac{\left( MeanBG - \frac{144mg}{dl} \right) \times 2.5}{\frac{1960mg}{dl} \div TDD} \right]$$

The iTDD is used in determining basal rates and bolus carb factors as follows:

$$Basal \left( \frac{U}{day} \right) = iTDD \times 0.48$$

$$CarbF = \frac{2.6 \times Weight(lb)}{iTDD} \; or \; \frac{5.7 \times Weight(kg)}{iTDD}$$

In related implementations, insulin dosage may be added as an event marker automatically in retrospective trend graphs. Insulin-on-board, calculated in known ways or in ways described above, may be incorporated into trend predictions, e.g., both in trend graphs and predictive alerts.

As noted above in the output section, the subject user, i.e., the "sharer" (of their data) may control followers' ability to view certain types of insulin data during setup or subsequently, e.g., basal rates, boluses, calibrations/BG values, and so on. Parents may be enabled to check sensor accuracy with user-entered BG values, thus being relieved of the necessity of constantly inquiring of their children as to whether they checked their blood sugar or not.

In some implementations, the app on the follower device may be provided with a bolus calculator, and the bolus calculator can be automatically triggered from calibration events in the trend graph. For example, followers can press on a calibration event marker and then they can choose to use the bolus calculator. Followers such as parents may view BG values, and suggest insulin doses through the app. The children can confirm or deny the recommendation/advice. The app may further prevent followers from suggesting boluses a certain amount of time after the calibration event, e.g., 5 - 10 minutes.

Example 6

As noted, bolus calculators may conveniently take advantage of data available from CGM to achieve particular convenience and accuracy for a user. In a particular implementation, a continuous contextual bolus calculator may be provided. In such a bolus calculator, the decision-support application/functionality may include an insulin absorption profile and a determination, or a facility to determine, insulin-on-board. Food or meal information may be provided by the user or by other meal input methods described above.

In this implementation, whenever the app is opened, the main screen may display bolus information in units for current EGV. If no correction is needed, an indicator may indicate such, or in another implementation no information may be displayed. In the case of a high alert, the alert notification may display bolus information in appropriate units if needed. Taking a bolus records the bolus event and sends notification of the same to, e.g., followers. The user may be provided an option to change the bolus event units. Upon delivering the user-specified insulin, the pump may communicate the bolus to the app, e.g., for insulin-on-board calculations. A user prompt may then ask the user to confirm the bolus information.

Example 7

In another bolus calculator implementation, it is noted that many insulin pumps show insulin-on-board (IOB) and have bolus calculators to help patients estimate future insulin needs and the impact of their current insulin levels on future glucose levels. Further, when market research is performed on this patient population, their expectations for incorporating insulin data into CGM apps is no more sophisticated than just adding IOB data. But in many cases this is suboptimal as the same is not directed to what the users really want to know which is: (1) whether they need insulin, (2) how much do they need, and (3) if they do not need insulin now, do they have too much insulin in their body and will it cause their glucose to drop dangerously low.

Thus, systems and methods according the present principles further relate to a running or real-time bolus calculator based on the information available. Instead of IOB, the systems and methods may display a live estimate for the bolus calculator that incorporates the insulin needed for correction and for meal coverage. Where additional information is available, the system may break down the components of the insulin bolus so that the user understands the breakdown between correction and meal coverage.

One advantage of this system is it further automates and simplifies the insulin calculation process for users and eliminates the need for users to make estimates based on IOB. Further, in cases where the bolus calculator is negative, the system may still show extra IOB, i.e., how much insulin will cause the user to drop lower than their target, or even automatically switch on a predictive low glucose feature. In this case, the prediction feature is primarily turned on because of the additional insulin data/knowledge available. In some cases, of course, insulin data may be unavailable, and in such cases, the system may still estimate the correction insulin needed, and based on historical glucose rises, due to meals and the subsequent glucose response due to an unknown insulin delivery, the decision-support application/functionality could learn a typical meal for the user and make estimates to still enable an effective real-time bolus calculator.

Example 8

In yet another bolus calculator implementation, it is noted that incorrect bolus recommendations can occur because the patient misestimates the amount of carbohydrates being consumed, the bolus calculator settings are not adequately tuned to the individual patient, or life events such as stress and illness temporarily change a patient's sensitivity to insulin. Incorporating a bolus calculator into a CGM system has the unique safety advantage of real-time alerts that enable people to safely treat high and low glucose levels caused by incorrect insulin boluses.

In a particular implementation, decision-support application/functionality may be implemented by a CGM system in data communication with a smart device running a mobile application. In some cases an insulin pump or pen may also be in data communication with the CGM system, the smart device, or both. The app may read, store, and visualize the insulin information, and also upload the insulin information for diabetes management retrospective software, as well as to a share system for remote monitoring by followers. The decision-support application/functionality may be implemented by the techniques described above, including the definition and termination of states associated with a patient.

The system may include a sensor, transmitter, receiver, and mobile app. The sensor is inserted into subcutaneous tissue where it continuously measures glucose concentration. The transmitter is connected to the sensor and uses an onboard algorithm to convert the sensor signal into glucose measurements. The transmitter communicates with the receiver and/or the mobile app using Bluetooth Low Energy (BLE) technology. The transmitter sends glucose values every 5 minutes and receives reference capillary blood glucose measurements when entered by the user for calibration. The receiver and mobile app display glucose readings and alert the user when glucose levels are outside of a target zone. The mobile app additionally provides connectivity to a share service for remote monitoring.

The CGM app can perform calculations and display clinical values of measured glucose, and can further perform other features, e.g., the reception of older glucose values from the transmitter following periods of missed communications to fill in data gaps on the trend graph, e.g., EGV data backfill. In addition, a user can configure different CGM alerts for multiple customizable time periods, e.g., day and night. A user may select a "mute switch override" option with a user-specified override volume for an urgent low alarm, e.g., 55 mg/dL, within a customizable time period. For example, the user might select to only use "mute switch override" for urgent low alarms during the day when their phone is on mute during meetings. A user may also select vibrate only for each CGM alert, except the urgent low alarm. Actionable notifications may be provided on a phone lock screen as well as on a smart watch, presenting different button options to users to allow feedback to the app without having to enter the app. With actionable notifications the user may acknowledge a CGM alert without opening the CGM app. For calibration notifications, the user may be enabled to select an action such as "enter blood glucose" and be taken directly to the part of the CGM app that allows blood glucose entry. For CGM threshold alerts, users may be given the option to "log carb or insulin events" without entering the CGM app. Customizable icons and text may be employed to make the user's current glucose state immediately visible on smart watches and smart phones. The current glucose state may in some cases have a lower resolution than the current glucose value, and in some cases may indicate whether the user is low, in range, or high.

A feature providing quick statistics may be employed on the CGM app and smart watch implementation to provide the user with glanceable, basic statistics about how they are doing with their diabetes management. The summary statistics may include the estimated A1C and percent time spent above, below, and in target range. The metrics may be computed, e.g., for the past day and past week of sensor wear. The target range selection may be different from the CGM threshold alerts selections.

Activity information, such as exercise, meal, and insulin, entered into other apps may be imported through an appropriate API, e.g., Apple HealthKit, and automatically logged as events in the CGM app. The user may then be able to view these additions on a landscape trend screen (see Fig. 24), which may be particularly useful in reflecting on diabetes management decisions made during that day. Such information may also be uploaded for diabetes management retrospective software into the share system for remote monitoring. Insulin information may be transmitted from a pump or pen, e.g., running an insulin app, to the CGM app, over a standard insulin app to app data sharing protocol. The user may, on a user interface of the CGM app, select from a menu of available insulin apps, selecting the one that corresponds to the insulin device they are using. The CGM app may then establish a secure link to that particular app and exchange data using published API standards. A server may provide details for each insulin app including the model of insulin device, the name of the device, and so on. Once the apps are linked, the CGM app can import real-time insulin device data for display and/or for retrospective analysis. Insulin-on-board and insulin device status can be displayed on the main trend screen, a "today" widget, and/or a smart watch. Insulin data can be employed as noted above to assist in real-time decision-making. In cases where only CGM data is received, insulin-on-board may be calculated from insulin dose as entered into events, or through the use of the CGM app bolus calculator, described below.

The user may switch from the CGM app to the insulin app by, e.g., tapping on an appropriate icon.

Insulin information received from the insulin app may also be displayed on the landscape trend screen, e.g., Fig. 24, and may be useful in reflecting on diabetes management decisions made during that day. For the CGM only system configuration, a historical insulin display may be provided for insulin doses entered into events or obtained through use of the CGM app bolus calculator. The insulin pump can send bolus calculation results, via its insulin app, to the CGM app for retrospective analysis. The CGM app may upload insulin information received from the insulin app for retrospective diabetes management and to "share" systems for remote monitoring.

The insulin pump may command the CGM app on the smart phone to vibrate, play an alert sound, and/or display an alert message, and to receive an acknowledgment that the user has seen the alert. In this way, the insulin pump can generate alerts and alarms.

Referring to Figs. 25A and 25B, and as noted above, the CGM app may include bolus calculator functionality. The bolus calculator will help patients determine how much insulin is needed to cover a meal and to correct for a high glucose level. Automating the complicated arithmetic used for insulin dose calculations reduces the burden of mental math for patients, minimizes mistakes, and helps patients achieve better glucose control. While bolus calculators are available on insulin pumps, patients using multiple daily injection therapy (CGM only system configuration or CGM with insulin pen system configuration) generally do not have access to an FDA-approved bolus calculator and so will especially benefit from this feature.

The CGM app allows the user to enter the amount of carbohydrates being consumed and their current meter blood glucose level. The same performs bolus calculator arithmetic and determines the total amount of insulin needed to cover the meal, to correct for a glucose value that differs from target glucose, and account for insulin on board. The bolus calculator additionally adjusts the insulin bolus based on the CGM trend, which is an added safety feature.

In more detail, meal data, e.g., carbohydrates being consumed data, may be entered in a number of ways. For example, the user may enter whether the meal is small, medium, or large, and as described above, such "fuzzy" inputs are learned (Step A of Figs. 12(A) and 12(B)) to correspond to various quantitative meal values that are meal compositions including carbohydrates. Alternatively, meals may be accessed from a food library app, and in this case data about the meal may be entered from the same. Meal memory apps may be employed. Patterns may be recognized such that a typical morning meal carb amount (e.g., a typical breakfast) may be estimated. Typical meals may be stored by the user and recalled via a suitable user interface. Glycemic index/meal absorption may be employed as an additional category. Meal detection algorithms may be employed to ask about additional boluses that were not entered through decision support but which may factor into calculations as insulin on board. Other inputs include exercise, and the same may be automatically read in by other apps on a smart device, e.g., smart phone or smart watch. As noted, insulin information may be communicated from a smart pen or pump.

In one implementation of a CGM app bolus calculator that uses both the current CGM glucose value and trend to determine insulin doses, for example:

*Insulin Bolus = Carb Coverage + Correction + Trend Adjustment - Insulin-on- board*

Or alternatively:

$$B = carbs/ICR + (Go\text{-}Gt)/CF - IOB + ROC^{*}\ \Delta T/CF.$$

Where $\Delta T$ may be learned from data in the following manner: Fig. 27 (A) illustrates an exemplary meal bolus case with a positive rate of change, from which $\Delta G$ may be learned. After many cases, the plot of Fig. 27 (B) may be made, whose slope is $\Delta T$. It is noted that the slopes may be different for positive and negative rates of change.

In this system, a user may choose to enter a meter glucose value instead of the CGM value, particularly when CGM values are unavailable. The trend adjustment is included to take into account where glucose is headed at the time of an insulin bolus, which can influence where glucose ends up relative to target after a meal. The trend adjustment may be calculated as the current rate of change (mg/dL/min) multiplied by 20 minutes and divided by the patient's personalized correction factor (mg/dL/units). Thus, the trend adjustment is correcting for the expected glucose change in the next 20 minutes. Trend adjustments can be turned off in the bolus calculator settings, but may be defaulted on. No trend adjustment may be made when the trend arrow is unavailable.

Additional safety measures include capping fast rates of change at 3 mg/dL/min (thus never correcting for more than a 60 mg/dL change) and not applying a trend-based correction in the two hours following a meal when active carbohydrates are expected to cause high positive rates of change.

Bolus calculator parameters generally include insulin-to-carb ratio, correction factor, glucose target, and insulin action time, although other parameters can be included as described here, including state.

In a particular example, systems and methods according to present principles are directed toward a method of performing a bolus calculation, in which bolus calculation parameters have been set including an insulin to carb ratio (ICR), insulin sensitivity factor (ISF), and target glucose value Gt. The method includes steps of receiving an input C indicating carbohydrates; receiving an input IOB indicating insulin on board; receiving an input Go representing a current glucose value; and determining an insulin bolus based on ICR, ISF, Gt, C, IOB, Go, and a rate of change of the glucose value.

In a variation, the rate of change is determined using values obtained by CGM. The rate of change of the glucose value is taken into account by way of a trend adjustment proportional to the rate of change and inversely proportional to a correction factor. The trend adjustment [units] = rate of change [mg/dL] * 20 [minutes] / correction factor [mg/dL/units]. The bolus determined is calculated by: $B = C/ICR + (Go-Gt)/ISF - IOB + ROC * \Delta T / CF$, where ROC is the rate of change of the glucose value and CF is a correction factor. And a non-transitory computer-readable medium may be provided, including instructions for causing a computing environment to perform the method above.

The CGM app can offer retrospective insights to patients on inappropriate bolus calculator settings, which are added safety benefits enabled only with the use of CGM. An example popup pattern report presented within the app is shown in Fig. 26, which points to a potential problem with the user's breakfast settings and suggests that the user.

In the CGM only system configuration, the bolus calculator may rely on user input of insulin doses to determine insulin-on-board (IOB). Insulin dose recommendations from the bolus calculator can be accepted or modified, and the same may then be used in future IOB determinations. Users can enter any other insulin doses they take into Events, and these may also be used in IOB determination. Users will be warned during initial setup that the bolus calculator relies on their insulin dose input for accurate estimates of insulin-on-board, and that there is a risk of dangerous insulin recommendations if they do not enter this information. During real-time use of the bolus calculator, the user may also be shown their last insulin dose, which can be updated if it is incorrect. Trend adjustments offer additional safety mitigations for incorrect estimates of insulin on board by reducing the suggested insulin dose when glucose is falling, i.e., when insulin is acting. When the CGM app is linked with an insulin app, the current insulin-on-board may be taken directly from the insulin app.

If the user chooses to use another meal database app to aid in carb counting, the CGM app may automatically pull those entered carbs from, e.g., Apple Healthkit. When the user accesses their bolus calculator, recently entered carbs may be presented to them and the user can choose to use this amount in the bolus calculation.

For those users who do not wish to count carbs and instead eat consistently sized meals each day, their HCP can elect to use "preset meal boluses" during initial setup and set typical breakfast, lunch, and dinner meal bolus amounts for their patient. The bolus calculator may recommend an insulin bolus that also accounts for glucose correction, trend adjustment, and insulin-on-board. The bolus calculator may also be informed by any determined state associated with the user (Step B) in performing a bolus calculation (Step C).

The CGM app bolus calculator in some implementations provides recommendations that are different from a bolus calculator on an insulin pump. This can be because of a difference in settings, insulin-on-board calculation, use of glucose trend, and other related factors, including the lifestyle factors described above. The user may be provided with a warning during initial setup informing them of these potential differences. In addition, for connected pump system configurations in which the insulin app provides its own bolus calculator, the CGM app bolus calculator will be deactivated to avoid user confusion. In this case, when the user presses the bolus calculator button in the CGM app, they will be taken to the bolus calculator in the insulin app, which is designed by that insulin device manufacturer.

User inputs to the bolus calculator and recommended bolus outputs may be uploaded to the cloud or a server for various purposes, including retrospective diabetes management and remote monitoring by followers. In addition, providing CGM and bolus/ basal data to a server enables messaging/e-mailing services to the provider and back to the patient. In some cases, the messaging/e-mailing may be triggered by a bolus change.

Various aspects of bolus calculator set up have been described above, e.g., insulin to carb ratio, preset meals, and the like. Personal lifestyle goals may also be included in the setup, e.g., desired exercise goals or the like, and the same may be entered into the CGM bolus calculator app through a series of set up questions. The bolus calculator could learn state information as described above, e.g., can learn a set of ISF profiles for a given user.

The use of trend or rate of change data has also been described, and variations of this will be understood. For example, in one variation, rate-based adjustments may be applied only when rate is unexpected, e.g., if there is no insulin on board but there is a negative rate of change. In another variation, the percentage adjustment of insulin on board may be based on rate of change. In another variation, trend arrow categories may be employed rather than continuous rate variations for simplicity for the user. In another variation, rate may be allowed to decrease bolus, but not increase it, thus only being used in a lower risk situation. In another variation, if the rate is positive, the user can be prompted to respond if they have already eaten and are delayed in calculating their meal bolus. In another variation, if the rate is positive, but is rising from hypoglycemia, then the meal bolus may be not increased for safety reasons. In yet another variation, rather than just using a trend arrow rate, the same may be compared to the two most recent point rates and the most conservative estimate used, i.e., the one that will result in the smallest insulin dose.

In other examples of the use of trend and other information within bolus calculators, if a high rate of change is detected and no meal is entered, the user can be prompted to determine a meal bolus. Users may be alerted if a meal rise is not seen in a reasonable amount of time following a bolus recommendation. If the user requests a bolus and their current glucose value is high, they may be given the option to set a temporary low glucose alert for the next, e.g., four hours, for safety. When CGM high alerts are given, correction bolus suggestions may be made. When CGM low alerts are given, carbohydrate suggestions may be provided if insulin on board is high, and the user is in a target glucose zone, but additional insulin onboard predicts that the user will become hypoglycemic, then an alert may be generated along with a therapy prompt that the user consume carbohydrates. If the user is high and the insulin on board is not large enough to cover, the user can be alerted that a correction bolus is needed and a correction bolus amount calculated and displayed as a therapy prompt.

As noted in the Inputs section, meal inputs are fuzzy. If the user gives a fuzzy meal size, e.g., small, medium, or large, then a default carb amount may be employed and the best carb amount learned through case-based reasoning for the different meals. This method may be employed instead of learning the insulin to carb ratio. In this case a glucose offset is addressed by additional insulin using an appropriate insulin sensitivity factor:

$$\text{Total insulin (original + additional)} = \text{carbs/ICR} + \text{(Go-Gt)/ISF} + \text{IOB}$$

Carbohydrate amounts may be learned from the above equation using ICR and ISF given for that case. From the above the user may learn that they get better control if they incorporate carbohydrate counting in their daily routine.

Besides bolus values, other therapy prompts may be provided to the user, as well as additional information. Certain information is described above with respect to Fig. 24, but referring to Figs. 28 and 29, other information can also be displayed. For example, charts can be displayed that show EGV along with bolus and carb values, on a day-to-day basis. EGV ranges may be shown e.g., high and low values, for a range of dates. Recommendation charts may be compared with charts based on previous cases, providing historical comparisons, along with appropriate date ranges. The comparisons may be on a day-to-day basis, showing the differences between the recommended EGV, bolus, and carbs. The charts may be provided with a "what if" analysis functionality. For example, models could be employed and used to illustrate how the EGV curve would change if the bolus or carb intake were changed. This functionality may incorporate machine learning to characterize the patient glucose response.

Other variations may include showing measures of "goodness"/"badness", along with recommendations. Reasoning may be included as noted above for recommendations, building trust in the use of the app.

For physician use, listings may be filtered to only show patients whose application parameters, e.g., ICR, require changes. Similarly, listings may be filtered to only show cases that require parameter tuning, e.g., ICR, for a particular patient.

Other variations include using the cloud or file sharing for exchanging data between the patient app and the clinical (HCP) app.

Advantages of the above implementation include mitigation of failure modes such as wrong or missing estimates of insulin sensitivity, current glucose, glucose target, insulin onboard, not accounting for rate of change, not accounting for meal composition, not accounting for time of day or timing of bolus, users requesting bolus when in hypoglycemia, pump failures, alert fatigue, and so on. For example, a particular method of mitigation includes disabling a "calculate bolus" button until carb entry has been filled in, which further differentiates meal boluses from correction boluses. Carb amounts may be cleared after insulin bolus calculation. Carb input ranges may be limited to a realistic range, or to a range associated with the user. To address wrong or missing estimates of current glucose, a finger stick may be requested, or a recommendation made that the user eat and then get a bolus recommendation when glucose has returned to euglycemia (the system could inform the user when this happens). To address meal composition issues, eight recommended dose may be split in two, and the system may prompt the user to use a lower dose for lower glycemic foods. The system may further provide an image of the type of foods they have eaten and further display to the user a chart of how their glucose behaved afterward. To address alert fatigue, the system may only require one confirmation (never double checking), confirm on a summary rather than individual inputs, only confirm if an unusual value is encountered, visually highlight items that are outside of the ordinary, allows the user to turn off future warnings, learn usual size of boluses over time, worn when a percentage of a total daily dose is reached or exceeded, specify treatment to type of diabetes, and so on.

Example 9 - Correction Bolus

Bolus calculator implementations according to present principles may also be employed to determine correction boluses. One exemplary difficulty associated with correction boluses is knowing when a correction bolus is needed, while wanting to correct as soon as possible

While certain inputs are described above, generally required inputs for correction bolus calculator determinations include target glucose, current glucose, and insulin sensitivity factor. Other inputs which may be employed include exercise, glucose rate of change, and so on.

A minimum time may be set between him meal bolus and a correction bolus, e.g., three hours. Real-time CGM glucose values may be automatically entered, and/or users may be allowed to enter BG values. CGM rate of change information may be automatically entered if available. Users may be enabled to enter other categorical information, e.g., exercise, alcohol, or other lifestyle factors. Any of these data may be employed as part of a learning step for learning user states within a state model.

Last dose recommendations may be automatically entered if it is within the insulin action time and may further include a confirmation screen that this was the last insulin dose. Users may adjust this option for insulin on board calculations.

One exemplary correction bolus determination is as follows:

$$B = (Go\text{-}Gt)/ISF - IOB + (ROC*\Delta T)/CF.$$

The correction bolus recommendation may allow the user to accept or reject the suggested bolus, and/or to adjust the same. The system may provide a warning if there is enough active insulin such that a correction bolus is not required. The system may further provide a warning to eat carbs if active insulin covers more than the change in glucose necessary. The user interface may provide an optional screen breakdown of how the dose was calculated.

SECOND LEVEL

Example 10 - Rescue Carbs

Challenges associated with the determination of rescue carbs includes determining the right amount of cards to increase glucose levels to a safe zone without overshooting into hyperglycemia, as well as choosing the right food type depending on the scenario, e.g., fast acting carbs versus more complex carbohydrates.

While certain inputs are described above, generally required inputs for rescue carb determinations include current glucose. Other potential inputs include glucose rate of change, insulin-on-board, and so on. These inputs may be determined as noted above.

**In** exemplary calculation of rescue carbs is as follows:

$$Carbs = IOB * ICR + (Gt\text{-}Go)*(ICR/ISF)$$

As an exemplary output, a picture example of the carb amount may be provided on the display, including as specified to typical carbs the user consumes. For example, if the system knows the user enjoys orange juice, a picture of a glass of orange juice may be displayed.

THIRD LEVEL AND OTHER EXAMPLES

Other examples are provided in the Table IV below, as delineated by either lifestyle goal, problem to be solved, or decision to be made. For each example, various exemplary inputs are provided, for content (I#) of the decision-support processing as well as for its form (e.g., afffectors, e.g., DSI#, DS#, DSO#). In certain cases potential states are also described. It is noted that where a cell in the table is empty, it is not necessarily the case that the same does not have data associated with it; the data may simply be unavailable.

Table IV

| USER-DESIRED FUNCTIONALITY, E.G., GOAL, PROBLEM TO BE SOLVED, DECISION TO BE MADE (CAN BE DEFAULT) | EXEMPLARY INPUTS, INCLUDING REAL-TIME INPUTS | EXEMPLARY DSI#, DS#, DSO# | EXEMPLARY STATES IN A STATE MODEL | EXEMPLARY OUTPUTS |
|---|---|---|---|---|
| RECOGNIZING PATTERNS AND LEARNING HOW TO TREAT THEM, SUCH AS GOING LOW AFTER EXERCISE | Glucose historical data glucose level<br>User glucose event data, e.g., histories of highs and lows | User-entered data regarding aggressiveness of therapy<br>User desire for assistance with pattern<br>User level of desired interaction | ISF profile states exercise states | carb suggestions insulin bolus recommendations<br>Output based on recognized patterns and current glucose level |
| USER PRE-ACTIVITY DECISIONS, E.G., DRIVING, SLEEPING, EXERCISING | EGV Insulin on board current basal rate Calendar / schedule Rate of change of EGV Meal (timing and amount) Exercise (timing and intensity / duration) prior pre-event occurrences pump status | User level of desired interaction | ISF profile states Exercise states | User will likely accept the significant disturbances before the event, but does not want to be interrupted during the event Carbohydrates/ins ulin recommendations Bolus history data basal history data |
| MITIGATION OF RISKS DUE TO SAFETY ISSUES | Safety risk data<br>CGM<br>Insulin<br>Meal<br>insulin sensitivity | The importance of User level of desired interaction may be lessened here, if safety is an issue | use retrospective pattern analysis to optimize meal/insulin states | CGM recommends target glucose/range influenced by safety risk dose recommendation may be just to a target range, not to target value insulin dose recommendations carbohydrate recommendations |
| GOAL ASSOCIATED WITH AN EXTERNAL EVENT | EGV<br>Glucose rate of change calendar/schedule | User level of desired interaction with respect to timing of the external event, e.g., more before, less or non-during, more after | State(s) associated with event, e.g., insulin related states, event related states ISF profiles | Insulin and carbohydrate recommendations |
| SYSTEM DEDUCES DESIRED USER LEVEL OF INTERACTIVITY | Level of user interactivity at input | Level of user interactivity at input | User interaction states | Output commensurate with user input |

(continued)

| | | | | |
|---|---|---|---|---|
| USER IS WORRIED ABOUT, E.G., DID I DOSE ENOUGH? | Pump data<br>Glucose rate of change<br>Glucose | Provide output as soon as it is known when the dosages "enough" | ISF profile states | Provide output indicating whether dose is "enough", and further indicate that an alert will be provided when the user is back "in zone" |
| USER WANTS TO WAKE UP WITH HIGH ENERGY, OR USER WANTS TO WAKE UP WITH EGV = 100 | Glucose value<br>Meal data<br>Exercise data<br>Calendar data | Provide output before user goes to bed | Sleep states<br>Meal states | Insulin and carbohydrate recommendations |
| WANT TO AVOID OR MINIMIZE BOLUSING AT SCHOO | Current EGV<br>Calendar (to determine school times) | Output adjusted to avoid interruptions during school time, unless emergency | School states<br>ISF profile states Meal states | Insulin and carbohydrate recommendations |
| USER HAS BIG EVENT OR MEETING COMING UP (BIG MEETING, ORCHESTRA CONCERT, SPORTING EVENT), AND USER DOES NOT WANT TO E.G., GO LOW | EGV<br>Calendar / schedule Rate of change of EGV | Minimize output during event, provide plenty of output before and after event, if needed, such that output can be minimize during event | Stress states<br>ISF profile states meal states states corresponding to event (if known) | User will likely accept the significant disturbances before the event, but does not want to be interrupted during the event |
| | | | | |
| PROVIDE TRIGGERED INSIGHT | Location<br>Meal<br>Exercise / activity<br>Glucose | Proactive insight pushes when an event/activity is anticipated based on detected variables | meal states exercise states ISF profile states | Displayed output corresponding to insight |
| USER MIS-ESTIMATES CARBS IN MEALS | Insulin delivered<br>Glucose level<br>Exercise<br>Health<br>Meal<br>Meal time | User is queried as to carbs in meals prior to scheduled mealtimes | ISF profile states meal states | Use retrospective analytics to estimate the amount of carbs in a meal |
| PATIENT HAS POORLY PREDICTED EGV AT A REGULAR (ACCORDING TO PATTERN) TIME | Time / calendar/schedule | Query user as to relevant parameters to better predict EGV | ISF profile states calendar related states | Provide user with reduced aggressiveness during such times |
| DESIRE TO TREAT OR AT LEAST RECOGNIZE HYPOGLYCEMIA | If system detects the top five hypoglycemia symptoms, the output can relate to solving the hypoglycemia problem: Meals Exercise stress (physical or emotional) | Prompt user when symptoms are detected | ISF profile states meal states exercise states stress states | Solution to hypoglycemia problem (eating meal, glucagon, etc.) |

(continued)

| | | | | |
|---|---|---|---|---|
| CORRECT INEFFECTIVE INSULIN DOSING | Historic Insulin dose times and sizes and outcomes thereof Diet and calorie intake Exercise type and duration | Prompt user prior to insulin dosing, if such a time as detected by patterns or other data | ISF profile states | More refined insulin dosing decisions Insight into prior failed insulin dosing decisions |
| PROVIDE WARNING TO PATIENT IF A DANGEROUS SITUATION ARISES, PARTICULARLY IF PATIENT HAS CERTAIN DATA "STACKED UP | High insulin sensitivity Preprandial is high, e.g., 350 True GV may be 250 Large meal, carb content uncertain | | ISF states meal states | Thus provide output to patient in one way or another that insulin dose may pose a risk, e.g., prompt user to enter more precise carb content, take photo of meal, provide less aggressive dose than usual, targeting 180 rather than 100 |
| MEAL BOLUS (DIFFICULT TO KNOW EXACT CARBS, POTENTIAL FOR OVER/UNDER ESTIMATING, INCORRECT ESTIMATE OF ISF AND ICR, HOW LONG INSULIN WILL TAKE, INCORRECT MENTAL MATH, ALCOHOL AFFECT, AND SO ON) | Meal data<br>Age<br>gender<br>Target glucose<br>Current glucose<br>Carb ratio<br>Insulin sensitivity factor<br>Exercise<br>Glucose rate of change<br>Insulin-on-board Rate of change, e.g., EGV going up, so must be meal time, or based on calendar, e.g., 6pm, so must be dinner time<br>Prior bolus information | Time of day<br>Stress<br>Illness<br>Exercise<br>Desire for discreteness during meal<br>Prior history of successful meal boluses, and how they were presented | Meal states<br>ISF profile states | Use meal bolus equation to determine bolus Bolus reminders may also be set up, e.g., curated by questions within a setup mode |
| IDENTIFYING CYCLE OF INSULIN RESISTANCE | Meal data<br>Glucose data<br>Glucose rate of change data | Is user in safe zone?<br>May be minimal, e.g., automatic | ISF profile states | When in safe zone, glucose sensor can send instructions to insulin pump to pump at a preprogrammed profile to learn IS/IR characteristics - This aspect can be repeated in various other examples as well, i.e., can inform lots of aspects |

(continued)

| | | | | |
|---|---|---|---|---|
| CORRECTON BOLUS (E.G., KNOWING WHEN, OR IF, A CORRECTION BOLUS IS NEEDED; A DESIRE TO CORRECT AS SOON AS POSSIBLE; NOT OVERCORRECTING ACCOUNTING FOR DIFFERENT INSULIN BRANDS HAVING DIFFERENT SPEED, AND SO ON. | Target glucose Current glucose Insulin sensitivity factor Exercise (we noticed from fitbit that you are exercising, so we're going to adjust your bolus based on that) Glucose rate of change, e.g., we detected you had dinner, so it may be time to consider if a correction bolus is needed Insulin-on-board / active insulin | Prompt user that correction bolus might be needed | ISF profile states Meal states Exercise states | Use correction bolus (math equation) to determine bolus |
| OVERCORRECTION OF HYPERGLYCEMIA | Pump and CGM information | | ISF profile states Carb states ICR states | |
| UNDER CORRECTION OF HYPERGLYCEMIA | CGM information | | ISF profile states Carb states ICR states | |
| OVERCORRECTION OF HYPOGLYCEMIA | Pump and CGM information | | ISF profile states Carb states ICR states | |
| UNDER CORRECTION OF HYPOGLYCEMIA | CGM information | | ISF profile states Carb states ICR states | Predictive alerts with advanced warning, e.g., before low occurs and judgment is impaired |
| OVER BOLUS FOR A MEAL | Pump and CGM information | | ISF profile states Carb states ICR states | |
| PRE-MEAL HYPERGLYCEMIA | Meal data CGM information | | ISF profile states Carb states ICR states | |
| PRE-MEAL HYPOGLYCEMIA | CGM information | | ISF profile states Carb states ICR states | |
| POSTMEAL HYPOGLYCEMIA | CGM information Insulin information carb information | | ISF profile states Carb states ICR states | |
| POSTMEAL HYPERGLYCEMIA | CGM information Insulin information Carb information | | ISF profile states Carb states ICR states | |

(continued)

| | | | | |
|---|---|---|---|---|
| NOCTURNAL HYPO-GLYCEMIA | CGM information | | ISF profile states<br>Carb states<br>ICR states | |
| DAWN PHENOMEN-ON | CGM information | | ISF profile states<br>Carb states<br>ICR states | |
| HYPERGLYCEMIA SHORTLY AFTER GOING TO BED | CGM information | | ISF profile states<br>Carb states<br>ICR states | |
| PRE-WAKING HYPOGLYCEMIA | CGM information | | ISF profile states<br>Carb states<br>ICR states | |
| HYPERGLYCEMIA UPON WAKING | CGM information | | ISF profile states<br>Carb states<br>ICR states | |
| HYPOGLYCEMIA UPON WAKING | CGM information | | ISF profile states<br>Carb states<br>ICR states | |
| EXCESSIVE GLYCE-MIC VARIABILITY | CGM information | | ISF profile states<br>Carb states<br>ICR states | |
| HYPOGLYCEMIA AFTER EXERCISE | CGM information or BG in-formation, to show that glu-cose was low, indication that user has exercised Activity Monitor, accelerometer or heart rate, sweat, adrenaline Indicator of duration of exercise GPS data social media updates calendar data | | ISF profile states<br>Carb states<br>ICR states | |
| HYPERGLYCEMIA AFTER EXERCISE | CGM information | | ISF profile states<br>Carb states<br>ICR states | |
| HYPERGLYCEMIA DURING EXERCISE | CGM information | | ISF profile states<br>Carb states<br>ICR states | |
| HYPOGLYCEMIA DURING EXERCISE | CGM information | | ISF profile states<br>Carb states<br>ICR states | |

(continued)

| | | | | |
|---|---|---|---|---|
| PUMP PROBLEMS AFFECTING INSU-LIN DELIVERY | CGM information | | ISF profile states<br>Carb states<br>ICR states | Safety oriented pump alarms, e.g., low reservoir alerts, tubing kinked or oc-cluded, battery low |

Besides the above, other types of decisions for which states may be determined and decision support provided include: correction bolus after a meal when not needed, not taking into account down arrow when taking a correction bolus, wrong insulin sensitivity factor, bad math calculation, overreaction (rage bolus), not taking into account exercise, not taking into account insulin-on-board with taking a correction bolus, stacked insulin, setting too low a target glucose (which could be time of day dependent, concern about insulin delivery (was it because pump wasn't working that you went high and so over corrected last time), fear of overcorrection, correction factor needs adjustment, bad insulin, insulin delivery issue, e.g., pump occlusion, pen misfire, stress, illness, caffeine consumption, eating too many carbs, lowered basal too much or suspended basal when should have, eating the wrong type of food, e.g., too fast of a sugar, being really hungry when low and eating too much out of hunger, being very anxious when low and eating too much out of anxiousness, impaired judgment, caused by low, can delay reaction to low, continued low or moderate activity, not taking into account meal composition, eyes versus appetite, incorrect assumption about insulin sensitivity, alcohol, unplanned meals, bolused too early, postmeal walk, and life transitions.

Implementations Of Systems And Methods According To Present Principles Enabling Decision-Support Application/ Functionality.

Various systems may be employed to implement decision-support application/functionality according to present principles. For example, in one implementation, a mobile computer device may be employed that is dedicated to health and diabetes management. Though dedicated to health/diabetes management, the device may be configurable by the user to suit the user's lifestyle and preferences. The mobile device may be based on an Android (or other) OS and utilize a touchscreen interface. In some cases the operating system may be customized or controlled for administrative services, e.g., to provide data to the cloud, and to optimize/control elements of the user experience. The device may include one or more common radio communication links, such as Bluetooth Low Energy. It may also include WiFi, or other data connectivity technology, for remote monitoring, data transfer, and software updates.

The mobile computer may be used in conjunction with ("tethered" to) a wearable computing device such as a Watch. The Watch may also function usefully without direct connection (untethered) to the mobile device.

The user may select from a curated ecosystem of Apps. This selection may include Apps from, for example, Databetes (meal memory), TrainingPeaks (activity/fitness), Tidepool, MyFitnessPal, Nike+, Withings, etc. Other apps may be included for retrospective insight/pattern recognition aimed at therapy optimization. The ecosystem may further include an app that provides basic diabetes management instructions for the newly diagnosed patient (or parent of patient) with T1D. A distinct set of instructions may be included for the newly diagnosed patient with T2D.

The mobile computer allows connectivity of data between user (patient) and clinician, via EMR (e.g. Epic). The mobile computer platform enables/facilitates patient/provider dialogue.

Variations will be understood. In one example, the mobile computer may simply serve as a CGM receiver. In another implementation, the mobile computer may provide real-time decision support to the user based on inputs from CGM and medicament (e.g. insulin) delivery systems, activity sensors, etc. The delivery system may be a pen or pump. In another exemplary use case, the mobile computer may provide real-time medicament (insulin) delivery control to an infusion pump, based on similar data streams as used by real-time decision support.

In another particular implementation, the mobile computer may be configured such that it will not include functionality unrelated to health management.

In many implementations, particularly where the implementation is that of a bolus calculator, an initial phase will occur where the system sets up the bolus calculator, tuning the same according to the needs of the particular patient. Systems and methods according to present principles, implementing decision-support application/functionality, e.g., using data about states associated with the user, may advantageously aid in such set up, e.g., may essentially set up most or all parameters, which the HCP or patient may provide confirmation for.

Other safety settings may also be implemented. For example, the decision-support application/functionality may be disabled until such time as CGM data has been collected. In this way, the settings may be data-driven and data validated. For example, one week of data may be required, two weeks of data may be required, one month of data may be required, and so on. An initial set up or training may be performed without data, or using data from a prior user device (associated with the subject user). This may be followed up with a subsequent optimization, and in particular the decision-support application/functionality may determine that enough data has been collected to allow optimization, and may alert the user or the HCP to perform the same. Alternatively, the decision-support application/functionality may implement optimization, but require confirmation from the user or HCP. In a particular implementation, the algorithm may determine that the bolus calculator parameters can be optimized according to the methods described above. Subsequently, the user will generally see better results with the optimized calculator recommendations. The system may further set a range of acceptable insulin to carb ratios and correction factors, and within the range, the decision-support application/functionality may be enabled to automatically update the same according to determination of user state.

The particular settings will include those employed in bolus calculators, as well as the behavioral or lifestyle settings (input parameters and variables) described here. In one implementation, these settings may include how trend information is to be used to moderate insulin dose e.g., how much to reduce insulin if the trend arrow is 45° down.

What has been described are systems and methods for achieving decision-support, especially where based on lifestyle factors and patient states. Numerous variations of the above will be understood given this description.

For example, if offering separate buttons/app pathways to computing a correction bolus vs. a premeal bolus, the system can disable the correction bolus option when the CGM glucose level is below a specific value or when specific combinations of glucose level and trend are measured (e.g. must be >120 mg/dL with flat arrow or up arrows, >140 mg/dL with diagonal down arrow, > 160 with vertical down arrow, etc.

As another variation, if a user has calculated a bolus, and the CGM profile in following hours does not fit what would be expected from having administered that bolus (e.g. based on a physiological model or pattern recognition), the user may be asked whether or not the bolus was truly administered. Similarly, pattern recognition/modeling could be used to detect boluses that are administered without having been calculated with the app. Knowing historical boluses is important for calculating insulin on board for future bolus calculations.

In another variation, aggressiveness of glucose control can be modulated, depending on user-specific settings that impact mitigations of insulin over and under doses. For example, if a user has a low alert threshold of 90 mg/dL, the bolus calculator may target a more aggressive (lower) glucose level because there the glucose alert provides an effective mitigation of potential hypoglycemia, whereas a less aggressive glucose level should be used if the user has a low alert threshold of 55 mg/dL. Another indication of the effectiveness of alerts as mitigations would be historical data capture by the receiver, which indicates whether the user is good about keeping their phone/receiver near them and therefore are more likely to respond to an alert.

In another variation, in the app, there may be at least one confirmation screen at the time of bolus calculation to verify that the information entered by the user is correct. The confirmation screen may be modulated (whether the confirmation screen is provided vs. skipped) depending on whether the entered values are typical for that user, or to visually highlight user inputs that seem atypical in the confirmation screen. Typical vs. atypical values could be based on aggregate data across many users, and could be refined based on individual users over time. This is a similar idea to credit card fraud protection, where atypical behavior is immediately flagged and triggers verification with the credit card holder that a purchase was not fraudulent. Similarly as with credit cards, there may be a mode that a user provides (such as a "holiday mode") that causes the app to use looser criteria for flagging atypical behavior for a set period of time, analogous to notifying a credit card company that you will be vacationing in a foreign country ahead of time.

In another variation, for entering meal carb content, users could enter their own photos of previous meals and these meal photos could be provided with a set of buttons or a sliding scale as an estimate of carb content. For example, if a user took a photo and associated it with a large carb content in previous app interactions, and also took a photo of a meal with low carb content, these two photos could be provided on the ends of a sliding scale as a user input of carb estimates for future meals. Internally, the app would associate these historical meals with a carb content and the sliding scale would set the new carb content to a weighted average of the two extremes, based on the slider position.

Users could have "standard" meals with preset carb estimates as an option in the carb entry screen. For example, in the morning, the user could opt to either press a button labeled "typical breakfast" if they are having the same breakfast as usual, or could enter an alternative carb estimate if that day’s breakfast is atypical. The system can also learn the user’s standard meal sizes over time, as well as if the user is known to over or under estimate meal compositions including carb amounts. Users may be prompted to confirm if significant variations are seen, either in entered data or in glucose responses.

## Sensor System

FIG. 30 depicts an example system 100, in accordance with some example implementations. The system 100 includes a continuous analyte sensor system 8 including sensor electronics 12 and a continuous analyte sensor 10. The system 100 may include other devices and/or sensors, such as medicament delivery pump 2 and glucose meter 4. The continuous analyte sensor 10 may be physically connected to sensor electronics 12 and may be integral with (e.g., non-releasably attached to) or releasably attachable to the continuous analyte sensor 10. The sensor electronics 12, medicament delivery pump 2, and/or glucose meter 4 may couple with one or more devices, such as display devices 14, 16, 18, and/or 20.

In some example implementations, the system 100 may include a cloud-based analyte processor 490 configured to analyze analyte data (and/or other patient-related data) provided via network 406 (e.g., via wired, wireless, or a combination thereof) from sensor system 8 and other devices, such as display devices 14-20 and the like, associated with the host (also referred to as a patient) and generate reports providing high-level information, such as statistics, regarding the measured analyte over a certain time frame. A full discussion of using a cloud-based analyte processing system may be found in U.S. Patent Publication No. US-2013-0325352-A1, entitled "Cloud-Based Processing of Analyte Data" and filed on March 7, 2013. In some implementations, one or more steps of the factory calibration algorithm can be performed in the cloud.

In some example implementations, the sensor electronics 12 may include electronic circuitry associated with measuring and processing data generated by the continuous analyte sensor 10. This generated continuous analyte sensor data may also include algorithms, which can be used to process and calibrate the continuous analyte sensor data, although these algorithms may be provided in other ways as well. The sensor electronics 12 may include hardware, firmware, software, or a combination thereof, to provide measurement of levels of the analyte via a continuous analyte sensor, such as a continuous glucose sensor. An example implementation of the sensor electronics 12 is described further below with respect to FIG. 31.

In one implementation, the factory calibration algorithms described herein may be performed by the sensor electronics.

The sensor electronics 12 may, as noted, couple (e.g., wirelessly and the like) with one or more devices, such as display devices 14, 16, 18, and/or 20. The display devices 14, 16, 18, and/or 20 may be configured for presenting information (and/or alarming), such as sensor information transmitted by the sensor electronics 12 for display at the display devices 14, 16, 18, and/or 20.

The display devices may include a relatively small, key fob-like display device 14, a relatively large, hand-held display device 16, a cellular phone 18 (e.g., a smart phone, a tablet, and the like), a computer 20, and/or any other user equipment configured to at least present information (e.g., medicament delivery information, discrete self-monitoring glucose readings, heart rate monitor, caloric intake monitor, and the like).

In one implementation, the factory calibration algorithms described herein may be performed at least in part by the display devices.

In some example implementations, the relatively small, key fob-like display device 14 may comprise a wrist watch, a belt, a necklace, a pendent, a piece of jewelry, an adhesive patch, a pager, a key fob, a plastic card (e.g., credit card), an identification (ID) card, and/or the like. This small display device 14 may include a relatively small display (e.g., smaller than the large display device 16) and may be configured to display certain types of displayable sensor information, such as a numerical value, and an arrow, or a color code.

In some example implementations, the relatively large, hand-held display device 16 may comprise a hand-held receiver device, a palm-top computer, and/or the like. This large display device may include a relatively larger display (e.g., larger than the small display device 14) and may be configured to display information, such as a graphical representation of the continuous sensor data including current and historic sensor data output by sensor system 8.

In some example implementations, the continuous analyte sensor 10 comprises a sensor for detecting and/or measuring analytes, and the continuous analyte sensor 10 may be configured to continuously detect and/or measure analytes as a non-invasive device, a subcutaneous device, a transdermal device, and/or an intravascular device. In some example implementations, the continuous analyte sensor 10 may analyze a plurality of intermittent blood samples, although other analytes may be used as well.

In some example implementations, the continuous analyte sensor 10 may comprise a glucose sensor configured to measure glucose in the blood or interstitial fluid using one or more measurement techniques, such as enzymatic, chemical, physical, electrochemical, spectrophotometric, polarimetric, calorimetric, iontophoretic, radiometric, immunochemical, and the like. In implementations in which the continuous analyte sensor 10 includes a glucose sensor, the glucose sensor may comprise any device capable of measuring the concentration of glucose and may use a variety of techniques to measure glucose including invasive, minimally invasive, and non-invasive sensing techniques (e.g., fluorescence monitoring), to provide data, such as a data stream, indicative of the concentration of glucose in a host. The data stream may be sensor data (raw and/or filtered), which may be converted into a calibrated data stream used to provide a value of glucose to a host, such as a user, a patient, or a caretaker (e.g., a parent, a relative, a guardian, a teacher, a doctor, a nurse, or any other individual that has an interest in the wellbeing of the host). Moreover, the continuous analyte sensor 10 may be implanted as at least one of the following types of sensors: an implantable glucose sensor, a transcutaneous glucose sensor, implanted in a host vessel or extracorporeally, a subcutaneous sensor, a refillable subcutaneous sensor, an intravascular sensor.

Although the disclosure herein refers to some implementations that include a continuous analyte sensor 10 comprising a glucose sensor, the continuous analyte sensor 10 may comprise other types of analyte sensors as well. Moreover, although some implementations refer to the glucose sensor as an implantable glucose sensor, other types of devices capable of detecting a concentration of glucose and providing an output signal representative of glucose concentration may be used as well. Furthermore, although the description herein refers to glucose as the analyte being measured, processed, and the like, other analytes may be used as well including, for example, ketone bodies (e.g., acetone, acetoacetic acid and beta hydroxybutyric acid, lactate, etc.), glucagon, acetyl-CoA, triglycerides, fatty acids, intermediaries in the citric acid cycle, choline, insulin, cortisol, testosterone, and the like.

FIG. 31 depicts an example of sensor electronics 12, in accordance with some example implementations. The sensor electronics 12 may include sensor electronics that are configured to process sensor information, such as sensor data, and generate transformed sensor data and displayable sensor information, e.g., via a processor module. For example, the processor module may transform sensor data into one or more of the following: filtered sensor data (e.g., one or more filtered analyte concentration values), raw sensor data, calibrated sensor data (e.g., one or more calibrated analyte concentration values), rate of change information, trend information, rate of acceleration/deceleration information, sensor diagnostic information, location information, alarm/alert information, calibration information such as may be determined by calibration algorithms, smoothing and/or filtering algorithms of sensor data, and/or the like.

In some embodiments, a processor module 214 is configured to achieve a substantial portion, if not all, of the data processing, including data processing pertaining to factory calibration. Processor module 214 may be integral to sensor electronics 12 and/or may be located remotely, such as in one or more of devices 14, 16, 18, and/or 20 and/or cloud 490. In some embodiments, processor module 214 may comprise a plurality of smaller subcomponents or submodules. For example, processor module 214 may include an alert module (not shown) or prediction module (not shown), or any other suitable module that may be utilized to efficiently process data. When processor module 214 is made up of a plurality of submodules, the submodules may be located within processor module 214, including within the sensor electronics 12 or other associated devices (e.g., 14, 16, 18, 20 and/or 490). For example, in some embodiments, processor module 214 may be located at least partially within a cloud-based analyte processor 490 or elsewhere in network 406.

In some example implementations, the processor module 214 may be configured to calibrate the sensor data, and the data storage memory 220 may store the calibrated sensor data points as transformed sensor data. Moreover, the processor module 214 may be configured, in some example implementations, to wirelessly receive calibration information from a display device, such as devices 14, 16, 18, and/or 20, to enable calibration of the sensor data from sensor 12. Furthermore, the processor module 214 may be configured to perform additional algorithmic processing on the sensor data (e.g., calibrated and/or filtered data and/or other sensor information), and the data storage memory 220 may be configured to store the transformed sensor data and/or sensor diagnostic information associated with the algorithms. The processor module 214 may further be configured to store and use calibration information determined from a calibration.

In some example implementations, the sensor electronics 12 may comprise an application-specific integrated circuit (ASIC) 205 coupled to a user interface 222. The ASIC 205 may further include a potentiostat 210, a telemetry module 232 for transmitting data from the sensor electronics 12 to one or more devices, such as devices 14, 16, 18, and/or 20, and/or other components for signal processing and data storage (e.g., processor module 214 and data storage memory 220). Although FIG. 31 depicts ASIC 205, other types of circuitry may be used as well, including field programmable gate arrays (FPGA), one or more microprocessors configured to provide some (if not all of) the processing performed by the sensor electronics 12, analog circuitry, digital circuitry, or a combination thereof.

In the example depicted in FIG. 31, through a first input port 211 for sensor data the potentiostat 210 is coupled to a continuous analyte sensor 10, such as a glucose sensor to generate sensor data from the analyte. The potentiostat 210 may also provide via data line 212 a voltage to the continuous analyte sensor 10 to bias the sensor for measurement of a value (e.g., a current and the like) indicative of the analyte concentration in a host (also referred to as the analog portion of the sensor). The potentiostat 210 may have one or more channels depending on the number of working electrodes at the continuous analyte sensor 10.

**In** some example implementations, the potentiostat 210 may include a resistor that translates a current value from the sensor 10 into a voltage value, while in some example implementations, a current-to-frequency converter (not shown) may also be configured to integrate continuously a measured current value from the sensor 10 using, for example, a charge-counting device. In some example implementations, an analog-to-digital converter (not shown) may digitize the analog signal from the sensor 10 into so-called "counts" to allow processing by the processor module 214. The resulting counts may be directly related to the current measured by the potentiostat 210, which may be directly related to an analyte level, such as a glucose level, in the host.

The telemetry module 232 may be operably connected to processor module 214 and may provide the hardware, firmware, and/or software that enable wireless communication between the sensor electronics 12 and one or more other devices, such as display devices, processors, network access devices, and the like. A variety of wireless radio technologies that can be implemented in the telemetry module 232 include Bluetooth, Bluetooth Low-Energy, ANT, ANT+, ZigBee, IEEE 802.11, IEEE 802.16, cellular radio access technologies, radio frequency (RF), infrared (IR), paging network communication, magnetic induction, satellite data communication, spread spectrum communication, frequency hopping communication, near field communications, and/or the like. In some example implementations, the telemetry module 232 comprises a Bluetooth chip, although Bluetooth technology may also be implemented in a combination of the telemetry module 232 and the processor module 214.

The processor module 214 may control the processing performed by the sensor electronics 12. For example, the processor module 214 may be configured to process data (e.g., counts), from the sensor, filter the data, calibrate the data, perform fail-safe checking, and/or the like.

In some example implementations, the processor module 214 may comprise a digital filter, such as for example an infinite impulse response (IIR) or a finite impulse response (FIR) filter. This digital filter may smooth a raw data stream received from sensor 10. Generally, digital filters are programmed to filter data sampled at a predetermined time interval (also referred to as a sample rate). In some example implementations, such as when the potentiostat 210 is configured to measure the analyte (e.g., glucose and/or the like) at discrete time intervals, these time intervals determine the sampling rate of the digital filter. In some example implementations, the potentiostat 210 may be configured to measure continuously the analyte, for example, using a current-to-frequency converter. In these current-to-frequency converter implementations, the processor module 214 may be programmed to request, at predetermined time intervals (acquisition time), digital values from the integrator of the current-to-frequency converter. These digital values obtained by the processor module 214 from the integrator may be averaged over the acquisition time due to the continuity of the current measurement. As such, the acquisition time may be determined by the sampling rate of the digital filter.

The processor module 214 may further include a data generator (not shown) configured to generate data packages for transmission to devices, such as the display devices 14, 16, 18, and/or 20. Furthermore, the processor module 214 may generate data packets for transmission to these outside sources via telemetry module 232. In some example implementations, the data packages may, as noted, be customizable for each display device, and/or may include any available data, such as a time stamp, displayable sensor information, transformed sensor data, an identifier code for the sensor and/or sensor electronics 12, raw data, filtered data, calibrated data, rate of change information, trend information, error detection or correction, and/or the like.

The processor module 214 may also include a program memory 216 and other memory 218. The processor module 214 may be coupled to a communications interface, such as a communication port 238, and a source of power, such as a battery 234. Moreover, the battery 234 may be further coupled to a battery charger and/or regulator 236 to provide power to sensor electronics 12 and/or charge the battery 234.

The program memory 216 may be implemented as a semi-static memory for storing data, such as an identifier for a coupled sensor 10 (e.g., a sensor identifier (ID)) and for storing code (also referred to as program code) to configure the ASIC 205 to perform one or more of the operations/functions described herein. For example, the program code may configure processor module 214 to process data streams or counts, filter, perform the calibration methods, perform fail-safe checking, and the like.

The memory 218 may also be used to store information. For example, the processor module 214 including memory 218 may be used as the system's cache memory, where temporary storage is provided for recent sensor data received from the sensor. In some example implementations, the memory may comprise memory storage components, such as read-only memory (ROM), random-access memory (RAM), dynamic-RAM, static-RAM, non-static RAM, easily erasable programmable read only memory (EEPROM), rewritable ROMs, flash memory, and the like.

The data storage memory 220 may be coupled to the processor module 214 and may be configured to store a variety of sensor information. In some example implementations, the data storage memory 220 stores one or more days of continuous analyte sensor data. For example, the data storage memory may store 1, 2, 3, 4, 5, 6, 7, 8, 9, 10, 11, 12, 13, 14, 15, 20, and/or 30 (or more days) of continuous analyte sensor data received from sensor 10. The stored sensor information may include one or more of the following: a time stamp, raw sensor data (one or more raw analyte concentration values), calibrated data, filtered data, transformed sensor data, and/or any other displayable sensor information, calibration information (e.g., reference BG values and/or prior calibration information such as from factory calibration), sensor diagnostic information, and the like.

The user interface 222 may include a variety of interfaces, such as one or more buttons 224, a liquid crystal display (LCD) 226, a vibrator 228, an audio transducer (e.g., speaker) 230, a backlight (not shown), and/or the like. The components that comprise the user interface 222 may provide controls to interact with the user (e.g., the host). One or more buttons 224 may allow, for example, toggle, menu selection, option selection, status selection, yes/no response to on-screen questions, a "turn off" function (e.g., for an alarm), an "acknowledged" function (e.g., for an alarm), a reset, and/or the like. The LCD 226 may provide the user with, for example, visual data output. The audio transducer 230 (e.g., speaker) may provide audible signals in response to triggering of certain alerts, such as present and/or predicted hyperglycemic and hypoglycemic conditions. In some example implementations, audible signals may be differentiated by tone, volume, duty cycle, pattern, duration, and/or the like. In some example implementations, the audible signal may be configured to be silenced (e.g., acknowledged or turned off) by pressing one or more buttons 224 on the sensor electronics 12 and/or by signaling the sensor electronics 12 using a button or selection on a display device (e.g., key fob, cell phone, and/or the like).

Although audio and vibratory alarms are described with respect to FIG. 31, other alarming mechanisms may be used as well. For example, in some example implementations, a tactile alarm is provided including a poking mechanism configured to "poke" or physically contact the patient in response to one or more alarm conditions.

The battery 234 may be operatively connected to the processor module 214 (and possibly other components of the sensor electronics 12) and provide the necessary power for the sensor electronics 12. In some example implementations, the battery is a Lithium Manganese Dioxide battery, however any appropriately sized and powered battery can be used (e.g., AAA, Nickel-cadmium, Zinc-carbon, Alkaline, Lithium, Nickel-metal hydride, Lithium-ion, Zinc-air, Zinc-mercury oxide, Silver-zinc, or hermetically-sealed). In some example implementations, the battery is rechargeable. In some example implementations, a plurality of batteries can be used to power the system. In yet other implementations, the receiver can be transcutaneously powered via an inductive coupling, for example.

A battery charger and/or regulator 236 may be configured to receive energy from an internal and/or external charger. In some example implementations, a battery regulator (or balancer) 236 regulates the recharging process by bleeding off excess charge current to allow all cells or batteries in the sensor electronics 12 to be fully charged without overcharging other cells or batteries. In some example implementations, the battery 234 (or batteries) is configured to be charged via an inductive and/or wireless charging pad, although any other charging and/or power mechanism may be used as well.

One or more communication ports 238, also referred to as external connector(s), may be provided to allow communication with other devices, for example a PC communication (com) port can be provided to enable communication with systems that are separate from, or integral with, the sensor electronics 12. The communication port, for example, may comprise a serial (e.g., universal serial bus or "USB") communication port, and allow for communicating with another computer system (e.g., PC, personal digital assistant or "PDA," server, or the like). In some example implementations, the sensor electronics 12 is able to transmit historical data to a PC or other computing device for retrospective analysis by a patient and/or HCP. As another example of data transmission, factory information may also be sent to the algorithm from the sensor or from a cloud data source.

The one or more communication ports 238 may further include a second input port 237 in which calibration data may be received, and an output port 239 which may be employed to transmit calibrated data, or data to be calibrated, to a receiver or mobile device. Fig. 31 illustrates these aspects schematically. It will be understood that the ports may be separated physically, but in alternative implementations a single communication port may provide the functions of both the second input port and the output port.

In some continuous analyte sensor systems, an on-skin portion of the sensor electronics may be simplified to minimize complexity and/or size of on-skin electronics, for example, providing only raw, calibrated, and/or filtered data to a display device configured to run calibration and other algorithms required for displaying the sensor data. However, the sensor electronics 12 (e.g., via processor module 214) may 31 be implemented to execute prospective algorithms used to generate transformed sensor data and/or displayable sensor information, including, for example, algorithms that: evaluate a clinical acceptability of reference and/or sensor data, evaluate calibration data for best calibration based on inclusion criteria, evaluate a quality of the calibration, compare estimated analyte values with time corresponding measured analyte values, analyze a variation of estimated analyte values, evaluate a stability of the sensor and/or sensor data, detect signal artifacts (noise), replace signal artifacts, determine a rate of change and/or trend of the sensor data, perform dynamic and intelligent analyte value estimation, perform diagnostics on the sensor and/or sensor data, set modes of operation, evaluate the data for aberrancies, and/or the like.

Although separate data storage and program memories are shown in FIG. Z2, a variety of configurations may be used as well. For example, one or more memories may be used to provide storage space to support data processing and storage requirements at sensor electronics 12.

In one preferred embodiment, the analyte sensor is an implantable glucose sensor, such as described with reference to U.S. Patent 6,001,067 and U.S. Patent Publication No. US-2005-0027463-A1. In another preferred embodiment, the analyte sensor is a transcutaneous glucose sensor, such as described with reference to U.S. Patent Publication No. US-2006-0020187-A1. In still other embodiments, the sensor is configured to be implanted in a host vessel or extracorporeally, such as is described in U.S. Patent Publication No. US-2007-0027385-A1, U.S. Patent Publication No. US-2008-0119703-A1 (now abandoned), U.S. Patent Publication No. US-2008-0108942 A1 (now abandoned) and U.S. Patent No. US 7,828,728. In one alternative embodiment, the continuous glucose sensor comprises a transcutaneous sensor such as described in U.S. Patent 6,565,509 to Say et al., for example. In another alternative embodiment, the continuous glucose sensor comprises a subcutaneous sensor such as described with reference to U.S. Patent 6,579,690 to Bonnecaze et al. or U.S. Patent 6,484,046 to Say et al., for example. In another alternative embodiment, the continuous glucose sensor comprises a refillable subcutaneous sensor such as described with reference to U.S. Patent 6,512,939 to Colvin et al., for example. In another alternative embodiment, the continuous glucose sensor comprises an intravascular sensor such as described with reference to U.S. Patent 6,477,395 to Schulman et al., for example. In another alternative embodiment, the continuous glucose sensor comprises an intravascular sensor such as described with reference to U.S. Patent 6,424,847 to Mastrototaro et al.

The connections between the elements shown in the figures illustrate exemplary communication paths. Additional communication paths, either direct or via an intermediary, may be included to further facilitate the exchange of information between the elements. The communication paths may be bi-directional communication paths allowing the elements to exchange information.

The various operations of methods described above may be performed by any suitable means capable of performing the operations, such as various hardware and/or software component(s), circuits, and/or module(s). Generally, any operations illustrated in the figures may be performed by corresponding functional means capable of performing the operations.

The various illustrative logical blocks, modules and circuits described in connection with the present disclosure (such as the blocks of Fig. 31) may be implemented or performed with a general purpose processor, a digital signal processor (DSP), an application specific integrated circuit (ASIC), a field programmable gate array signal (FPGA) or other programmable logic device (PLD), discrete gate or transistor logic, discrete hardware components or any combination thereof designed to perform the functions described herein. A general purpose processor may be a microprocessor, but in the alternative, the processor may be any commercially available processor, controller, microcontroller or state machine. A processor may also be implemented as a combination of computing devices, e.g., a combination of a DSP and a microprocessor, a plurality of microprocessors, one or more microprocessors in conjunction with a DSP core, or any other such configuration.

In one or more aspects, the functions described may be implemented in hardware, software, firmware, or any combination thereof. If implemented in software, the functions may be stored on or transmitted over as one or more instructions or code on a computer-readable medium. Computer-readable media includes both computer storage media and communication media including any medium that facilitates transfer of a computer program from one place to another. A storage media may be any available media that can be accessed by a computer. By way of example, and not limitation, such computer-readable media can comprise various types of RAM, ROM, CD-ROM or other optical disk storage, magnetic disk storage or other magnetic storage devices, or any other medium that can be used to carry or store desired program code in the form of instructions or data structures and that can be accessed by a computer. Also, any connection is properly termed a computer-readable medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, WiFi, Bluetooth®, RFID, NFC, and microwave are included in the definition of medium. Disk and disc, as used herein, includes compact disc (CD), laser disc, optical disc, digital versatile disc (DVD), floppy disk and Blu-ray® disc where disks usually reproduce data magnetically, while discs reproduce data optically with lasers. Thus, in some aspects a computer-readable medium may comprise non-transitory computer-readable medium (e.g., tangible media). In addition, in some aspects a computer-readable medium may comprise transitory computer-readable medium (e.g., a signal). Combinations of the above should also be included within the scope of computer-readable media.

Certain aspects may comprise a computer program product for performing the operations presented herein. For example, such a computer program product may comprise a computer-readable medium having instructions stored (and/or encoded) thereon, the instructions being executable by one or more processors to perform the operations described herein. For certain aspects, the computer program product may include packaging material.

Software or instructions may also be transmitted over a transmission medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of transmission medium.

Further, it should be appreciated that modules and/or other appropriate means for performing the methods and techniques described herein can be downloaded and/or otherwise obtained by a user terminal and/or base station as applicable. For example, such a device can be coupled to a server to facilitate the transfer of means for performing the methods described herein. Alternatively, various methods described herein can be provided via storage means (e.g., RAM, ROM, a physical storage medium such as a compact disc (CD) or floppy disk, etc.), such that a user terminal and/or base station can obtain the various methods upon coupling or providing the storage means to the device. Moreover, any other suitable technique for providing the methods and techniques described herein to a device can be utilized.

Unless otherwise defined, all terms (including technical and scientific terms) are to be given their ordinary and customary meaning to a person of ordinary skill in the art, and are not to be limited to a special or customized meaning unless expressly so defined herein. It should be noted that the use of particular terminology when describing certain features or aspects of the disclosure should not be taken to imply that the terminology is being re-defined herein to be restricted to include any specific characteristics of the features or aspects of the disclosure with which that terminology is associated. Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide exemplary instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular embodiment of the invention. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

Where a range of values is provided, it is understood that the upper and lower limit and each intervening value between the upper and lower limit of the range is encompassed within the embodiments.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

Headings are included herein for reference and to aid in locating various sections. These headings are not intended to limit the scope of the concepts described with respect thereto. Such concepts may have applicability throughout the entire specification.

Furthermore, although the foregoing has been described in some detail by way of illustrations and examples for purposes of clarity and understanding, it is apparent to those skilled in the art that certain changes and modifications may be practiced. Therefore, the description and examples should not be construed as limiting the scope of the invention to the specific embodiments and examples described herein, the scope of the invention being defined by the appended claims.

The system and method may be fully implemented in any number of computing devices. Typically, instructions are laid out on computer readable media, generally non-transitory, and these instructions are sufficient to allow a processor in the computing device to implement the method of the invention. The computer-readable medium may be a hard drive or solid state storage having instructions that, when run, are loaded into random access memory. Inputs to the application, e.g., from the plurality of users or from any one user, may be by any number of appropriate computer input devices. For example, users may employ a keyboard, mouse, touchscreen, joystick, trackpad, other pointing device, or any other such computer input device to input data relevant to the calculations. Data may also be input by way of an inserted memory chip, hard drive, flash drives, flash memory, optical media, magnetic media, or any other type of file - storing medium. The outputs may be delivered to a user by way of a video graphics card or integrated graphics chipset coupled to a display that maybe seen by a user. Alternatively, a printer may be employed to output hard copies of the results. Given this teaching, any number of other tangible outputs will also be understood to be contemplated by the invention. For example, outputs may be stored on a memory chip, hard drive, flash drives, flash memory, optical media, magnetic media, or any other type of output. It should also be noted that the invention may be implemented on any number of different types of computing devices, e.g., personal computers, laptop computers, notebook computers, net book computers, handheld computers, personal digital assistants, mobile phones, smart phones, tablet computers, and also on devices specifically designed for these purpose. In one implementation, a user of a smart phone or wi-fi - connected device downloads a copy of the application to their device from a server using a wireless Internet connection. An appropriate authentication procedure and secure transaction process may provide for payment to be made to the seller. The application may download over the mobile connection, or over the WiFi or other wireless network connection. The application may then be run by the user. Such a networked system may provide a suitable computing environment for an implementation in which a plurality of users provide separate inputs to the system and method. In the below system where decision-support schemes are contemplated, the plural inputs may allow plural users to input relevant data at the same time.

## Claims

1. A non-transitory computer-readable medium, containing instructions for causing a computing environment to perform a method of operating and tuning or adapting a decision-support application that forms a part of a continuous glucose monitoring, CGM, application running on a smart device, the tuning or adapting performed in a way to support user-desired functionality to maintain the user's blood glucose level within a target range, the method comprising steps of:

   receiving an indication of user-desired functionality to maintain the user's blood glucose level within the target range;
   receiving insulin delivery data and an insulin sensitivity for the user;
   tuning or adapting the decision-support application to support the user-desired functionality, such that the tuning or adapting results in an insulin dose recommendation to maintain the user's blood glucose level within the target range on a user interface at least partially supported by the decision-support application to be based on the user-desired functionality and on input real-time data;
   wherein the real-time data comprises continuous glucose monitor, CGM, data from a sensor forming part of a continuous glucose monitor, and further comprises fuzzy input meal data entered by the user, the fuzzy input meal data being a user categorization of a content of a meal; and
   wherein the tuning or adapting comprises determining based on the fuzzy input meal data, a corresponding quantitative carbohydrate value, wherein the correspondence between the fuzzy input meal data and the corresponding carbohydrate value is learned using machine-learning from the user's glucose response for previous fuzzy input meal data, and
   wherein the insulin dose recommendation to maintain the user's blood glucose level within the target range is further based on the CGM data, the insulin delivery data, the insulin sensitivity and the carbohydrate value.

2. The computer-readable medium of claim 1, wherein the received insulin sensitivity is determined:

   using a direct insulin detector to measure the amount of insulin in the user's body and calculating the insulin sensitivity using glucose information for the user and insulin concentration within the user; or
   using knowledge of intake values of carbohydrates into glucose and basal or bolus amounts of insulin administered.

## Patentansprüche

1. Nichtflüchtiges computerlesbares Medium, das Anweisungen zum Veranlassen einer Rechenumgebung enthält, um ein Verfahren zum Betreiben und Feinabstimmen oder Anpassen einer Entscheidungsunterstützungsanwendung durchzuführen, die Teil einer auf einer Smart-Vorrichtung laufenden Anwendung zur kontinuierlichen Glukoseüberwachung, CGM, bildet, wobei das Feinabstimmen oder Anpassen in einer Weise durchgeführt wird, um vom Benutzer gewünschte Funktionalität zu unterstützen, um den Blutglukosespiegel des Benutzers innerhalb eines Zielbereichs zu halten, wobei das Verfahren die Schritte umfasst zum:

   Empfangen einer Angabe einer vom Benutzer gewünschten Funktionalität, um den Blutglukosespiegel des Benutzers innerhalb des Zielbereichs zu halten;
   Empfangen von Insulinabgabedaten und einer Insulinsensitivität für den Benutzer;
   Feinabstimmen oder Anpassen der Entscheidungsunterstützungsanwendung, um die vom Benutzer gewünschte Funktionalität zu unterstützen, sodass das Feinabstimmen oder Anpassen zu einer Insulindosisempfehlung, um den Blutglukosespiegel des Benutzers innerhalb des Zielbereichs zu halten, auf einer Benutzeroberfläche führt, die mindestens teilweise von der Entscheidungsunterstützungsanwendung unterstützt wird, um auf der vom Benutzer gewünschten Funktionalität und auf eingegebenen Echtzeitdaten zu basieren;
   wobei die Echtzeitdaten kontinuierliche Glukoseüberwachungs-, CGM-Daten von einem Sensor umfassen, der Teil einer kontinuierlichen Glukoseüberwachung bildet, und weiter ungenaue eingegebene Mahlzeitendaten umfassen, die vom Benutzer eingegeben werden, wobei die ungenauen eingegebenen Mahlzeitendaten eine Benutzerkategorisierung eines Inhalts einer Mahlzeit sind; und
   wobei das Feinabstimmen oder Anpassen das Bestimmen eines entsprechenden quantitativen Kohlenhydratwerts basierend auf den ungenauen eingegebenen Mahlzeitendaten umfasst, wobei die Entsprechung zwischen den ungenauen eingegebenen Mahlzeitendaten und dem entsprechenden Kohlenhydratwert unter Verwendung von maschinellem Lernen aus der Glukosereaktion des Benutzers bei früheren ungenauen eingegebenen Mahlzeitendaten gelernt wird, und

wobei die Insulindosisempfehlung, um den Blutglukosespiegel des Benutzers innerhalb des Zielbereichs zu halten, weiter auf den CGM-Daten, den Insulinabgabedaten, der Insulinsensitivität und dem Kohlenhydratwert basiert.

**2.** Computerlesbares Medium nach Anspruch 1, wobei die empfangene Insulinsensitivität bestimmt wird:

unter Verwendung eines direkten Insulindetektors, um die Insulinmenge im Körper des Benutzers zu messen, und Berechnen der Insulinsensitivität unter Verwendung von Glukoseinformationen für den Benutzer und der Insulinkonzentration im Benutzer; oder
unter Verwendung von Wissen über Aufnahmewerte von Kohlenhydraten in Glukose und Basal- oder Bolusmengen von Insulin, die verabreicht wurden.

## Revendications

**1.** Support non transitoire lisible par ordinateur, contenant des instructions permettant d'amener un environnement informatique à exécuter un procédé de fonctionnement et de réglage ou d'adaptation d'une application d'aide à la décision qui fait partie d'une application de surveillance continue du glucose, CGM, s'exécutant sur un dispositif intelligent, le réglage ou l'adaptation étant effectué de manière à prendre en charge une fonctionnalité souhaitée par l'utilisateur pour maintenir la glycémie de l'utilisateur dans une plage cible, le procédé comprenant les étapes de :

réception d'une indication de la fonctionnalité souhaitée par l'utilisateur pour maintenir la glycémie de l'utilisateur dans la plage cible ;
réception de données d'administration d'insuline et une sensibilité à l'insuline pour l'utilisateur ;
réglage ou adaptation de l'application d'aide à la décision pour prendre en charge la fonctionnalité souhaitée par l'utilisateur, de telle sorte que le réglage ou l'adaptation aboutisse à une recommandation de dose d'insuline pour maintenir la glycémie de l'utilisateur dans la plage cible sur une interface utilisateur au moins partiellement prise en charge par l'application d'aide à la décision, devant reposer sur la fonctionnalité souhaitée par l'utilisateur et sur des données d'entrée en temps réel ;
dans lequel les données en temps réel comprennent des données de surveillance continue du glucose, CGM, provenant d'un capteur faisant partie d'un glucomètre continu, et comprennent en outre des données de repas d'entrée floues saisies par l'utilisateur, les données de repas d'entrée floues étant une catégorisation par l'utilisateur d'un contenu d'un repas ; et
dans lequel le réglage ou l'adaptation comprend la détermination, sur la base des données de repas d'entrée floues, d'une valeur quantitative de glucides correspondante, dans lequel la correspondance entre les données de repas d'entrée floues et la valeur de glucides correspondante est apprise en utilisant l'apprentissage automatique à partir de la réponse glycémique de l'utilisateur pour les données de repas d'entrée floues précédentes, et
dans lequel la recommandation de dose d'insuline pour maintenir la glycémie de l'utilisateur dans la plage cible est en outre basée sur les données CGM, les données d'administration d'insuline, la sensibilité à l'insuline et la valeur de glucides.

**2.** Support lisible par ordinateur selon la revendication 1, dans lequel la sensibilité à l'insuline reçue est déterminée par :

l'utilisation d'un détecteur d'insuline direct pour mesurer la quantité d'insuline dans le corps de l'utilisateur et le calcul de la sensibilité à l'insuline en utilisant des informations sur le glucose de l'utilisateur et la concentration d'insuline chez l'utilisateur ; ou
l'utilisation de la connaissance de valeurs d'apport en glucides transformés en glucose et des quantités d'insuline basale ou en bolus administrées.

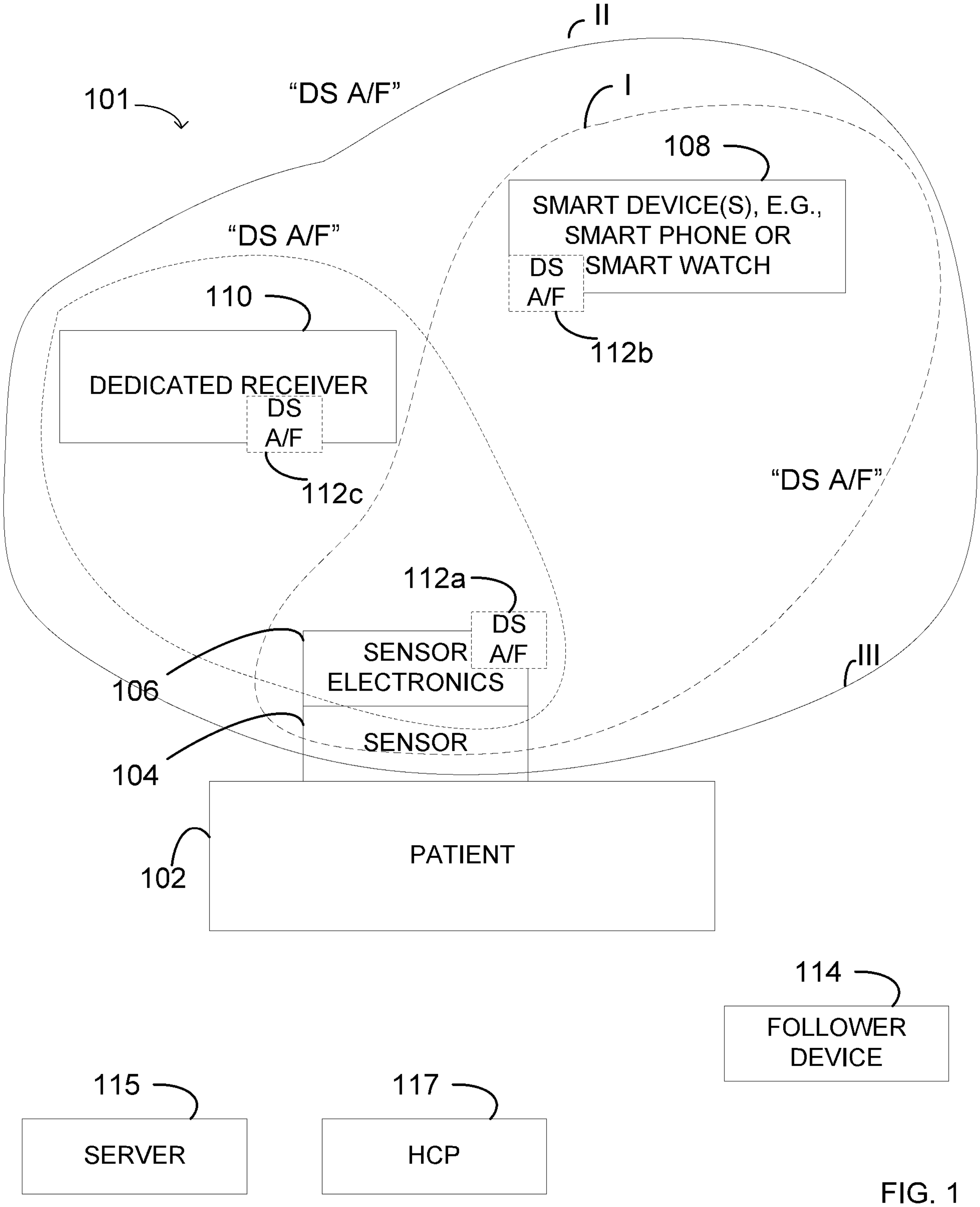
101
"DS A/F"
II
I
108
SMART DEVICE(S), E.G., SMART PHONE OR SMART WATCH
DS A/F
112b
"DS A/F"
110
DEDICATED RECEIVER
DS A/F
112c
"DS A/F"
112a
DS A/F
SENSOR ELECTRONICS
SENSOR
III
106
104
PATIENT
102
114
FOLLOWER DEVICE
115
SERVER
117
HCP

FIG. 1

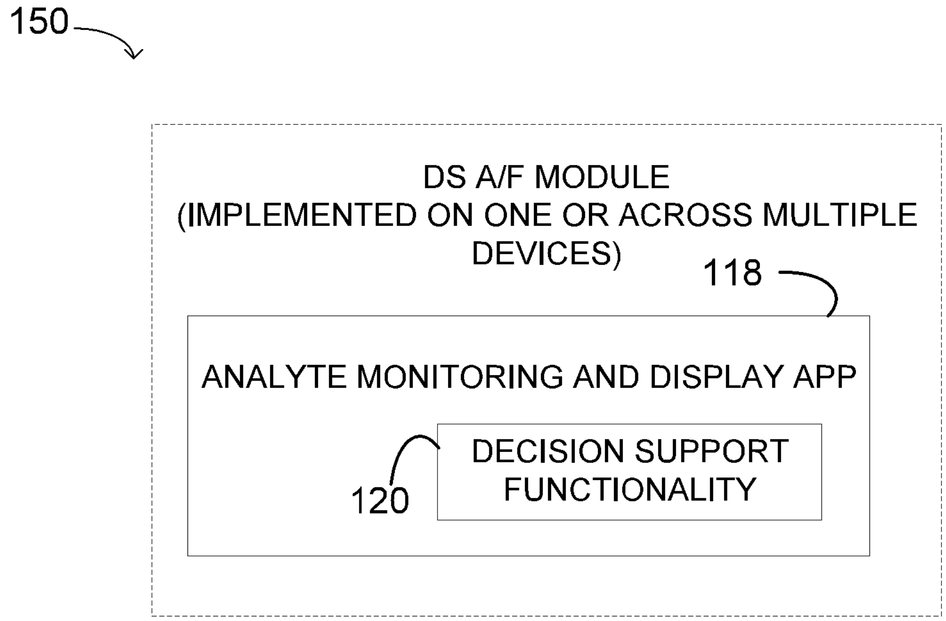
150
DS A/F MODULE
(IMPLEMENTED ON ONE OR ACROSS MULTIPLE DEVICES)
118
ANALYTE MONITORING AND DISPLAY APP
DECISION SUPPORT FUNCTIONALITY
120

FIG. 2

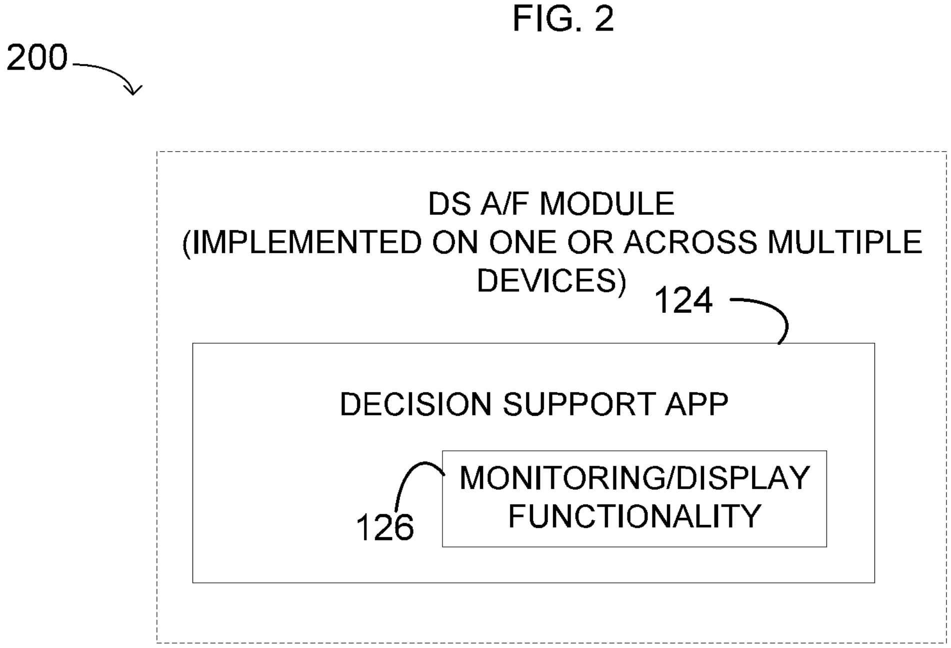
200
DS A/F MODULE
(IMPLEMENTED ON ONE OR ACROSS MULTIPLE DEVICES)
124
DECISION SUPPORT APP
MONITORING/DISPLAY FUNCTIONALITY
126

FIG. 3

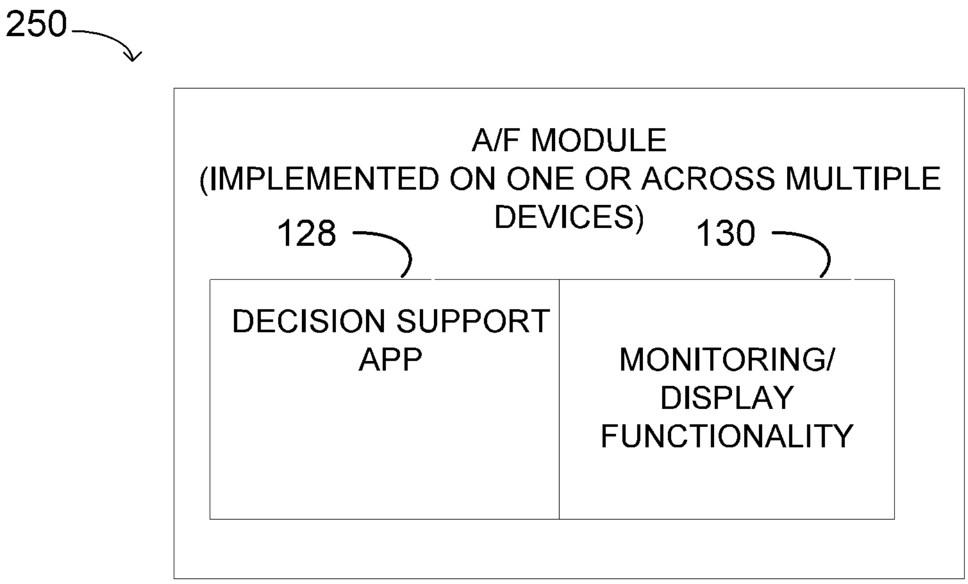
250
A/F MODULE
(IMPLEMENTED ON ONE OR ACROSS MULTIPLE DEVICES)
128
130
DECISION SUPPORT APP
MONITORING/ DISPLAY FUNCTIONALITY

FIG. 4

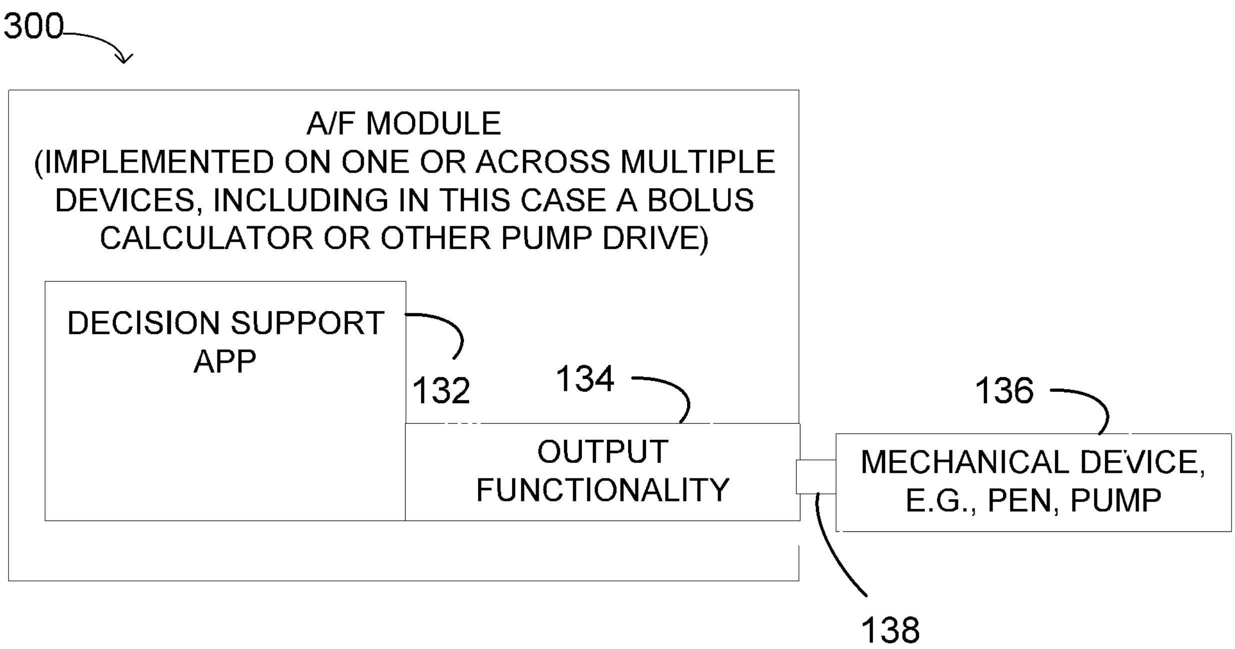
300
A/F MODULE
(IMPLEMENTED ON ONE OR ACROSS MULTIPLE DEVICES, INCLUDING IN THIS CASE A BOLUS CALCULATOR OR OTHER PUMP DRIVE)
DECISION SUPPORT APP
132
134
OUTPUT FUNCTIONALITY
136
MECHANICAL DEVICE, E.G., PEN, PUMP
138

FIG. 5

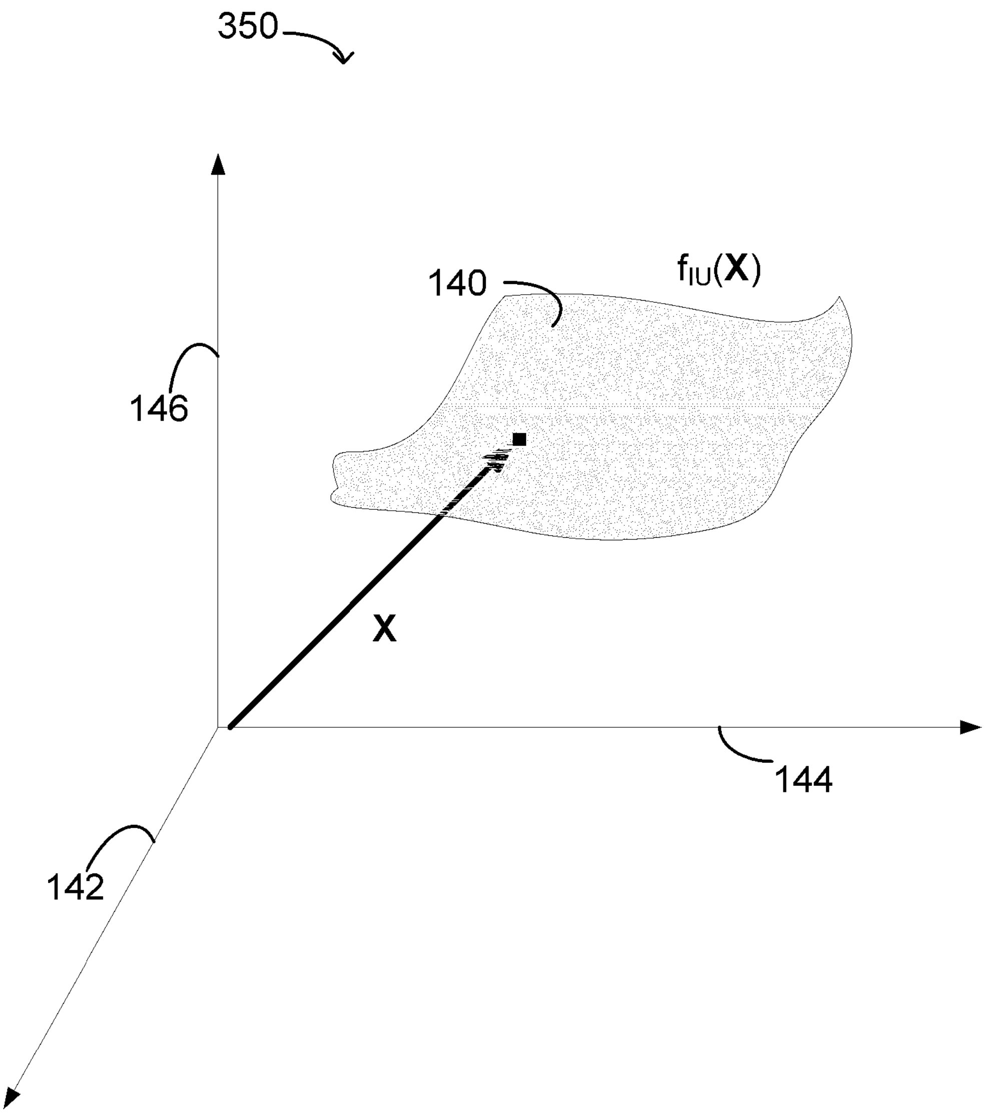


FIG. 6

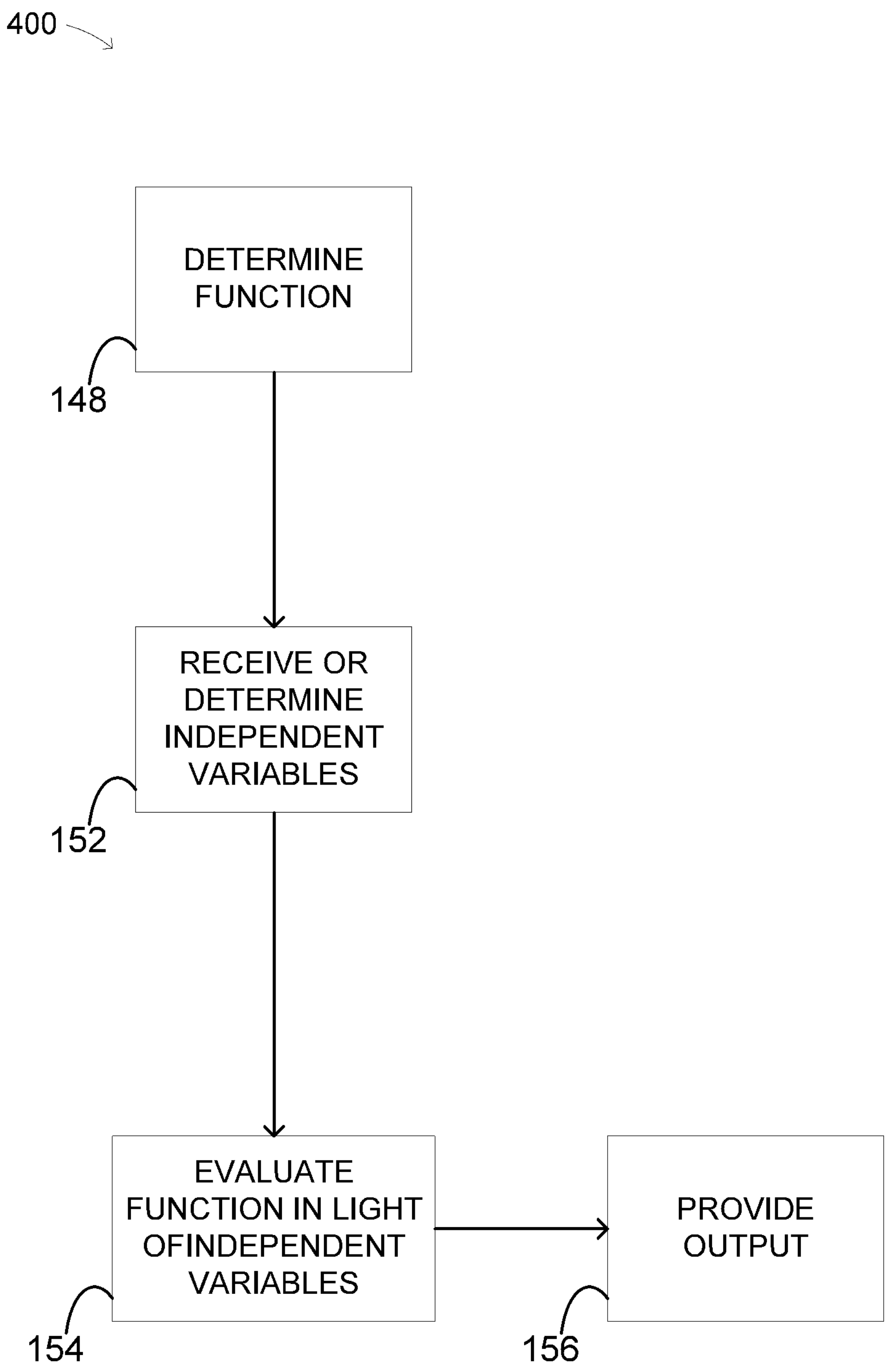
400
DETERMINE FUNCTION
148
RECEIVE OR DETERMINE INDEPENDENT VARIABLES
152
EVALUATE FUNCTION IN LIGHT OFINDEPENDENT VARIABLES
154
PROVIDE OUTPUT
156

FIG. 7

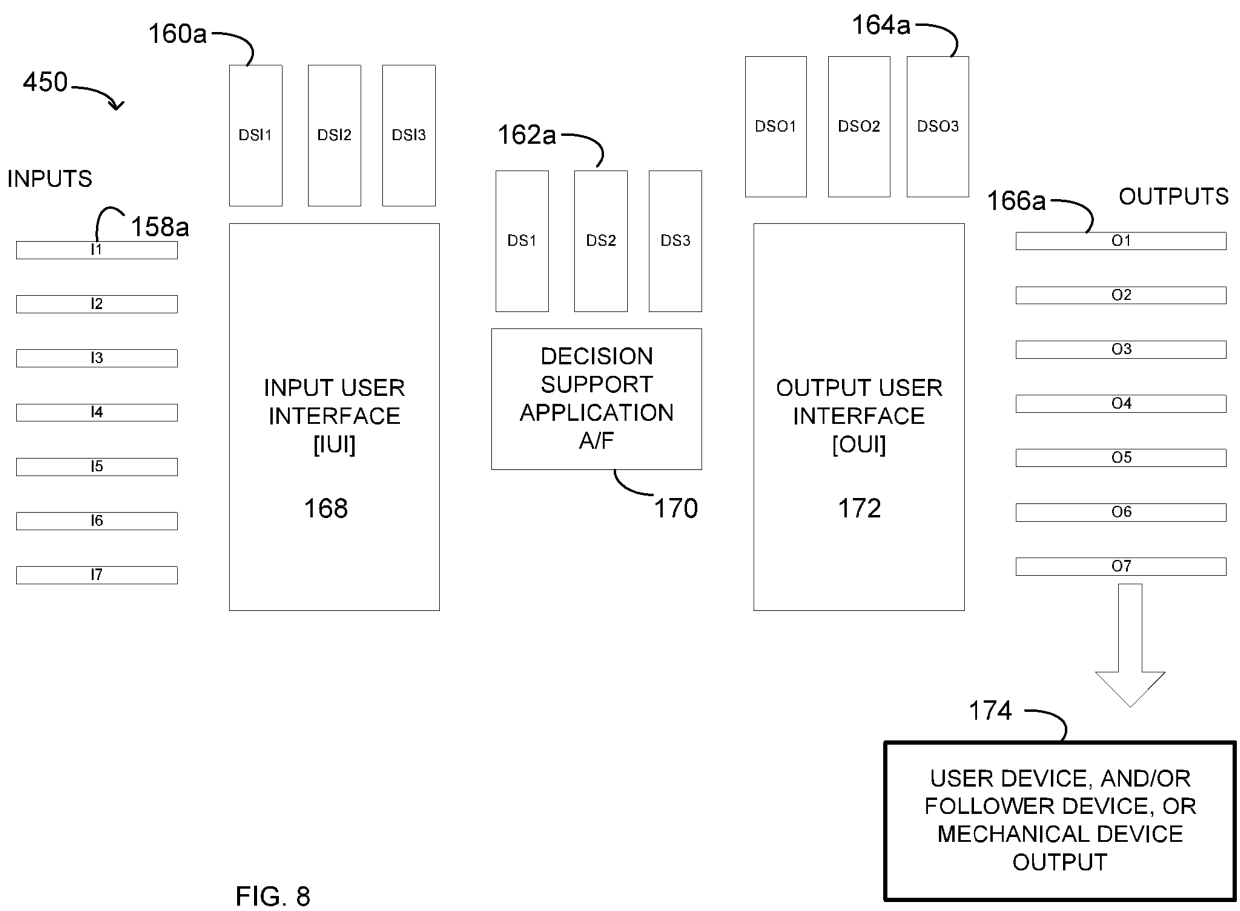
450
INPUTS
158a
I1
I2
I3
I4
I5
I6
I7
160a
DSI1
DSI2
DSI3
INPUT USER INTERFACE [IUI]
168
162a
DS1
DS2
DS3
DECISION SUPPORT APPLICATION A/F
170
164a
DSO1
DSO2
DSO3
OUTPUT USER INTERFACE [OUI]
172
166a
OUTPUTS
O1
O2
O3
O4
O5
O6
O7
174
USER DEVICE, AND/OR FOLLOWER DEVICE, OR MECHANICAL DEVICE OUTPUT

FIG. 8

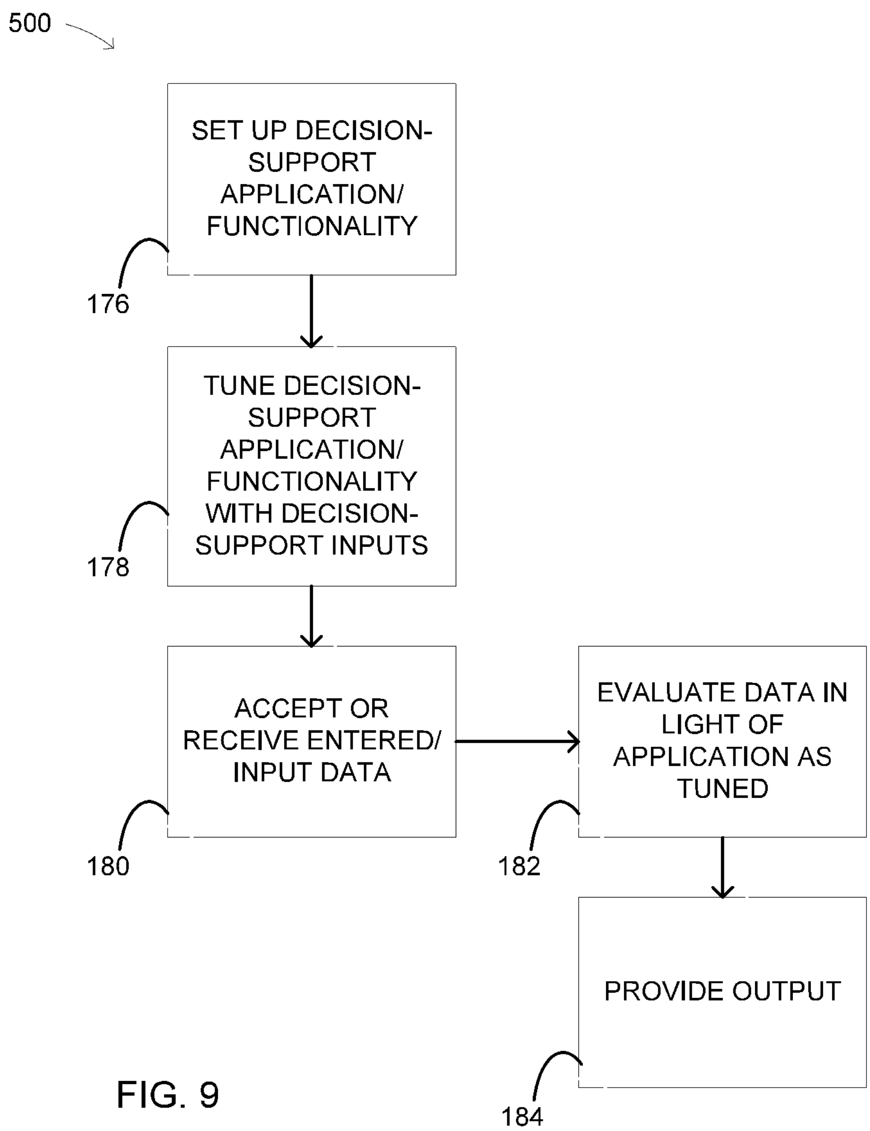
500
SET UP DECISION-SUPPORT APPLICATION/ FUNCTIONALITY
176
TUNE DECISION-SUPPORT APPLICATION/ FUNCTIONALITY WITH DECISION-SUPPORT INPUTS
178
ACCEPT OR RECEIVE ENTERED/ INPUT DATA
180
EVALUATE DATA IN LIGHT OF APPLICATION AS TUNED
182
PROVIDE OUTPUT
184

FIG. 9

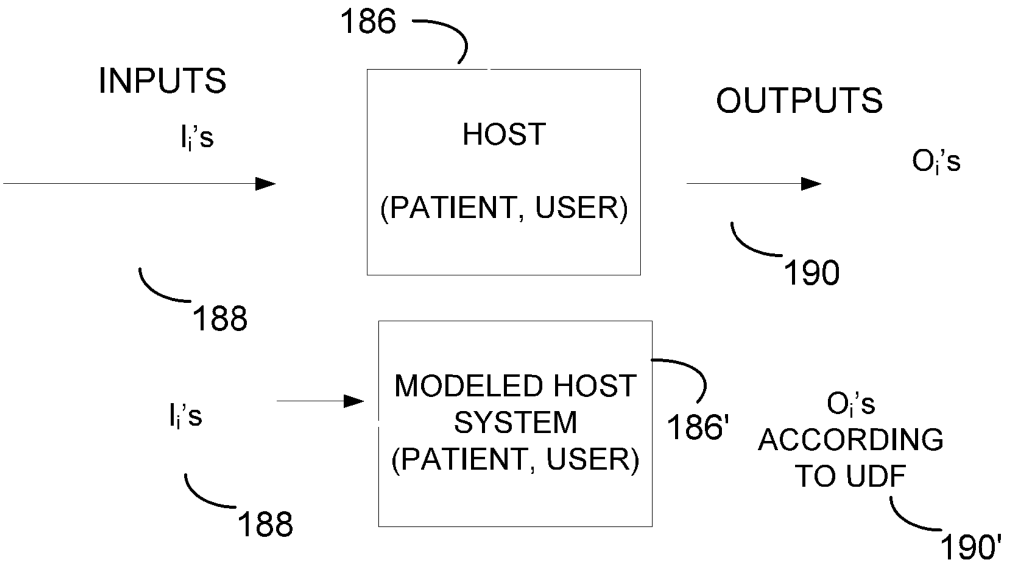


FIG. 10(A)

FIG. 10(B)

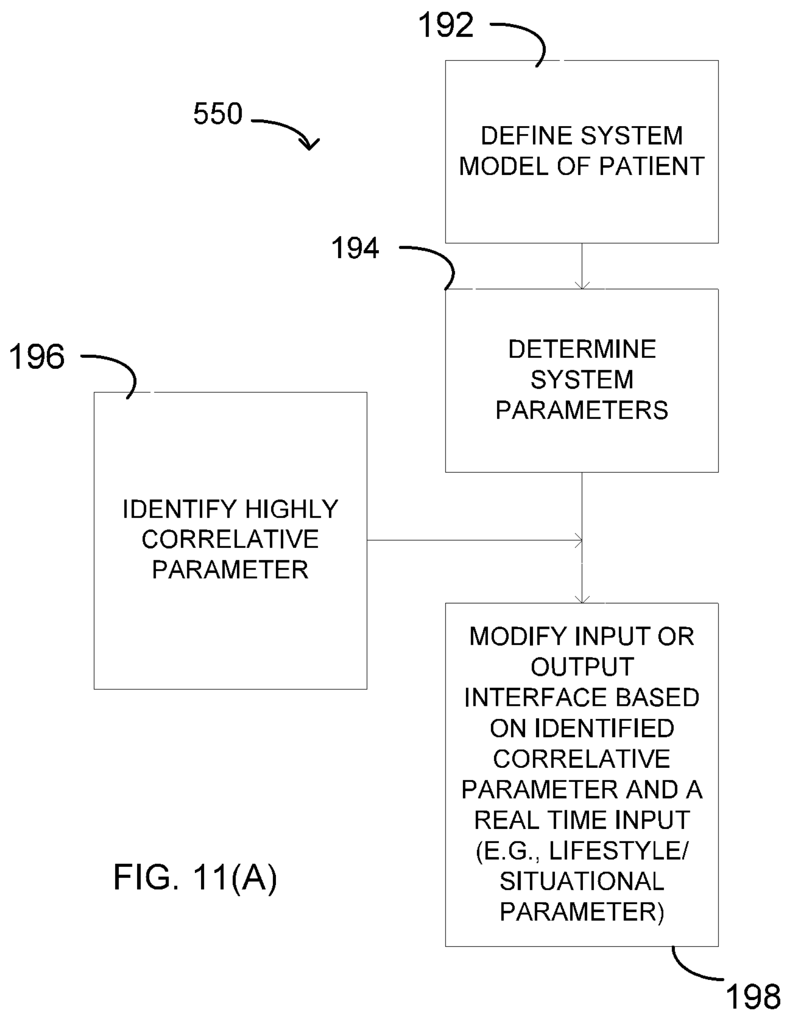


FIG. 11(A)

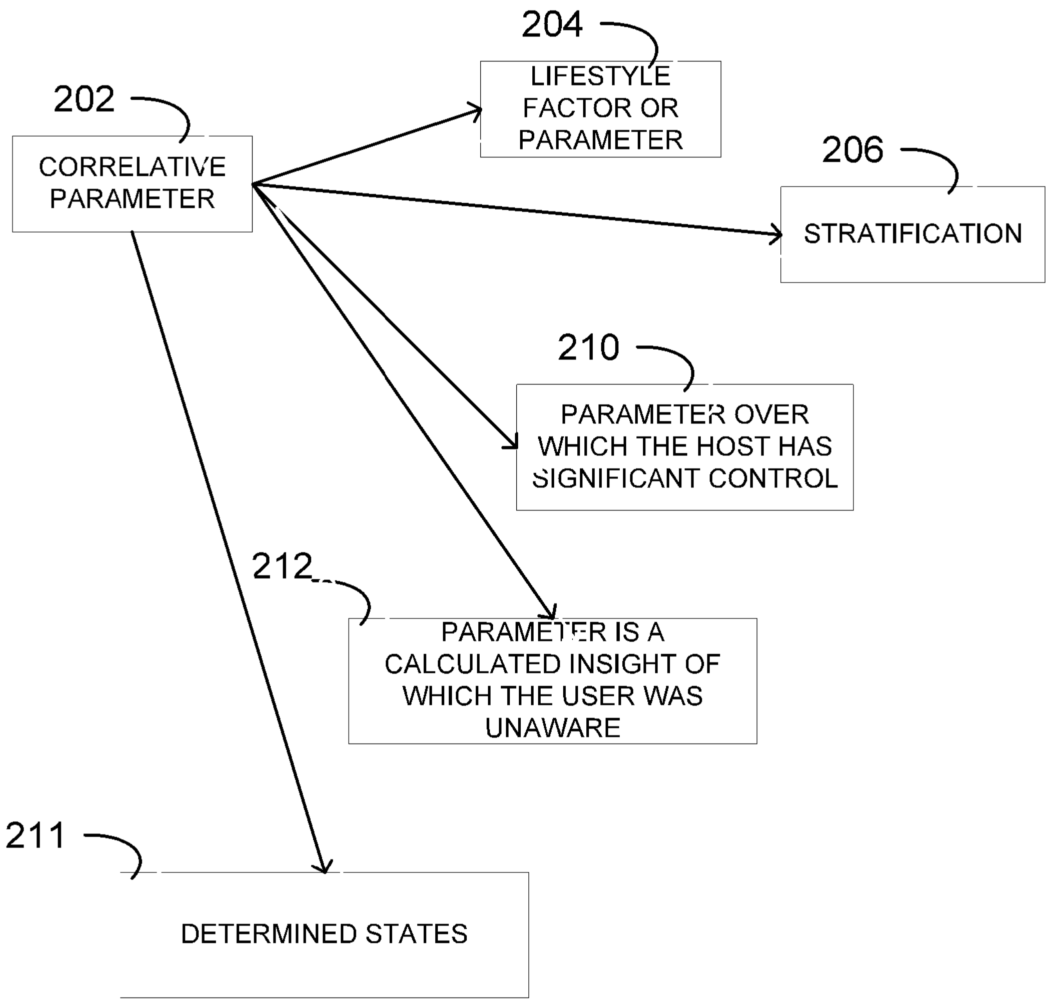
202
CORRELATIVE PARAMETER
204
LIFESTYLE FACTOR OR PARAMETER
206
STRATIFICATION
210
PARAMETER OVER WHICH THE HOST HAS SIGNIFICANT CONTROL
212
PARAMETER IS A CALCULATED INSIGHT OF WHICH THE USER WAS UNAWARE
211
DETERMINED STATES

FIG. 11(B)

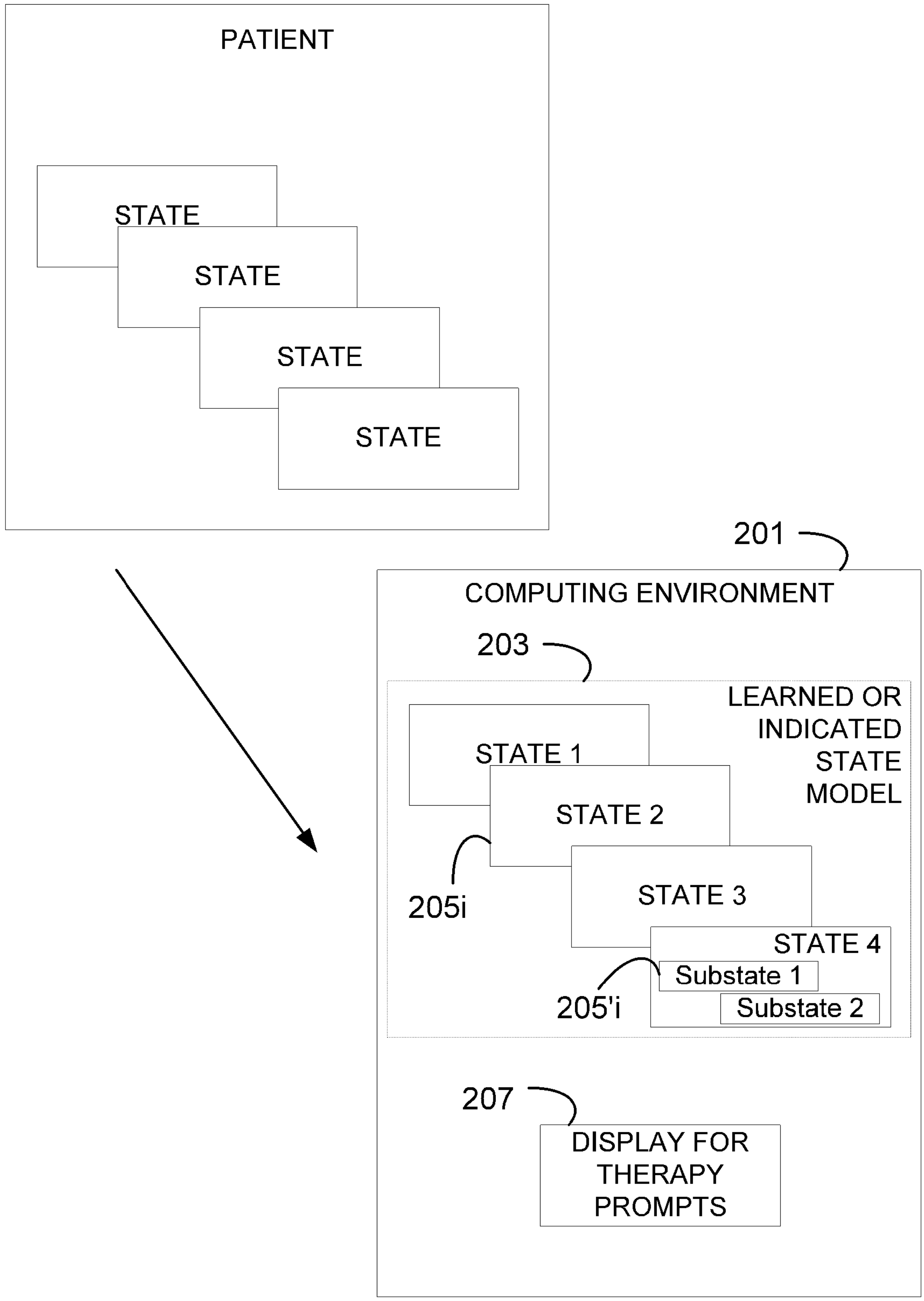
PATIENT
STATE
STATE
STATE
STATE
201
COMPUTING ENVIRONMENT
203
LEARNED OR INDICATED STATE MODEL
STATE 1
STATE 2
205i
STATE 3
STATE 4
Substate 1
Substate 2
205'i
207
DISPLAY FOR THERAPY PROMPTS

FIG. 11(C)

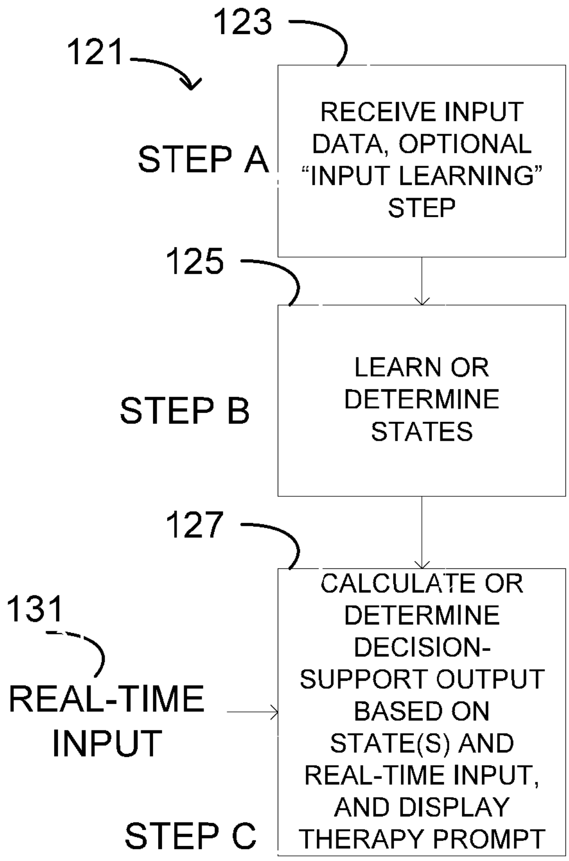


FIG. 12(A)

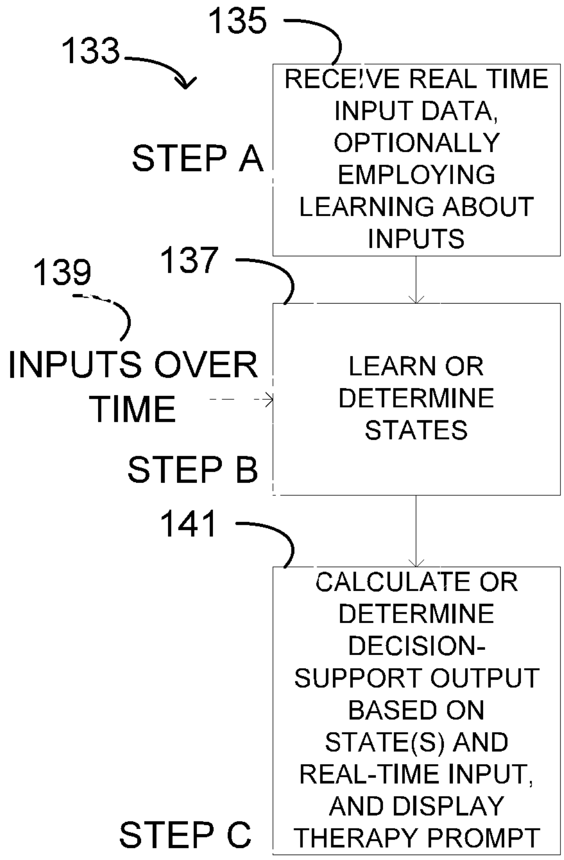


FIG. 12(B)

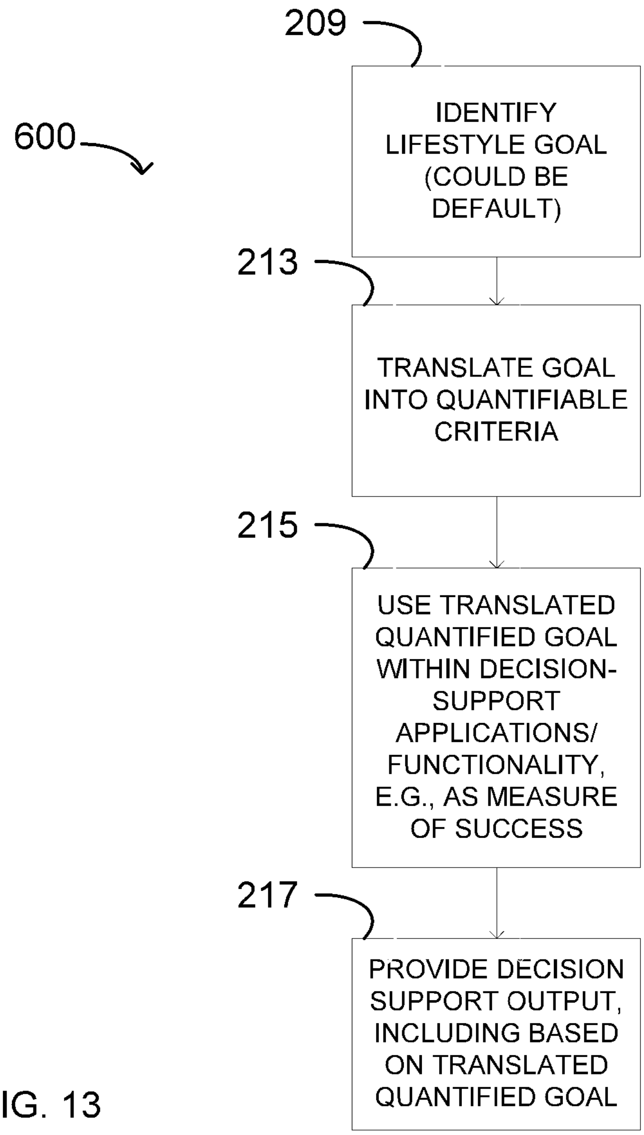
209
600
IDENTIFY LIFESTYLE GOAL (COULD BE DEFAULT)
213
TRANSLATE GOAL INTO QUANTIFIABLE CRITERIA
215
USE TRANSLATED QUANTIFIED GOAL WITHIN DECISION-SUPPORT APPLICATIONS/ FUNCTIONALITY, E.G., AS MEASURE OF SUCCESS
217
PROVIDE DECISION SUPPORT OUTPUT, INCLUDING BASED ON TRANSLATED QUANTIFIED GOAL

FIG. 13

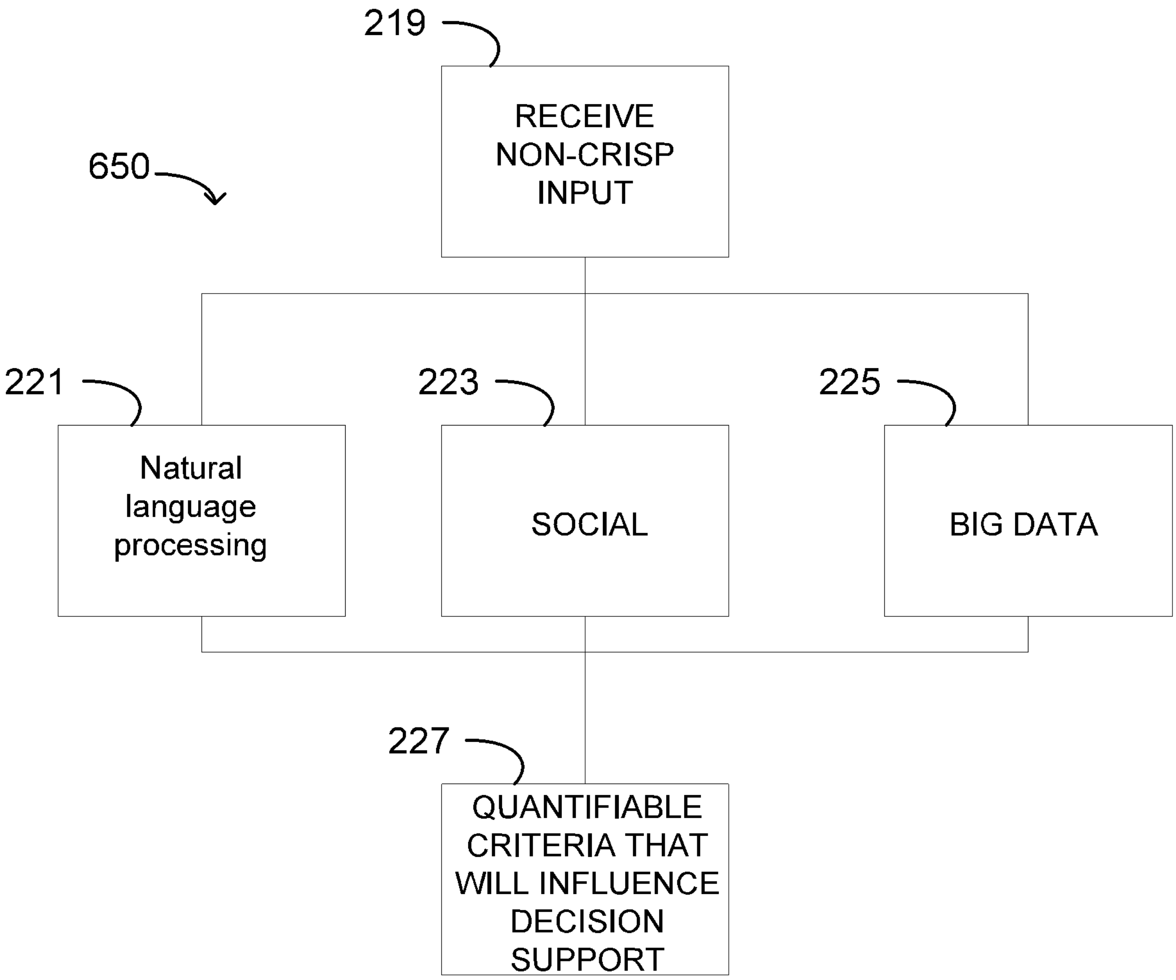
219
650
RECEIVE NON-CRISP INPUT
221
223
225
Natural language processing
SOCIAL
BIG DATA
227
QUANTIFIABLE CRITERIA THAT WILL INFLUENCE DECISION SUPPORT

FIG. 14

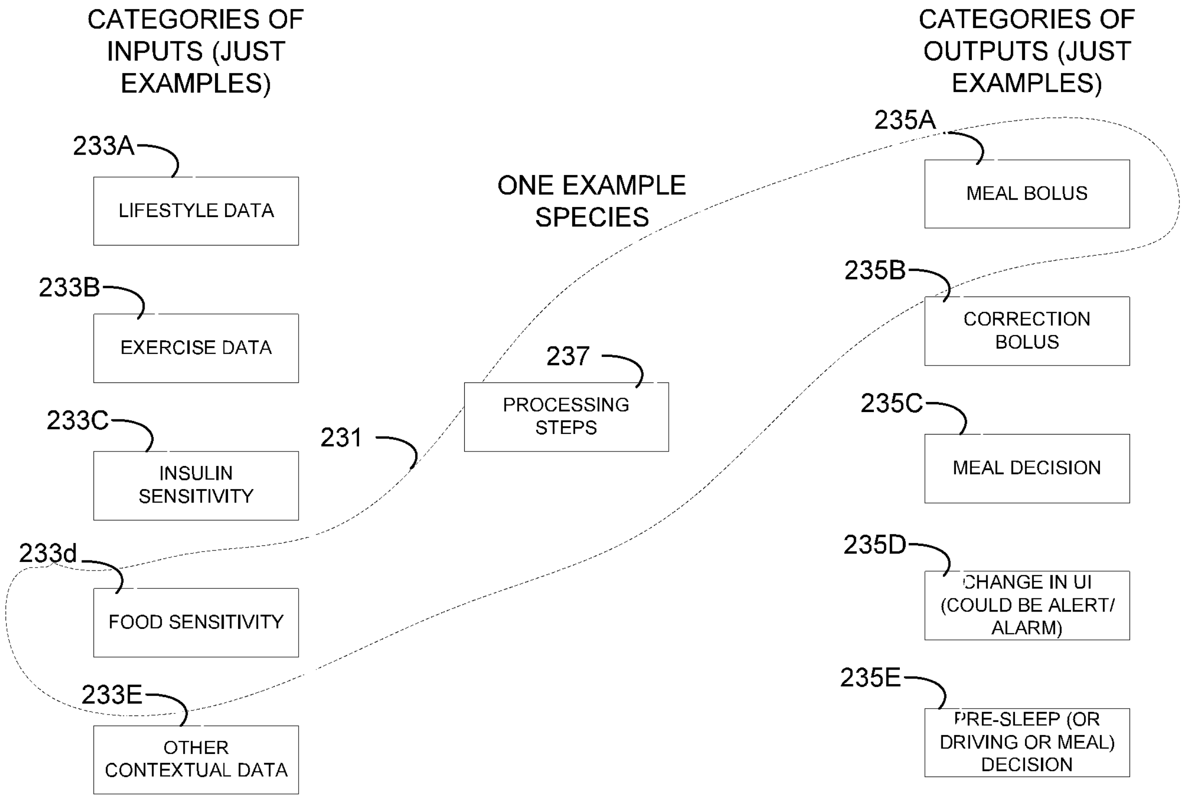
CATEGORIES OF INPUTS (JUST EXAMPLES)
CATEGORIES OF OUTPUTS (JUST EXAMPLES)
ONE EXAMPLE SPECIES
233A
LIFESTYLE DATA
233B
EXERCISE DATA
233C
INSULIN SENSITIVITY
233d
FOOD SENSITIVITY
233E
OTHER CONTEXTUAL DATA
231
237
PROCESSING STEPS
235A
MEAL BOLUS
235B
CORRECTION BOLUS
235C
MEAL DECISION
235D
CHANGE IN UI (COULD BE ALERT/ ALARM)
235E
PRE-SLEEP (OR DRIVING OR MEAL) DECISION

FIG. 15

| CATEGORY OF INPUT | SUB-CATEGORY | SUB-SUB-CATEGORY | SUB-SUB-SUB-CATEGORY | EXEMPLARY FUZZY INPUTS | EXEMPLARY CRISP INPUTS | Type of Context (Lifestyle, situational, clinical, Device) |
|---|---|---|---|---|---|---|
| Real Time Data | Data from Sensors | Physiological Sensors | CGM | Hyperglycemia | 120 mg/dL | |
| | | | | Euglycemia | | |
| | | | | Hypoglycemia | | clinical |
| | | | SMBG | Hyperglycemia | 120 mg/dL | |
| | | | | Euglycemia | | |
| | | | | Hypoglycemia | | clinical |
| | | | physiologic uncertainty/confidence | | | clinical |
| | | | Body Temperature (as temperature affects insulin absorption rate) | cold/normal/fever | 98.6 | clinical |
| | | | gait analysis | | | |
| | | | Skin Conductivity (stress or emotion) | unstressed / stressed | | clinical |
| | | | Hormone Data, e.g., cortisol, epinephrine, and so on | energy in/energy out, and so on | | clinical |
| | | | Heart Rate | low/normal/high | 70 bpm | clinical |
| | | Non-Physiological Sensors | Data from Pump | basal / bolus | 1 unit basal | clinical |
| | | | GPS | home / away | at gym on Main St. | situational |
| | | | Atmospheric pressure, e.g., flying | | flying / not flying | situational |
| | | | Accelerometer (can be used to detect exertion, sleep level) | low / medium / high exertion | ran 30 minutes | situational/Lifestyle |
| | | | Exercise from sensor triboelectric effects, GPS | low / medium / high exertion | ran 30 minutes | lifestyle |
| | | | Sensor Accuracy / Confidence Range / Signal Quality. May also include device uncertainty/confidence. E.g., CGM will only make decision recommendation if three previous five-minute values exist/are reported/are good, and are accompanied by trend arrow | 5% accuracy, 10% accuracy | | device |
| | | | External Temperature | cold / normal / high | 108 deg | situational |
| | User-Entered Data | SMBG | | hypo/eu/hyper | 120 mg/dL | clinical |
| | | Exercise | Direct user input, calendar data and/or pattern data | strenuous / medium / easy | burned 1000 calories, walked 3 miles | lifestyle |
| | | Goal (self-management, social goals, decision-support goals) | e.g., level of interaction, level of discretion, tight control, good control at work/school, good control before sleep so as to avoid being disturbed (may include calendar/clock as a input) | tight control at school & looser control | I want to wake up with my glucose at 100 | lifestyle/clinical |
| | | Problem to be Solved | nightly lows | stay within euglycemic range | stay within 20 mg/dL of desired target | lifestyle |
| | | A Desire for Therapy (level of therapy aggressiveness) | | "alert me a lot" | alert me 3x/day | lifestyle |
| | | Stress / Emotion / Feeling Data | | stressed out / feeling fine | heart rate elevated by x% | lifestyle/clinical |
| | | Pain level (can even be considered its own sensitivity) | | low/medium/high | pain level 1-10 | lifestyle/clinical |
| | | Sleep level | | high / low | slept 6.5 hours | lifestyle/clinical |
| | | Data from a follower device | | acknowledgement / advice | therapy recommendation | lifestyle |
| | | User Feedback | | prompt me for more feedback/prompt me for less feedback | prompt me for feedback no more than 1x/day | lifestyle/situational |
| | | type of insulin | | | | clinical |
| | | Desired readiness for upcoming activities (may sync with calendar data too): e.g., user has big event coming up, so they want to be reminded a lot before, but not during the event | | | | lifestyle |
| | | Meal Data | | big/medium/small | 20 g carbs | lifestyle |
| | | Meal Data | GPS (e.g., Restaurant data combined with pattern data) | home / away | at gym on Main St. | lifestyle |
| | | | Calendar data, e.g., combined with pattern data | | | lifestyle |
| | | | data from a microphone, e.g., "always on", Smart phone can identify when ordering at a restaurant, e.g., in combination with GPS | | | lifestyle |

FIG. 16

| | | | | | | |
|---|---|---|---|---|---|---|
| | | | inferred from signal, e.g., glucose signal or rate of change | | | lifestyle |
| | | | Pattern Recognition | | | lifestyle |
| | | | Derived from Camera App | small / medium / large | 30g carbs | lifestyle |
| | | Calendar | | weekend/weekday | Tuesday | lifestyle |
| | | GPS | | home / away | at gym on Main St. | lifestyle/situational |
| | | CGM App | | hypo/eu/hyper | 84 mg/dL | clinical |
| | | Historical Data or Other Information | | categorized pattern of X or Y | | clinical |
| | Data from Apps, via a suitable API | SMBG (where such is not user entered) | | hypo/eu/hyper | 84 mg/dL | clinical |
| | | Temperature Monitor (external or body) (where such is not user entered) | | cold / normal / hot | 101 deg | clinical |
| | | HealthKit | | | | various |
| | | Data from follower, e.g., from text messaging app | | acknowledgement / advice | therapy recommendation | lifestyle |
| | | Bolus Calculator App (including not just bolus info but type/brand of insulin, and duration of delivery) | | big bolus / medium bolus / small bolus | 1.5 unites | clinical |
| | | Time of Day | | day / night | 1654 | situational |
| | | "Relative" Time - one aspect of DS is that the user may want to bolus but that we know a more preferable time for them to bolus - so we prompt them accordingly, based on patterns, which then triggers a bolus calculator | | | | lifestyle/situational |
| | | Big Data | patient categorization | obese / normal | patients over X BMI should consider Y | various |
| | | analyte rate of change or acceleration | | rise / rapid rise | 10 mg/dL/hour | clinical |
| | | sensitivities / resistances | insulin | high/med/low | | lifestyle |
| | | | sleep | high/med/low | | lifestyle |
| | | | meal | high/med/low | | lifestyle |
| | | | exercise | high/med/low | | lifestyle |
| | | Hierarchy within Risk Stratification (as more/less data is available, move down/up risk stratification) | | high risk / med risk / low risk | | various |
| | | glucose variability | | high/med/low | 25 mg/dL | clinical |
| | Derived Data (data derived in real time) | User Emotion / Feeling, e.g., depression, whether user is talking with others a lot, whether they are trying to distract themselves by playing games, etc. Large # of emails, low bank balance, etc. | | | | lifestyle |
| | | ability to distribute insulin, e.g., after boluses delivered, e.g., "take a walk" | | | | clinical |
| | | User desire for interaction with device (derived from level of interaction) | | high/med/low | wants constant notification | lifestyle |
| | | Sensor accuracy / confidence range / signal quality | may bear on decision-support mode, e.g., a device determined decision-support mode | high/med/low | 10% | device |
| | | recognized patterns | nightly lows | typical / atypical | | lifestyle |
| | | | post-prandial highs, etc. | typical / atypical | | lifestyle |
| | | SMBG | | hypo/eu/hyper | | clinical |
| | | type and brand of insulin | fast acting, slow acting, and so one | | | clinical |
| | | User savviness with technology | | neophyte / normal / savvy | | lifestyle |
| | | Goal | Hypoglycemia avoidance versus hyperglycemia avoidance, more/less interaction, more/less alerts/alarms | high/med/low level of interaction | desire for specific low GV | lifestyle |
| | | Problem to be Solved | | | | lifestyle |
| | | Desire (e.g., level of user discretion, desired level of interaction) | | | | lifestyle |
| | | decision-support mode | therapeutic, non-therapeutic, adjunctive, non-adjunctive, user goal mode, right problem to be solved mode, decision to be made mode | | | lifestyle/device |
| | | Target glucose | | within eu range | 100 mg/dL +/- 10% | lifestyle/clinical |
| | User-Entered Data | type of diabetes (and whether they are insulin-dependent) | if insulin-dependent, are they intensive insulin dependent or non-intensive insulin-dependent (intensive insulin-dependent typically associated with higher glucose rates of change as compared to non-insulin users) | | | clinical |

FIG. 16 (cont.)

| | | | | | | |
|---|---|---|---|---|---|---|
| | | Workout Data (to allow patterns in post workout glycemic fluctuations to be found, and for appropriate automatic or manual therapy modifications to be made) | cardio 1, cardio 2 and so on | | | lifestyle |
| | | Age | | child/teen/young adult / middle age adult / old adult / elderly | 45 years old | Demographic |
| | | Cardio Health / Cholesterol | | good/poor | | clinical |
| | | Gender | | M/F | M/F | demographic |
| | | *Type of Mathematical Calculation* (may be entered by physician/technician/user - may be related to desired level of accuracy / interaction) | | fuzzy/approx versus as precise as possible | | device |
| | | Illness / Pregnancy / Menstruation | | type/level of illness, whether pregnant or menstruating | | various |
| | | Medications/ Smoking / Alcohol | | smoker/non-smoker | 10 cigs / day | lifestyle |
| | | Goal | | | | lifestyle |
| | Derived Data (data derived from analysis of past data (overlaps in some cases with derived data from real time data) | Historical Patterns | | see patterns above | | lifestyle/clinical |
| | | Gastric Emptying Duration, note that the same may also be user entered | | | time duration | clinical |
| | | decision-support mode | | | | lifestyle/device |
| | | situational parameter associated with variable or poorly predicted glucose levels, e.g., time of day, day of week, type of event, etc. May also be associated with glucose variability | | | | various |
| | | user savviness/level of comfort with technology | | "I really like data" versus "I don't want any interruptions" | | lifestyle |
| | | *Other Historial Conclusions About Data* | *glucose variability* | | | various |
| Non-Real Time Data, typically Historic Data, e.g., Stored Data | Other non-real-time data | data about follower, e.g., tone of communication, language used | | follower desires updates or not | follower desires hourly updates | lifestyle |

FIG. 16 (cont.)

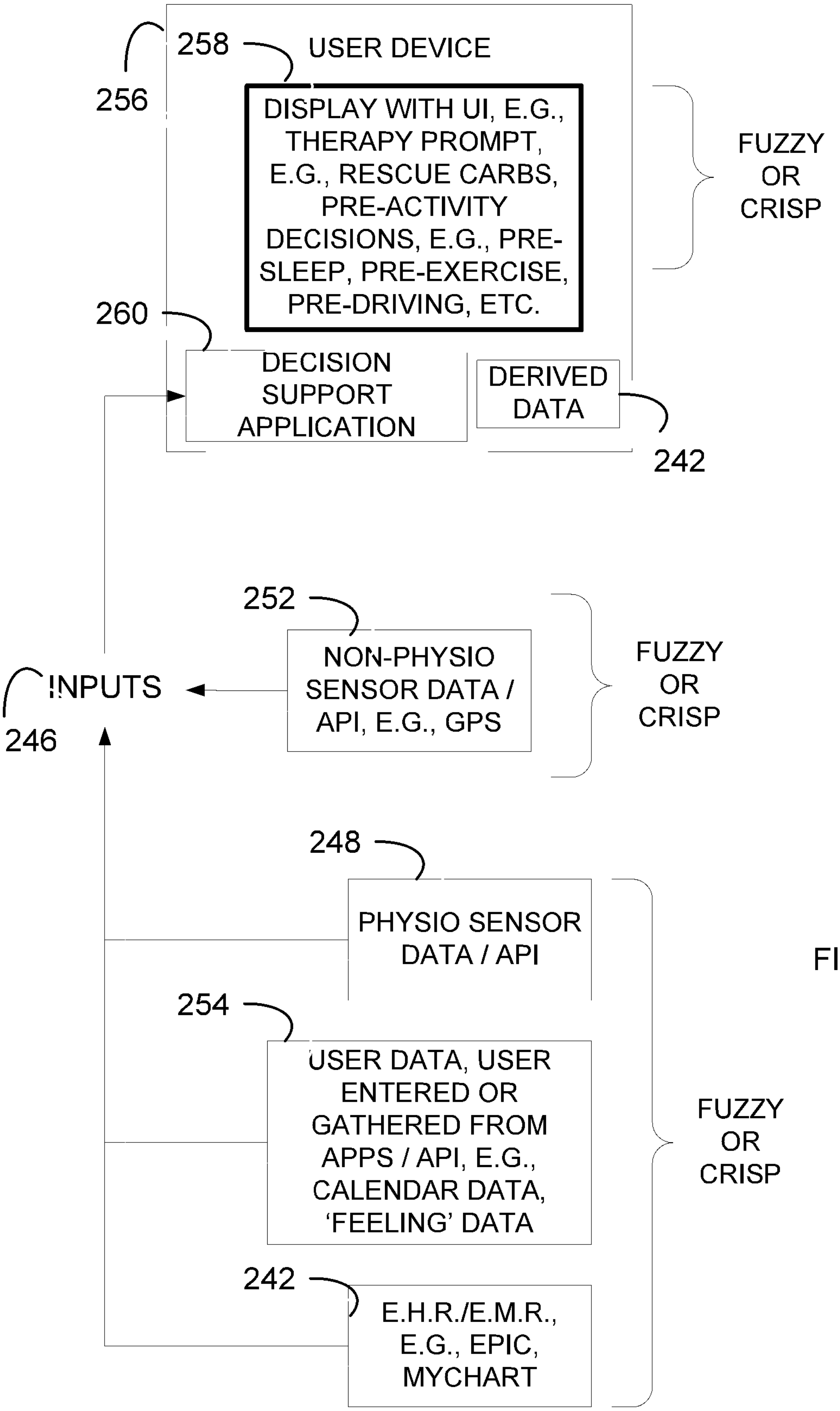
256
258
USER DEVICE
DISPLAY WITH UI, E.G., THERAPY PROMPT, E.G., RESCUE CARBS, PRE-ACTIVITY DECISIONS, E.G., PRE-SLEEP, PRE-EXERCISE, PRE-DRIVING, ETC.
FUZZY OR CRISP
260
DECISION SUPPORT APPLICATION
DERIVED DATA
242
252
INPUTS
246
NON-PHYSIO SENSOR DATA / API, E.G., GPS
FUZZY OR CRISP
248
PHYSIO SENSOR DATA / API
254
USER DATA, USER ENTERED OR GATHERED FROM APPS / API, E.G., CALENDAR DATA, 'FEELING' DATA
FUZZY OR CRISP
242
E.H.R./E.M.R., E.G., EPIC, MYCHART

FIG. 17

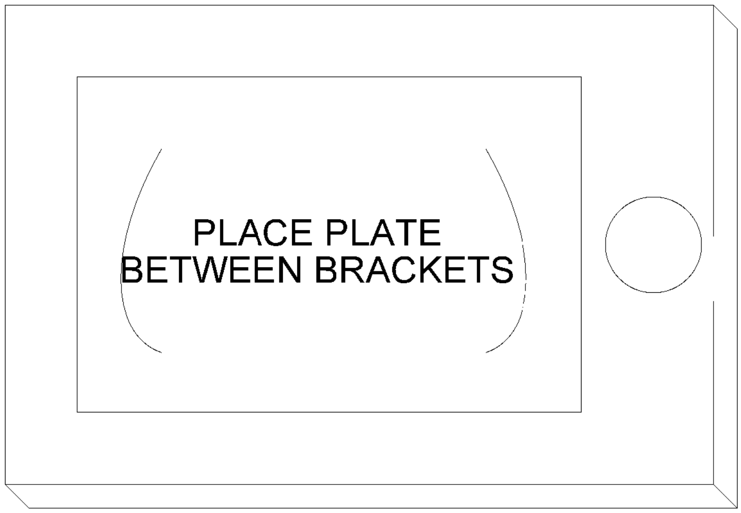


FIG. 18(A)

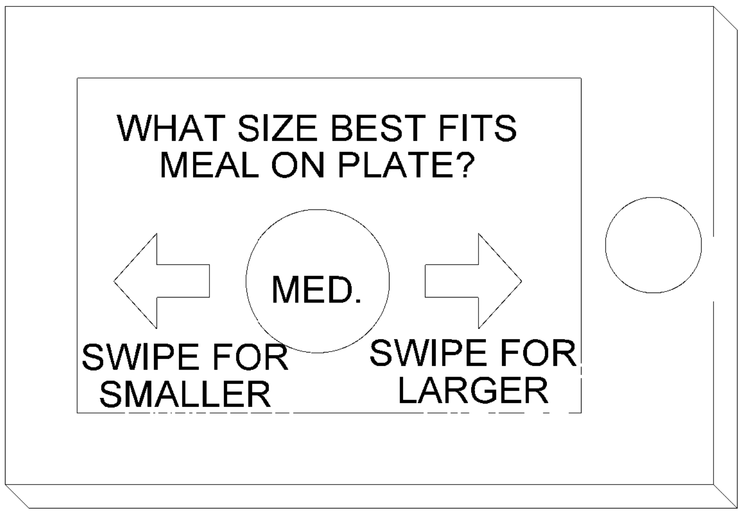


FIG. 18(B)

FIG. 18

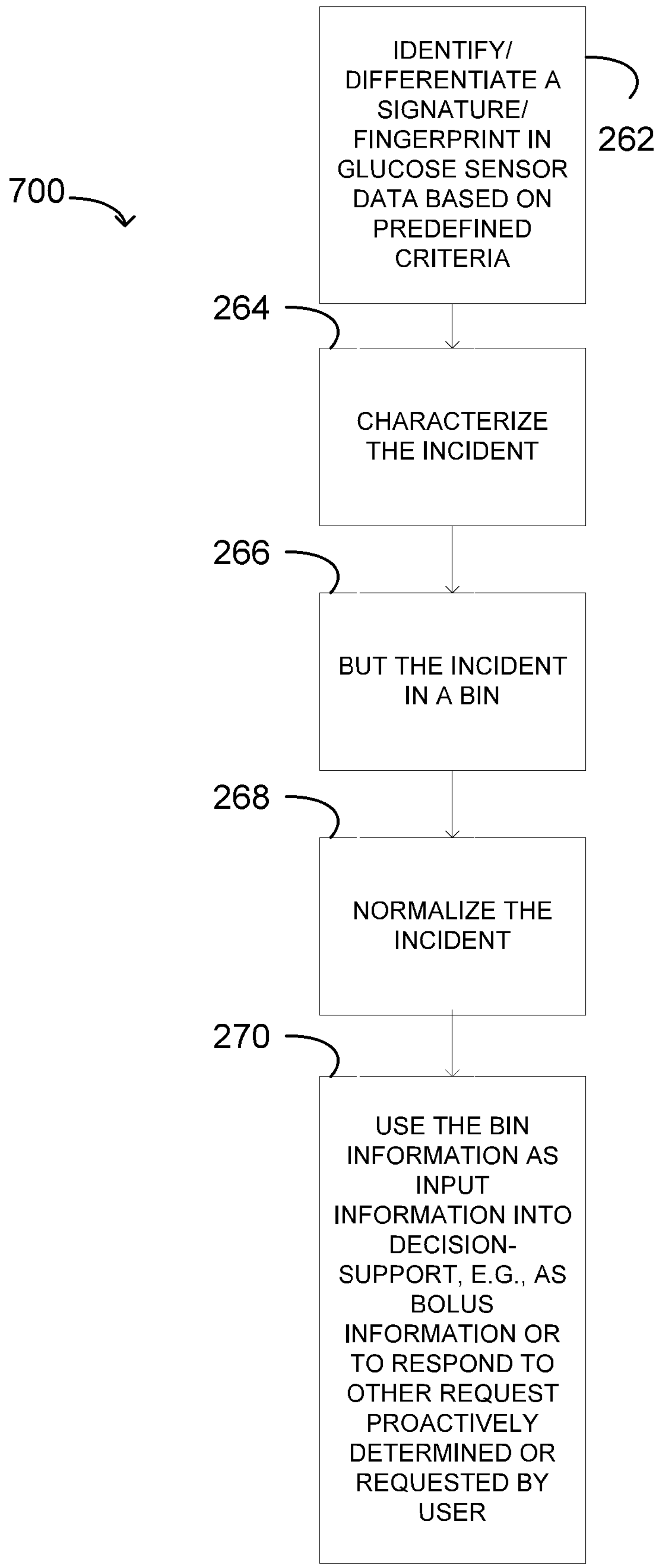
700
IDENTIFY/ DIFFERENTIATE A SIGNATURE/ FINGERPRINT IN GLUCOSE SENSOR DATA BASED ON PREDEFINED CRITERIA
262
264
CHARACTERIZE THE INCIDENT
266
BUT THE INCIDENT IN A BIN
268
NORMALIZE THE INCIDENT
270
USE THE BIN INFORMATION AS INPUT INFORMATION INTO DECISION-SUPPORT, E.G., AS BOLUS INFORMATION OR TO RESPOND TO OTHER REQUEST PROACTIVELY DETERMINED OR REQUESTED BY USER

FIG. 19

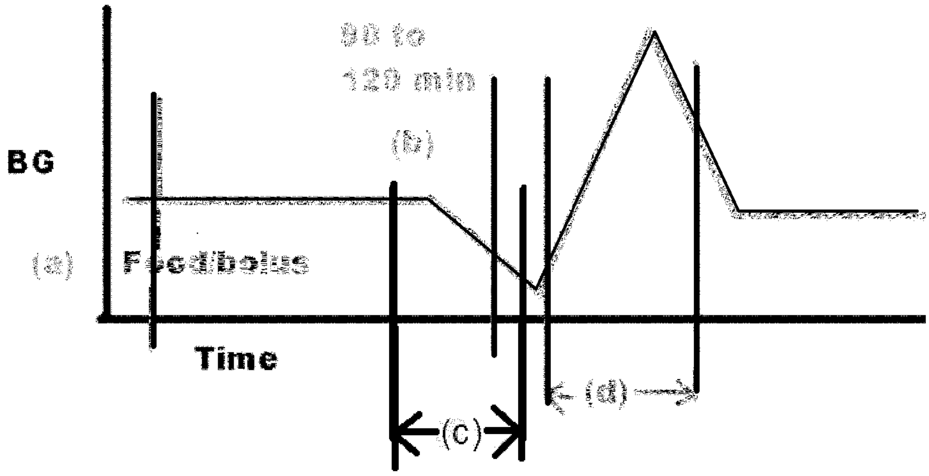


FIG. 20

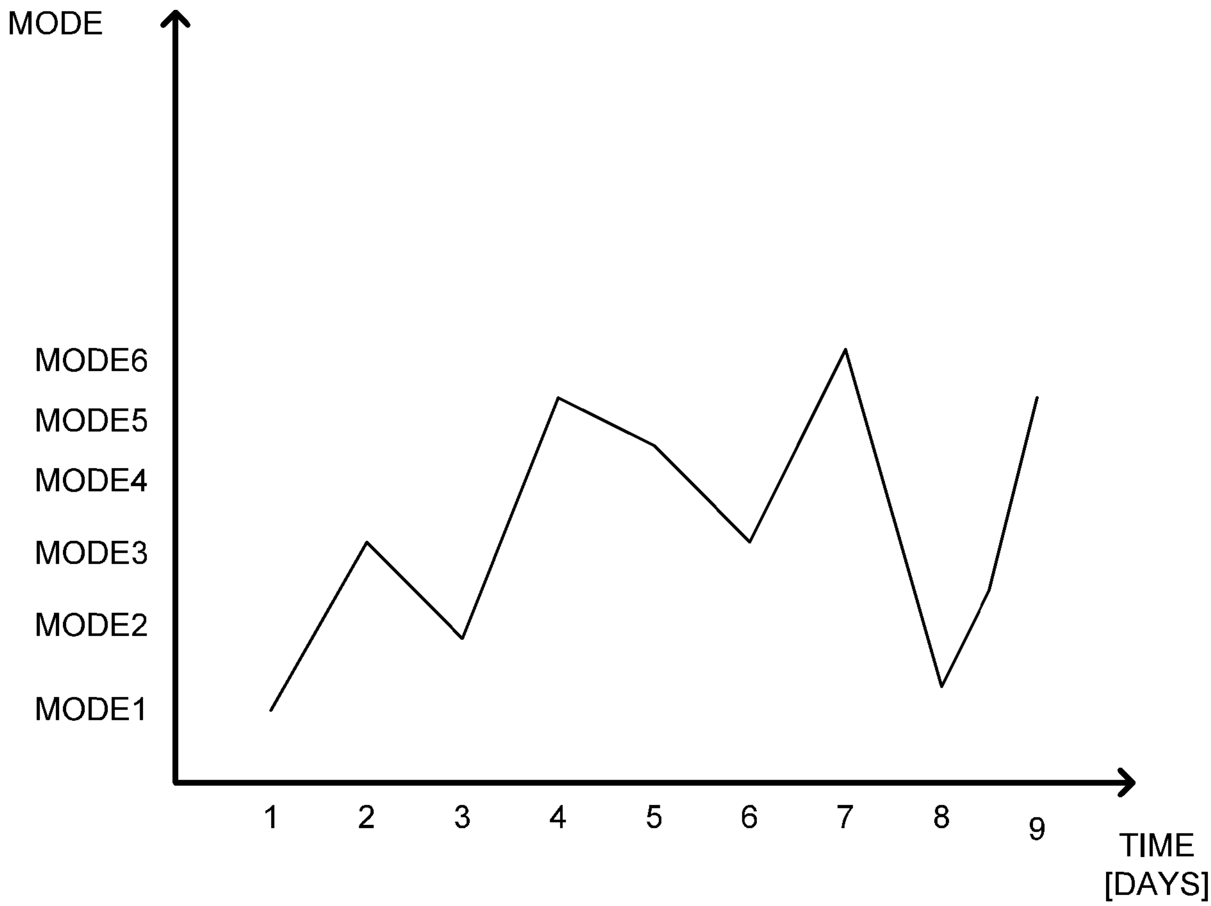
MODE
MODE6
MODE5
MODE4
MODE3
MODE2
MODE1
1
2
3
4
5
6
7
8
9
TIME
[DAYS]

FIG. 21

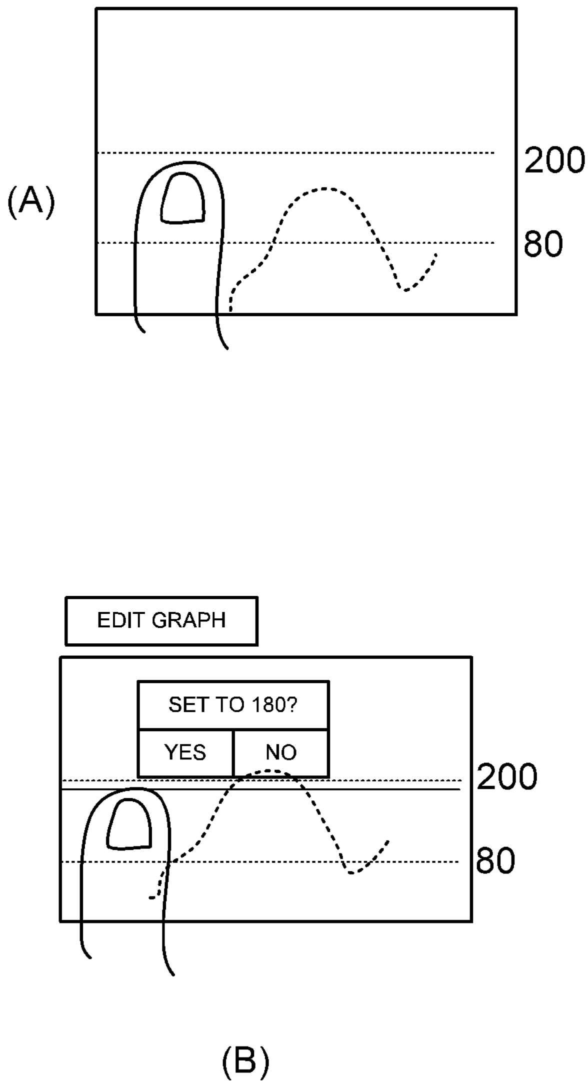


FIG. 22

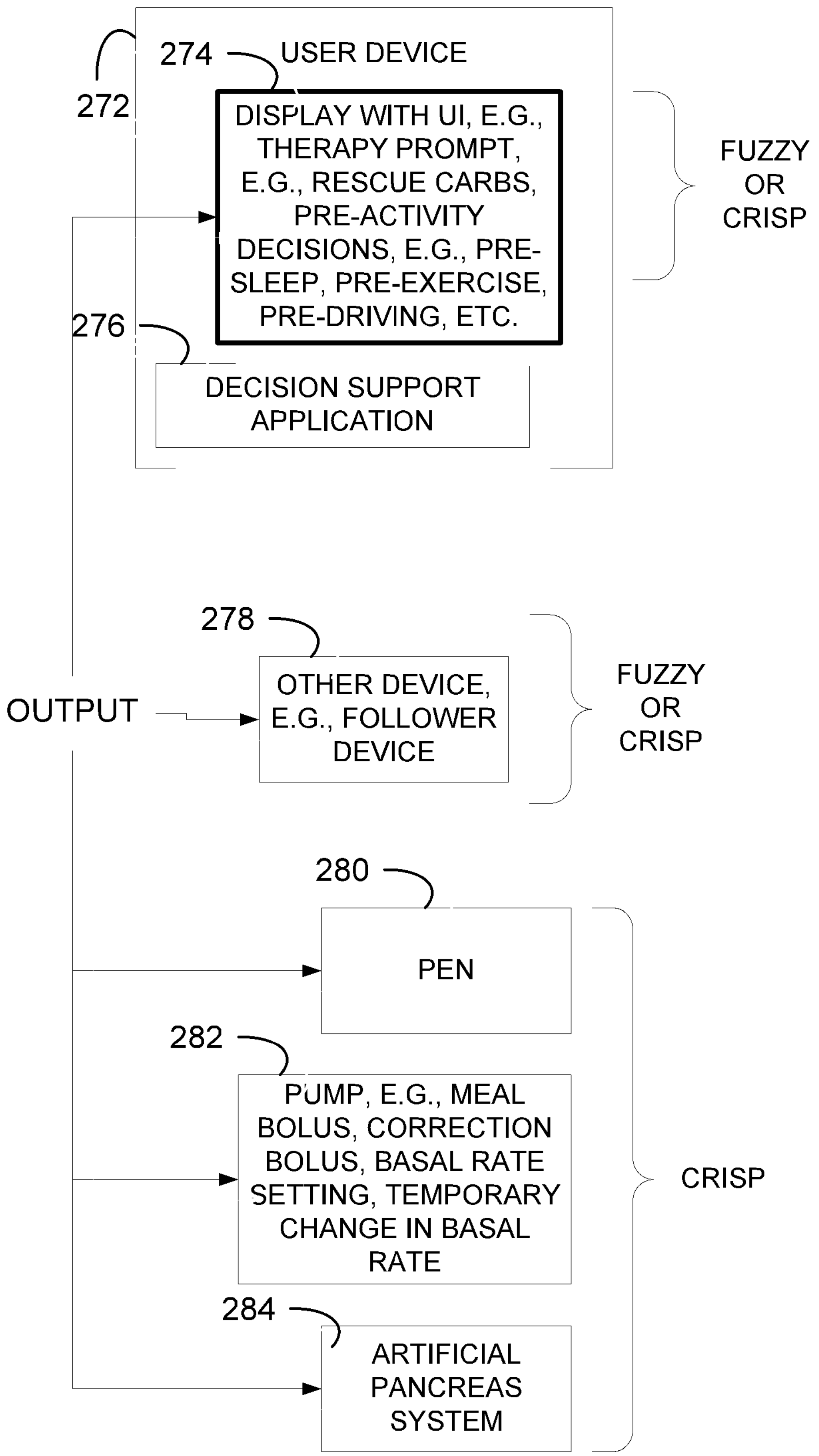
272
274
USER DEVICE
DISPLAY WITH UI, E.G., THERAPY PROMPT, E.G., RESCUE CARBS, PRE-ACTIVITY DECISIONS, E.G., PRE-SLEEP, PRE-EXERCISE, PRE-DRIVING, ETC.
276
DECISION SUPPORT APPLICATION
FUZZY OR CRISP
278
OUTPUT
OTHER DEVICE, E.G., FOLLOWER DEVICE
FUZZY OR CRISP
280
PEN
282
PUMP, E.G., MEAL BOLUS, CORRECTION BOLUS, BASAL RATE SETTING, TEMPORARY CHANGE IN BASAL RATE
284
ARTIFICIAL PANCREAS SYSTEM
CRISP

FIG. 23A

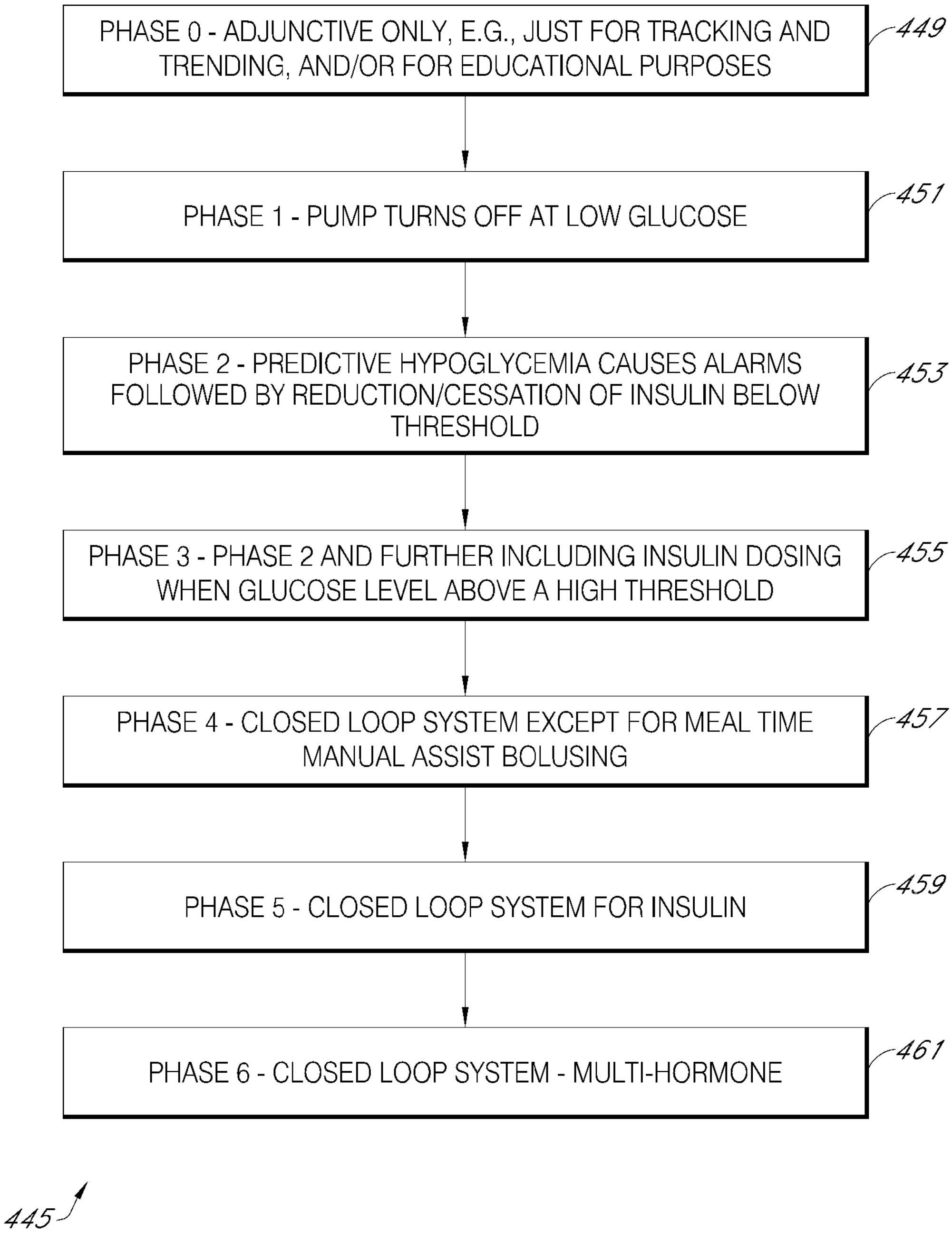
PHASE 0 - ADJUNCTIVE ONLY, E.G., JUST FOR TRACKING AND TRENDING, AND/OR FOR EDUCATIONAL PURPOSES
449
PHASE 1 - PUMP TURNS OFF AT LOW GLUCOSE
451
PHASE 2 - PREDICTIVE HYPOGLYCEMIA CAUSES ALARMS FOLLOWED BY REDUCTION/CESSATION OF INSULIN BELOW THRESHOLD
453
PHASE 3 - PHASE 2 AND FURTHER INCLUDING INSULIN DOSING WHEN GLUCOSE LEVEL ABOVE A HIGH THRESHOLD
455
PHASE 4 - CLOSED LOOP SYSTEM EXCEPT FOR MEAL TIME MANUAL ASSIST BOLUSING
457
PHASE 5 - CLOSED LOOP SYSTEM FOR INSULIN
459
PHASE 6 - CLOSED LOOP SYSTEM - MULTI-HORMONE
461
445

FIG. 23B

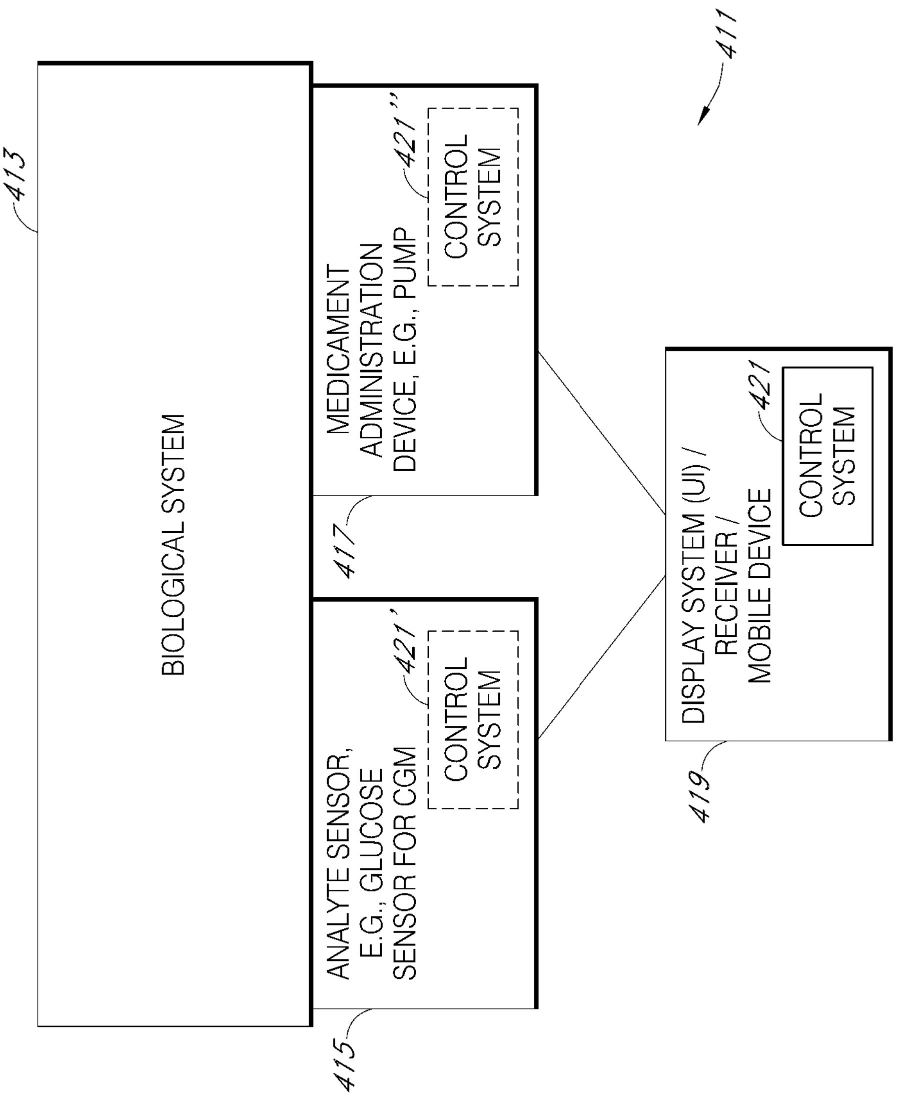
411
413
BIOLOGICAL SYSTEM
MEDICAMENT ADMINISTRATION DEVICE, E.G., PUMP
421''
CONTROL SYSTEM
417
ANALYTE SENSOR, E.G., GLUCOSE SENSOR FOR CGM
421'
CONTROL SYSTEM
415
DISPLAY SYSTEM (UI) / RECEIVER / MOBILE DEVICE
421
CONTROL SYSTEM
419

FIG. 23C

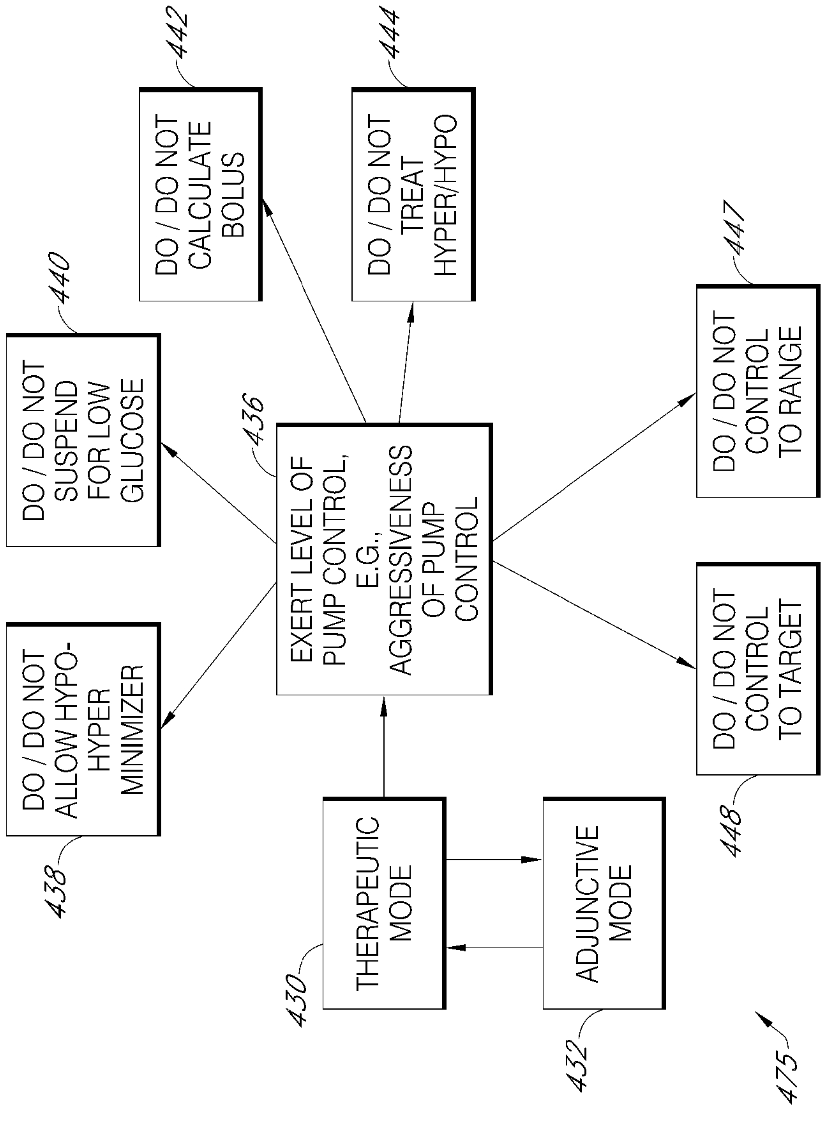
THERAPEUTIC MODE
430
ADJUNCTIVE MODE
432
EXERT LEVEL OF PUMP CONTROL, E.G., AGGRESSIVENESS OF PUMP CONTROL
436
DO / DO NOT ALLOW HYPO-HYPER MINIMIZER
438
DO / DO NOT SUSPEND FOR LOW GLUCOSE
440
DO / DO NOT CALCULATE BOLUS
442
DO / DO NOT TREAT HYPER/HYPO
444
DO / DO NOT CONTROL TO RANGE
447
DO / DO NOT CONTROL TO TARGET
448
475

FIG. 23D

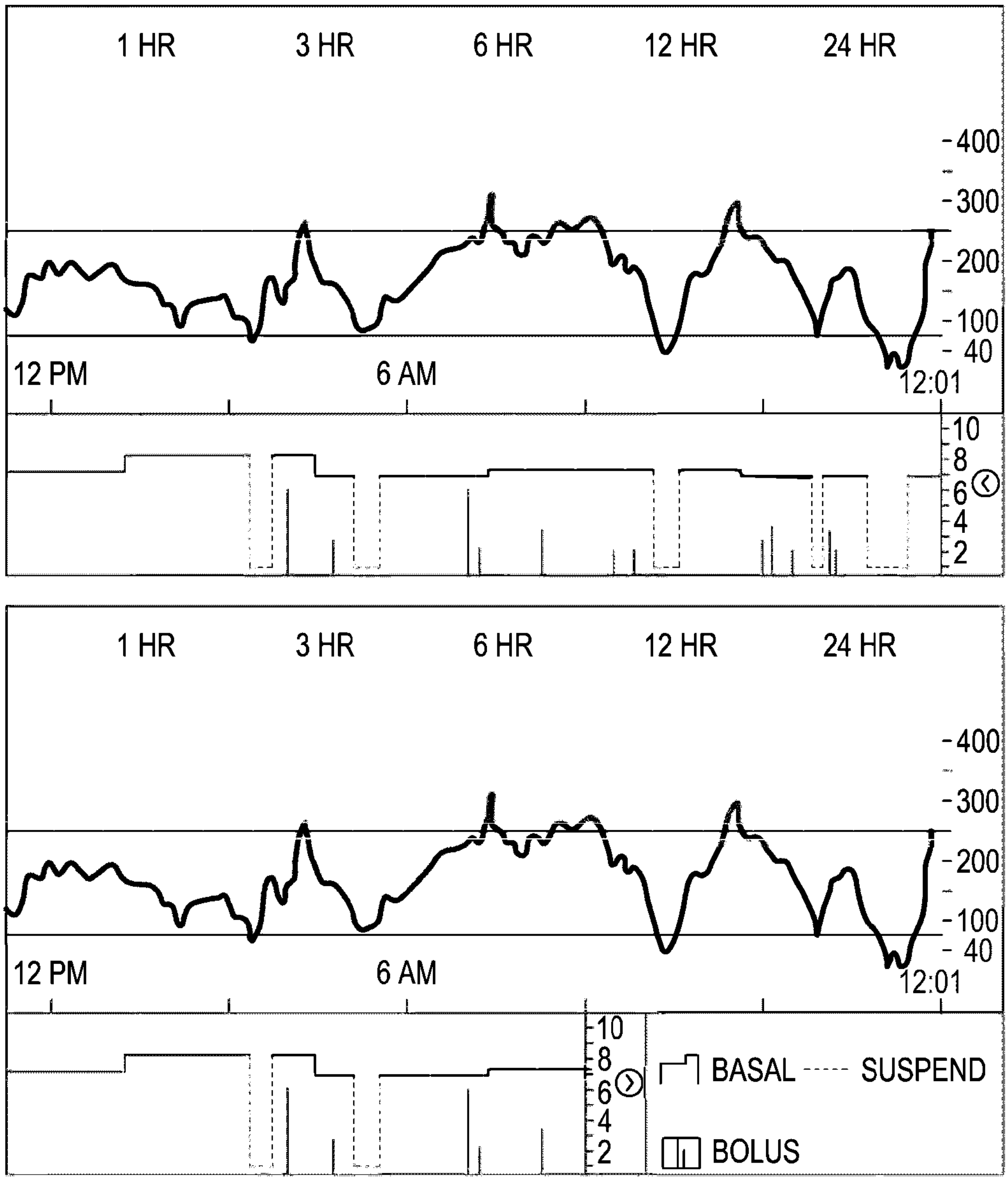
1 HR
3 HR
6 HR
12 HR
24 HR
400
300
200
100
40
12 PM
6 AM
12:01
10
8
6
4
2
1 HR
3 HR
6 HR
12 HR
24 HR
400
300
200
100
40
12 PM
6 AM
12:01
10
8
6
4
2
BASAL
SUSPEND
BOLUS

FIG. 24

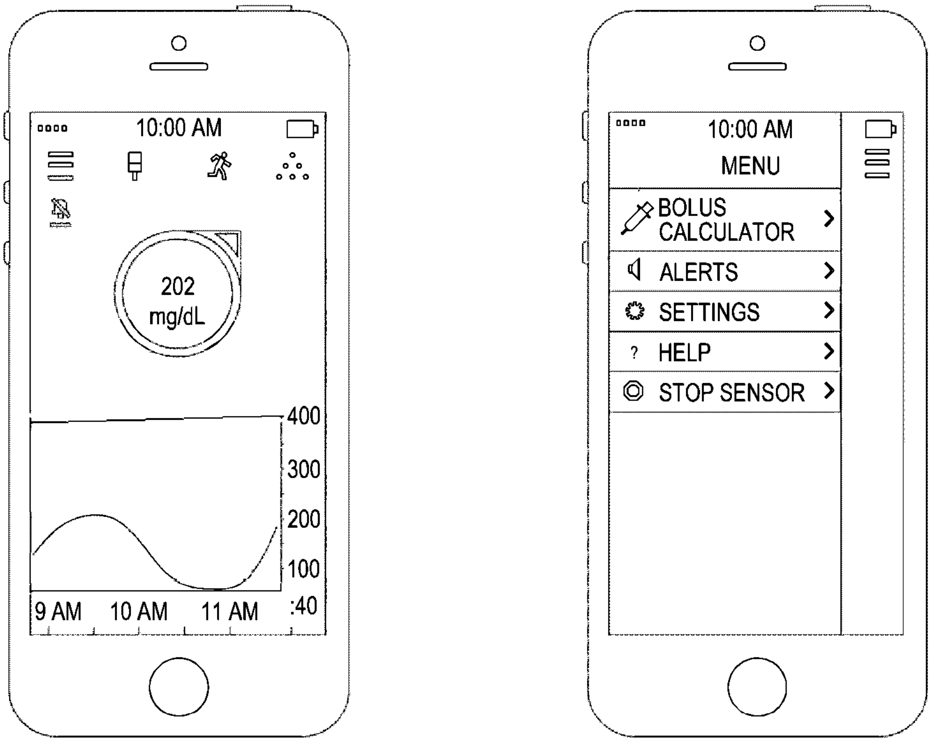
10:00 AM
202
mg/dL
400
300
200
100
:40
9 AM
10 AM
11 AM
10:00 AM
MENU
BOLUS CALCULATOR
ALERTS
SETTINGS
HELP
STOP SENSOR

FIG. 25A

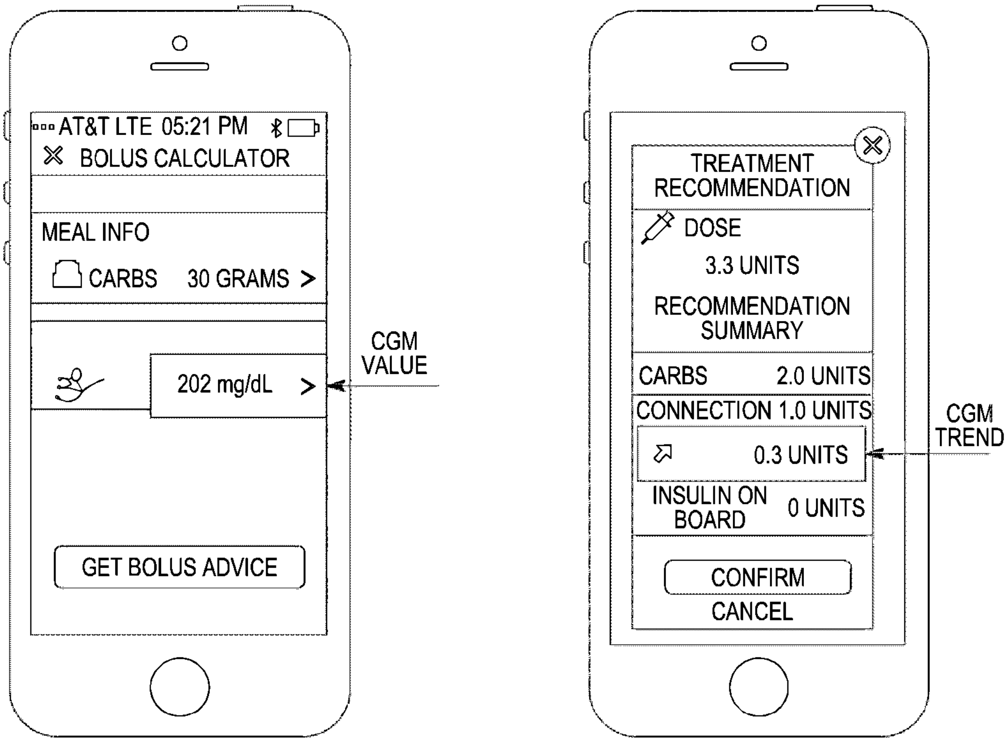
AT&T LTE 05:21 PM
BOLUS CALCULATOR
MEAL INFO
CARBS 30 GRAMS >
202 mg/dL >
GET BOLUS ADVICE
CGM VALUE
TREATMENT RECOMMENDATION
DOSE
3.3 UNITS
RECOMMENDATION SUMMARY
CARBS 2.0 UNITS
CONNECTION 1.0 UNITS
0.3 UNITS
INSULIN ON BOARD 0 UNITS
CONFIRM
CANCEL
CGM TREND

FIG. 25B

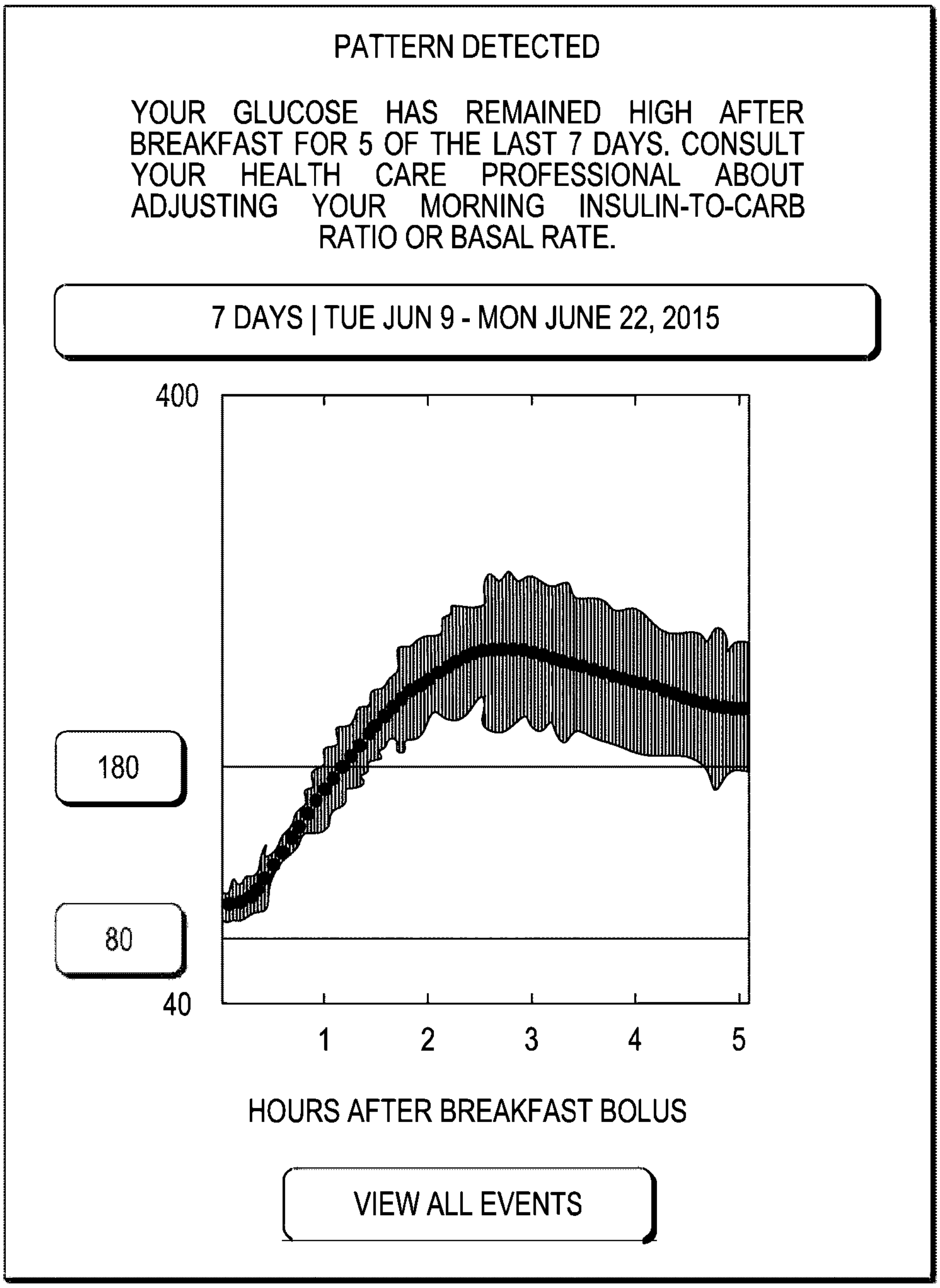
PATTERN DETECTED
YOUR GLUCOSE HAS REMAINED HIGH AFTER BREAKFAST FOR 5 OF THE LAST 7 DAYS. CONSULT YOUR HEALTH CARE PROFESSIONAL ABOUT ADJUSTING YOUR MORNING INSULIN-TO-CARB RATIO OR BASAL RATE.
7 DAYS | TUE JUN 9 - MON JUNE 22, 2015
400
180
80
40
1
2
3
4
5
HOURS AFTER BREAKFAST BOLUS
VIEW ALL EVENTS

FIG. 26

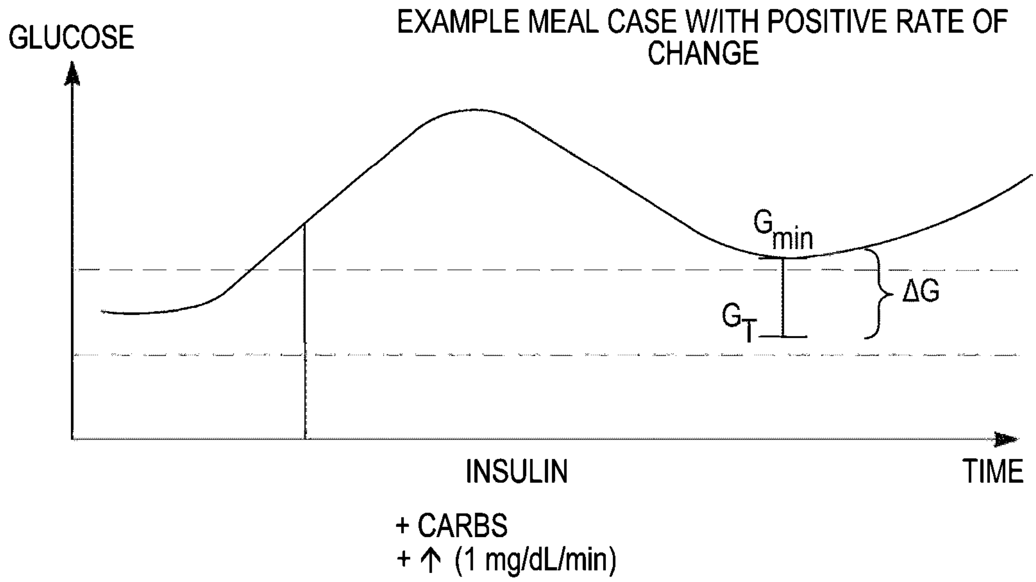


FIG. 27A

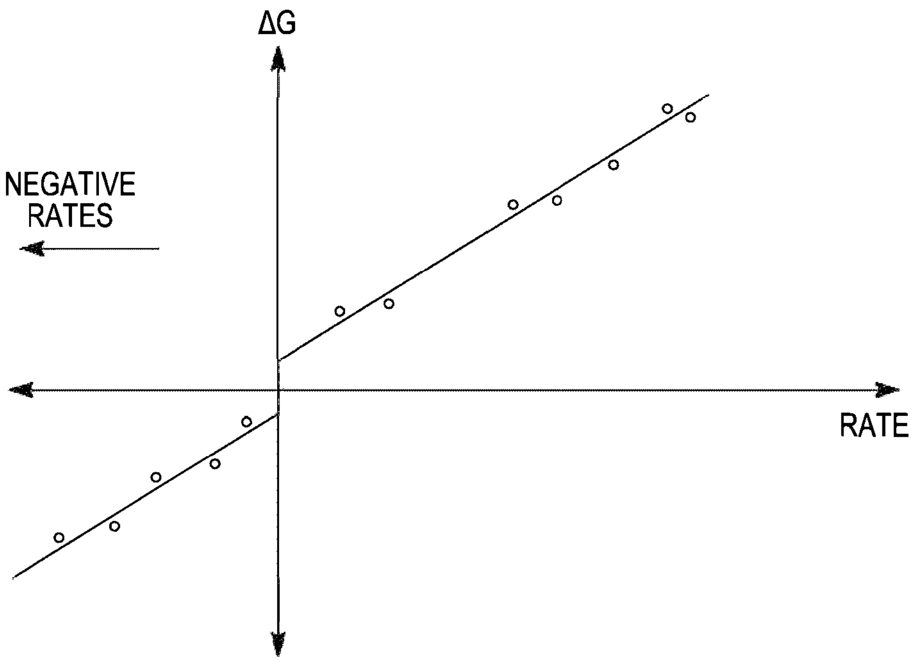


FIG. 27B

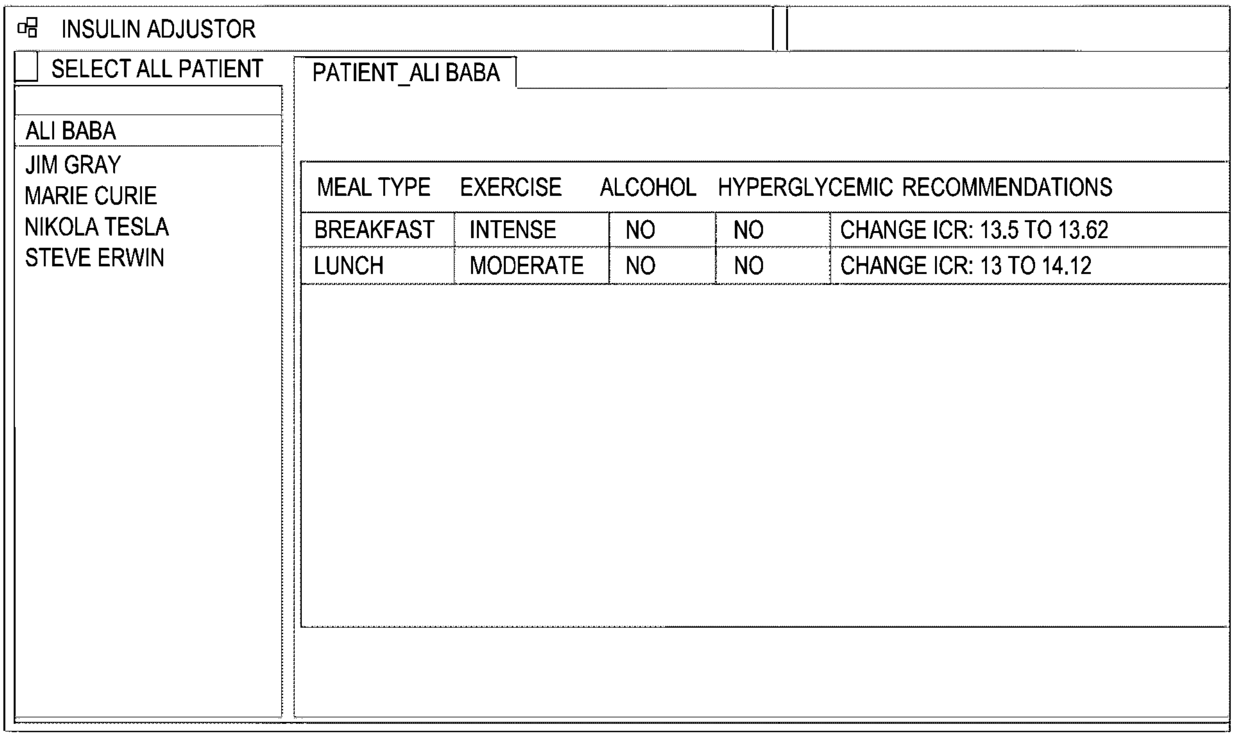
INSULIN ADJUSTOR
SELECT ALL PATIENT
ALI BABA
JIM GRAY
MARIE CURIE
NIKOLA TESLA
STEVE ERWIN
PATIENT_ALI BABA
MEAL TYPE
EXERCISE
ALCOHOL
HYPERGLYCEMIC
RECOMMENDATIONS
BREAKFAST
INTENSE
NO
NO
CHANGE ICR: 13.5 TO 13.62
LUNCH
MODERATE
NO
NO
CHANGE ICR: 13 TO 14.12

FIG. 28

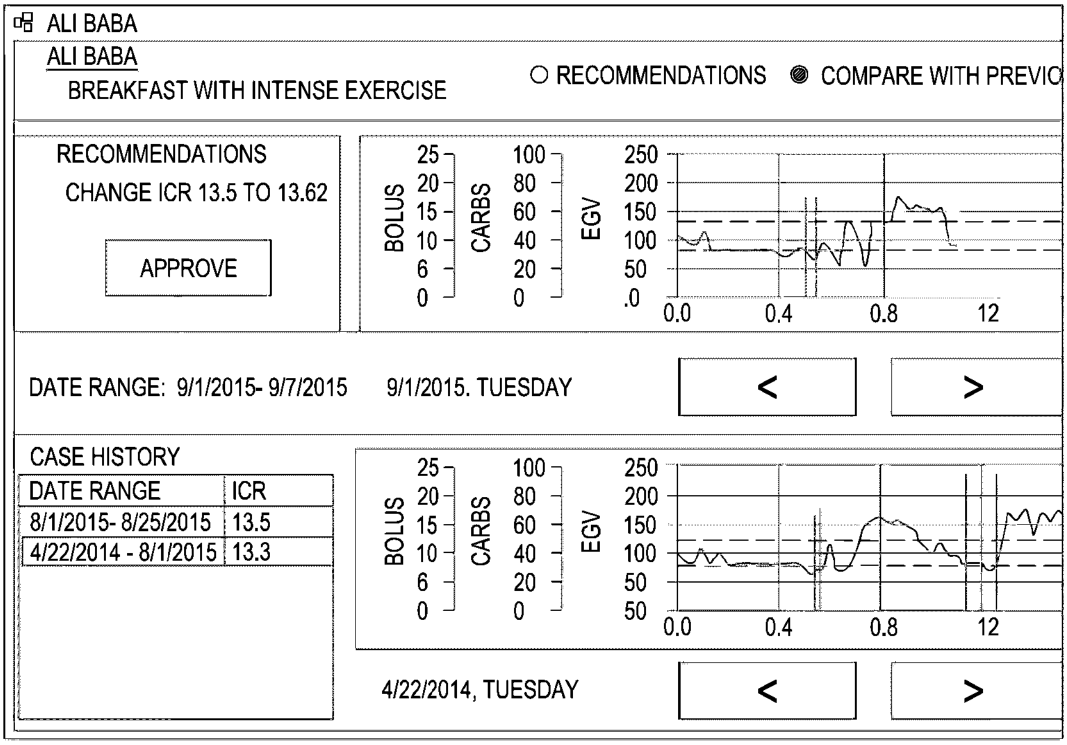
ALI BABA
ALI BABA
BREAKFAST WITH INTENSE EXERCISE
RECOMMENDATIONS
COMPARE WITH PREVIO
RECOMMENDATIONS
CHANGE ICR 13.5 TO 13.62
APPROVE
BOLUS
CARBS
EGV
25
20
15
10
6
0
100
80
60
40
20
0
250
200
150
100
50
.0
0.0
0.4
0.8
12
DATE RANGE: 9/1/2015- 9/7/2015
9/1/2015. TUESDAY
<
>
CASE HISTORY
DATE RANGE
ICR
8/1/2015- 8/25/2015
13.5
4/22/2014 - 8/1/2015
13.3
4/22/2014, TUESDAY

FIG. 29

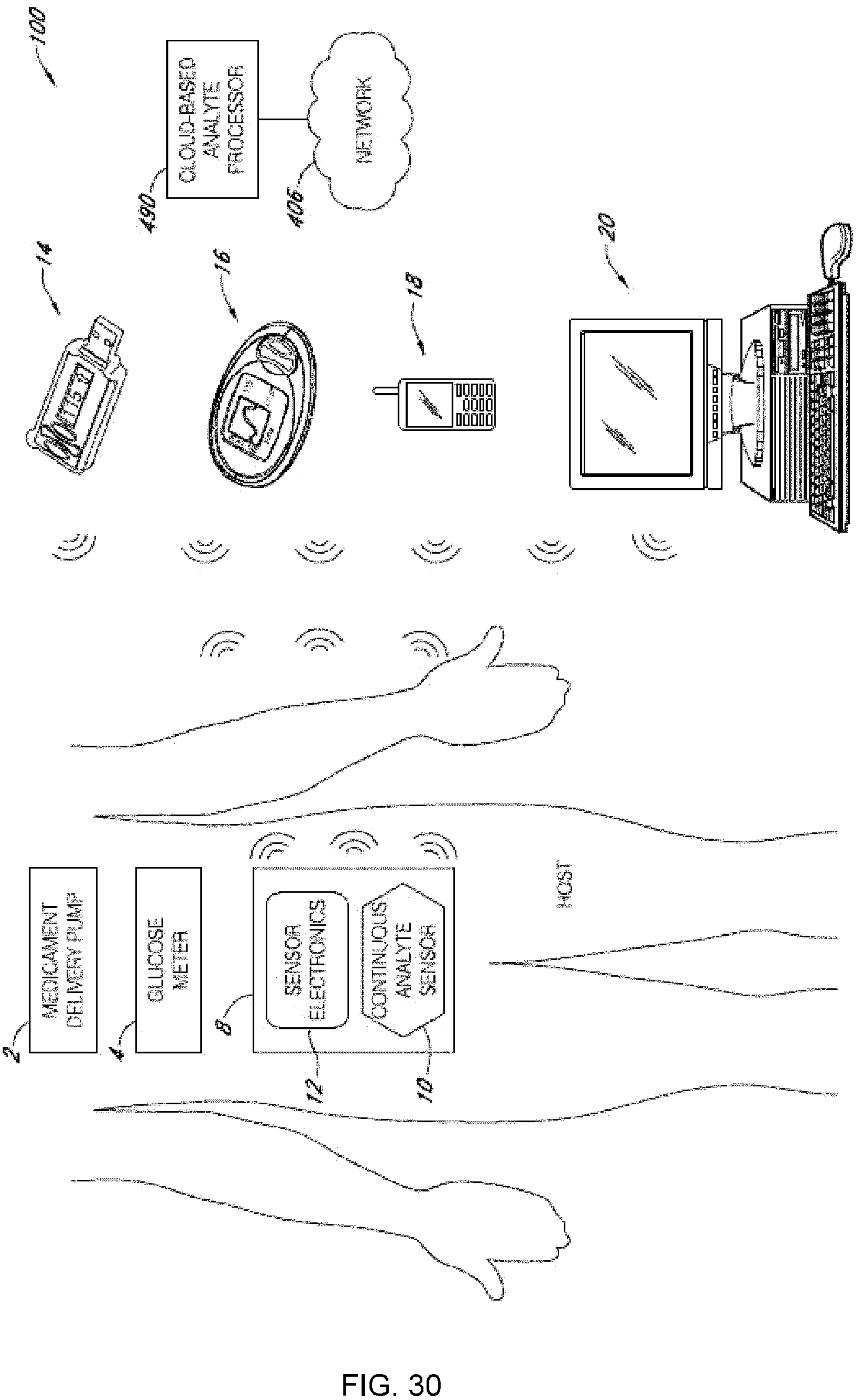
100
CLOUD-BASED ANALYTE PROCESSOR
490
NETWORK
406
14
16
18
20
MEDICAMENT DELIVERY PUMP
2
GLUCOSE METER
4
SENSOR ELECTRONICS
12
CONTINUOUS ANALYTE SENSOR
10
8
HOST

FIG. 30

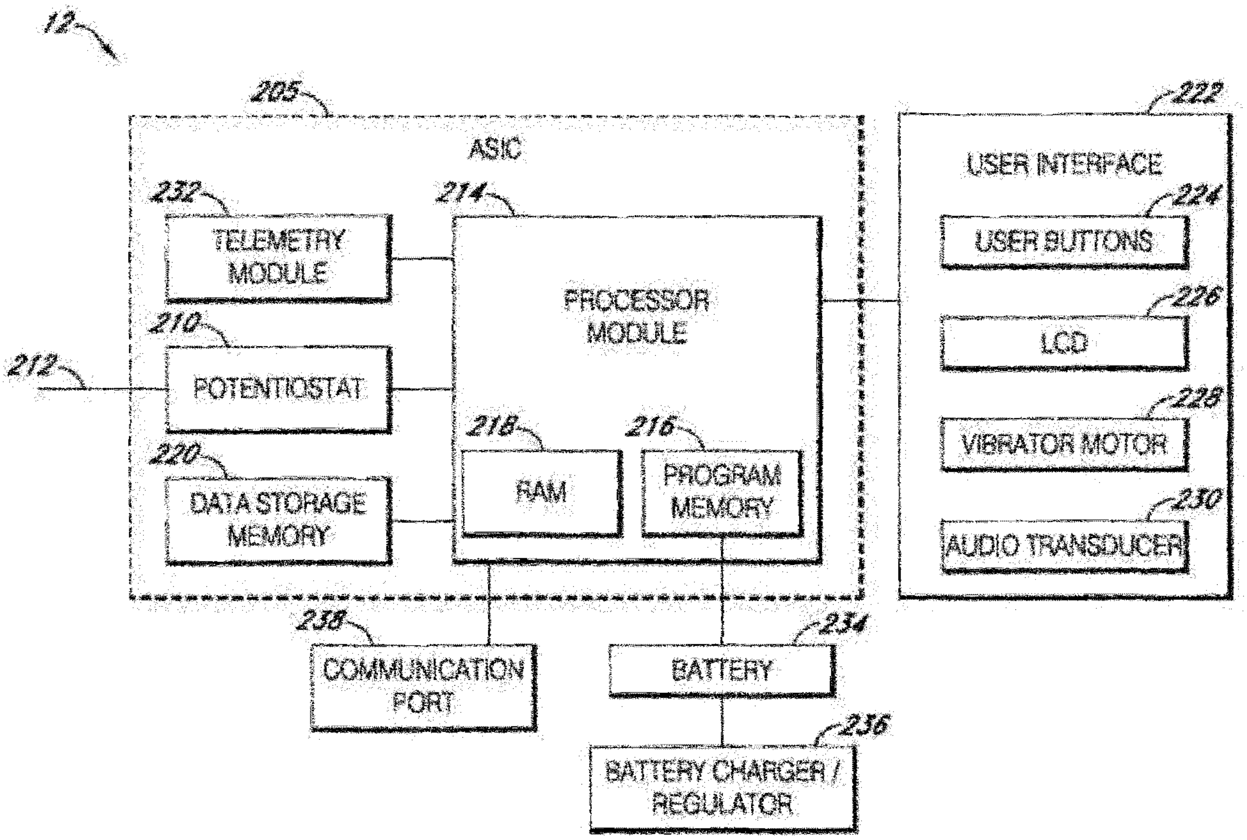
12
205
ASIC
232
TELEMETRY MODULE
210
212
POTENTIOSTAT
220
DATA STORAGE MEMORY
214
PROCESSOR MODULE
218
RAM
216
PROGRAM MEMORY
222
USER INTERFACE
224
USER BUTTONS
226
LCD
228
VIBRATOR MOTOR
230
AUDIO TRANSDUCER
238
COMMUNICATION PORT
234
BATTERY
236
BATTERY CHARGER / REGULATOR

FIG. 31

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6001067 A
- US 20110027127 A1
- US 20060020187 A1
- US 20090137887 A1
- US 20070027385 A1
- US 80144513
- US 20160081597 A1
- US 20130325352 A1
- US 20050027463 A1
- US 20080119703 A1
- US 20080108942 A1
- US 7828728 B
- US 6565509 B, Say
- US 6579690 B, Bonnecaze
- US 6484046 B, Say
- US 6512939 B, Colvin
- US 6477395 B, Schulman
- US 6424847 B, Mastrototaro


### Non-patent literature cited in the description


- **KLAUS DONSA et al.** *Towards Personalization of Diabetes Therapy Using Computerized Decision Support and Machine Learning: Some Open Problems and Challenges*, 01 January 2015